# EUROPEAN PATENT SPECIFICATION

(11) **EP 0 673 417 B1**
(45) Date of publication and mention of the grant of the patent: **11.06.2003**
(21) Application number: 94900226.5
(22) Date of filing: 19.11.1993
(51) Int. Cl.: C12N 15/00, C07K 16/00, A61K 47/48

(54) **LIGAND BINDING VARIABLE DOMAIN (V-MIN) COMPRISING A FRAMEWORK REGION WITH A CYCLICALLY PERMUTED CENTRAL $g(b)-BARREL**
LIGAND-BINDENDE VARIABELE DOMANE (V-MIN), DIE EINE "FRAMEWORK"-REGION ENTHÄLT MIT EINEM ZYKLISCH PERMUTIERTEN ZENTRALEN $g(b)-TUBUS
DOMAINES VARIABLES DE LIAISON DE LIGANDS (V-MIN) COMPRENANT UNE REGION D'ENCADREMENT PRESENTANT UNE PERMUTATION CYCLIQUE DE LA STRUCTURE CENTRALE EN BARIL

(30) Priority: 23.11.1992 GB 9224588; 31.12.1992 GB 9227189
(43) Date of publication of application: 27.09.1995
(73) Proprietor: AstraZeneca AB, 151 85 Södertälje (SE)
(72) Inventor: SLATER, Anthony Michael, Chinley, Cheshire SK12 6DQ (GB); TIMMS, David, Macclesfield, Cheshire SK10 2DR (GB)
(74) Representative: Giles, Allen Frank
(86) International application number: GB9302375
(87) International publication number: WO94012625

(56) References cited:
- US-A- 4 946 778
- PROTEINS. STRUCTURE, FUNCTION AND GENETICS vol. 1, no. 4, December 1986, NEW YORK, NY, USA, pages 342 - 362 R. FINE ET AL. 'Predicting antibody hypervariable loop conformations. II: Minimization and molecular dynamics studies of MCPC603 from many randomly generated loop conformations.'
- CURRENT OPINION IN STRUCTURAL BIOLOGY vol. 2, no. 4 , 1992 , PHILADELPHIA, PA, USA pages 593 - 596 L. PRESTA 'Antibody engineering'

## Description

The present invention relates to ligand binding variable domains (V-mins) having structural rearrangement of the binding domain of immunoglobulins and other ligand binding members of the immunoglobulin superfamily.

An immunoglobulin consists of a pair of light chains (each light chain, L, comprising approximately 220 residues) and a pair of heavy chains (each heavy chain, H, comprising approximately 400 residues). Each chain contains domains of approximately 100 residues in the form of β sheets. (Kabat, E.A., Wu, T.T., Bilofsky, H., Reid-Milner, M., Perry, H., 1983, Sequences of Proteins of Immunological Interest, Public Health Service N.I.H. Washington DC). The sequence variable N-terminal domains in the light and heavy chains (V_{L} and V_{H}) associate to form a substructure (Fv) which contains the antigen recognition site. V_{L} and V_{H} domain fragments isolated by proteolysis (Hochman, J., Inbar, D., Givol, D. Biochemistry, 1973, 12, 1130 - 1135) or recombinant methods (US 4,642,334 from DNAX Corporation) have been shown to fold and combine to form an Fv fragment which retains antigen binding activity.

The advent of genetic engineering techniques has opened up the possibility of engineering antibodies, but since a different gene codes for each of the polypeptide chains of an antibody it is necessary to clone and express a gene for each polypeptide chain. These genes may be either expressed in separate hosts, in which case the resulting polypeptide chains from each host must be allowed to refold in solution and combine, or the genes may be expressed in the same host simultaneously in which case folding and association into the native structure with biological activity can occur after expression. The need to achieve expression of multiple genes, whether simultaneously in the same host or in multiple and different hosts, has proved to be problematic and inefficient. The impact of genetic engineering on monoclonal antibodies has been reviewed by winter & Milstein in Nature, 349, 1991, 293-299.

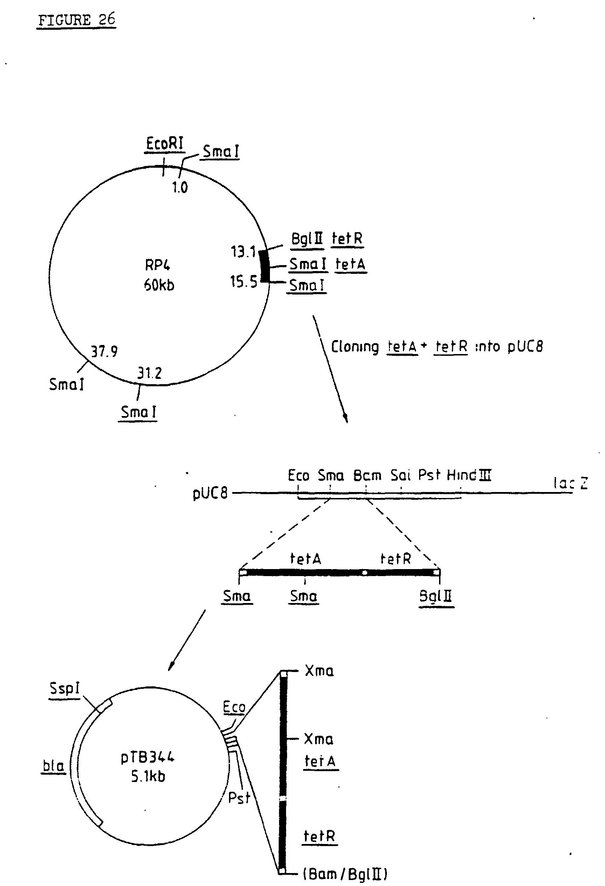

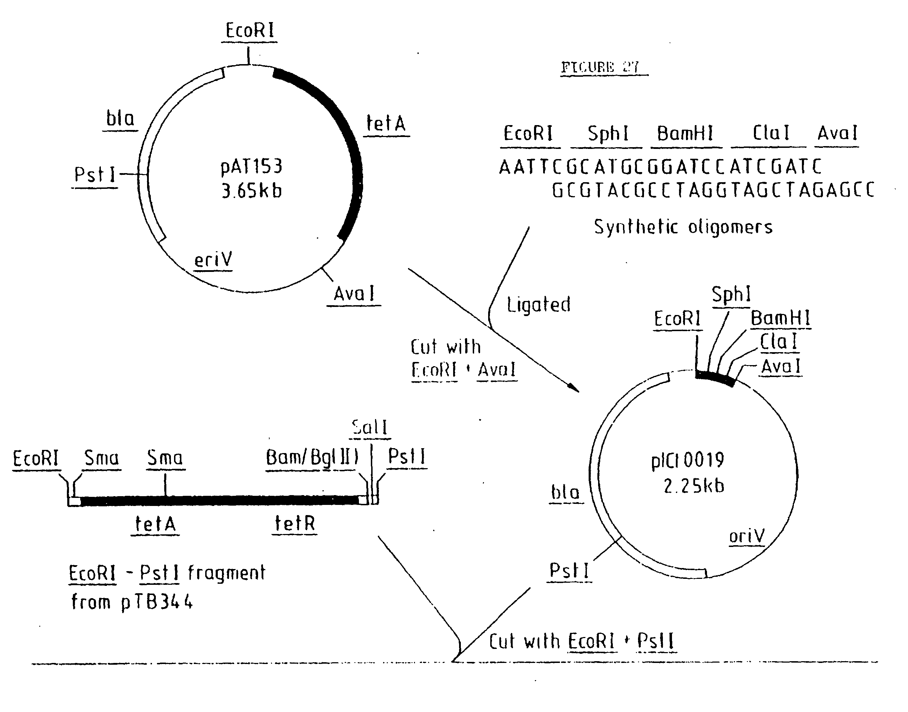

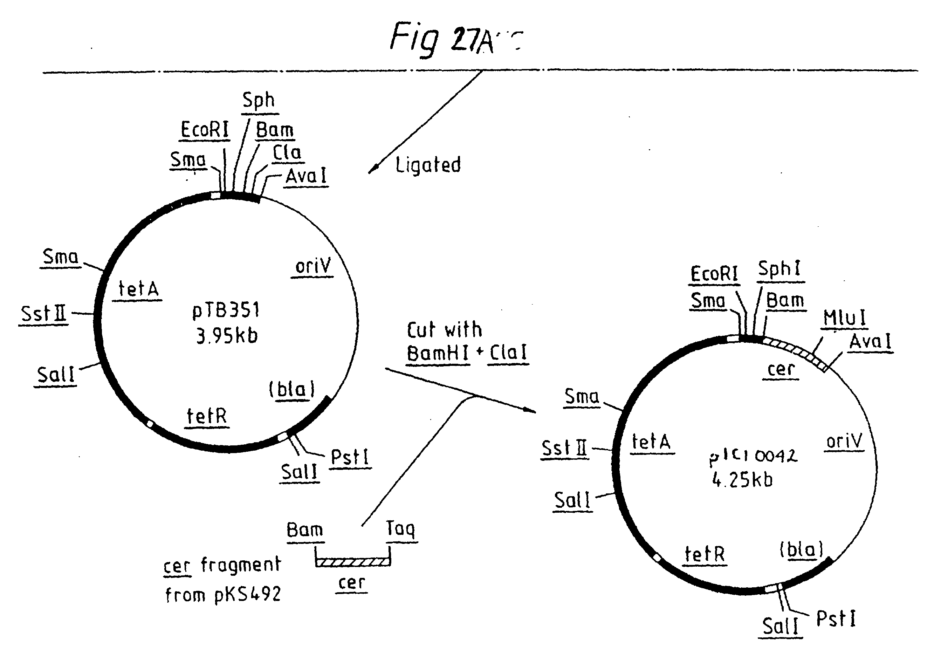

The Fv fragment represents a known antigen recognition factor comprising only about 220 residues out of approximately 1500 residues in the intact antibody, it still comprises two polypeptide chains. A single chain Fᵥ-like molecule has been described in US patent 4,946,778 of Genex Corporation in which a single peptide linker sequence foreign to the native Fv fragment is introduced to connect the C-terminus of one chain to the N terminus of the other chain. Three such constructs (TRY59, TRY61 & TRY104B) having 234-250 amino acids were made and tested for binding. Numerous references in Genex are made to the fact that loss of residues from the native protein is to be minimised (e.g. col. 7, lines 23-24; col. 16, lines 45-47, 57) presumeably fearing that loss of sequence would lead to loss of activity and/or stability. However on occasions when modelling a single linker does not appear feasible a 2 linker construct is suggested. Note the 2 linker approach is only suggested when the one linker approach does not work (col. 7, lines 28-29). One such 2 linker construct (TRY 40) having 225 amino acids was prepared and tested and again the reader is taught to minimise the loss of native protein sequences (col. 27, line 6). An error has been noted in Genex's description of construct TRY 40. The sequence listed in col. 33, lines 63-64 of Genex is not consistent with the full listing of the construct in Fig. 24 therein. The 2 linker construct TRY 40 described in Genex has lost residues L41-43 and H105-109 from the strands; thus destroying 3 inter-strand H-bonds and one β-bulge. The disruption of the strands in the central β-barrel is problematic since it destroys part of the key element for maintaining a robust structure in the variable region.

The present invention is based on recognising that the minimal elements necessary to retain good antigen binding affinity, the complementarity determining region (CDRs) and the central beta barrel structure for the Fv fragment, may be retained positioned appropriately in space by cyclic permutation of the central beta barrel structure and leaving the strands of the β barrel intact. The present invention is also independently based on recognising that after cyclic permutation of the central beta barrel the framework region of the Fv can be pared down through creation of novel linkages in the structure.

According to one aspect of the present invention there is provided a ligand binding variable domain (V-min) comprising:
(a) a cyclically permuted central β-barrel having intact strands;
(b) outer β-sheet segments;
(c) complementarity determining regions (CDRs); and
(d) linker sections for linking integers (a), (b) & (c) into a V-min, whereby said central β-barrel, optionally in combination with said outer β-sheet segments, orients said CDRs in space for binding ligand.

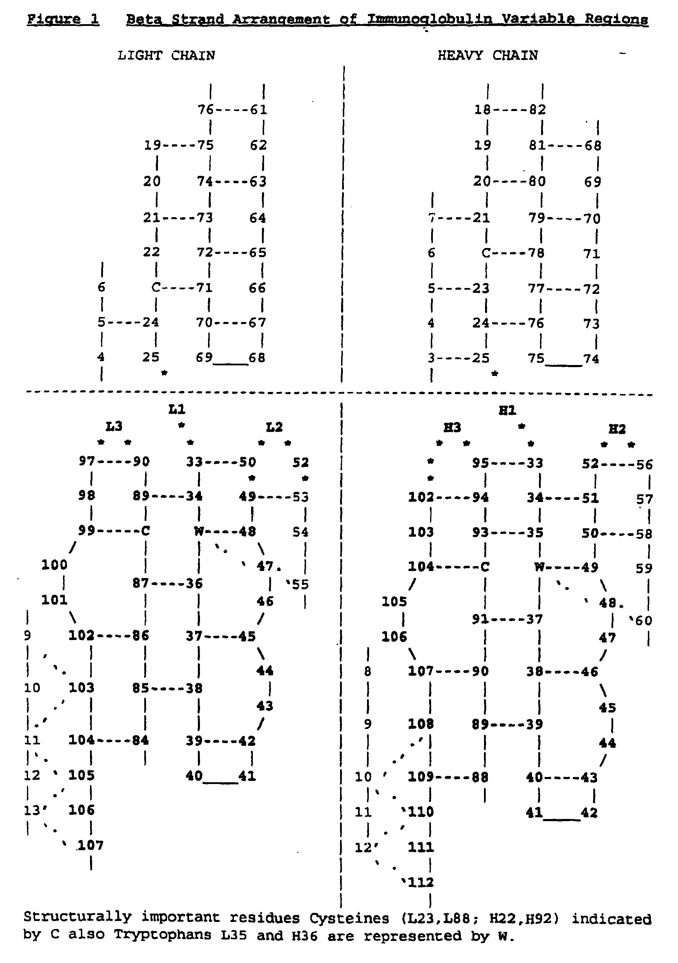

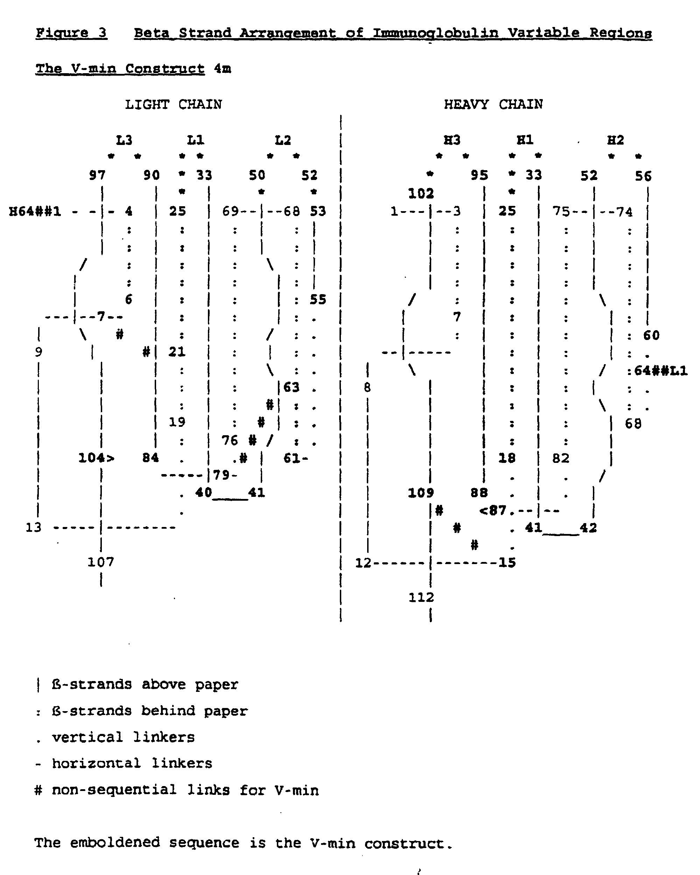

The term "central β-barrel" means the 8 stranded cylindrical barrel formed (at the VL-VH interface) by packing of two β-sheets (see Figure 3 in Novotny et al., 1983, Journal of Biological Chemistry, 258, 14433-14437). Note that Figure 3 in Novotny denotes the barrel as 9 stranded compared with 8 strands in our model (see Figure 1) but the inclusion of an extra strand (5 strands from the heavy chain rather than 4) is in our view incorrect due to it not representing a true strand because of insufficient H-bonding to any of the 8 principal strands. Elements of the extra strand in Novotny are more correctly denoted as outer β-sheet segments (see below). Preferably the linker sections provide covalent linking.

The term "cyclically permuted central β-barrel" means that the linear amino acid sequence order of appearance of strands in the central β-barrel is permuted compared with the linear amino acid sequence order of appearance of strands in a parent central β-barrel of an antibody.

The term "having intact strands" means that the individual strands are mostly complete and undisrupted whereby interactions such as H-bonds between strands of the central β-barrel are generally maintained despite cyclic permutation of the barrel. The following (canonically numbered) sequences define the strands: L33-40; L41-50, L84-90, L97-104; H33-41; H42-52; H88-95 and H102-109. Preferably at most one inter-strand H-bond is lost and especially no inter-strand H-bonds are lost. Conveniently no 8-bulges are lost from the strands. Preferably at most 3 amino acids are lost from the strands, more preferably at most 2 amino acids are lost from the strands, more preferably at most 1 amino acid is lost from the strands and especially the strands are completely intact.

The term "outer β-sheet segments" means segments of β-sheet, not being part of the central β-barrel of an antibody, which participate with the central β-barrel in providing a framework for orienting CDRs for binding ligand.

The term ''CDRs" means the hypervariable polypeptide loops which together determine ligand binding specificity and affinity; said CDRs are attached to a central β-barrel and/or outer β-sheet segments. Preferably there are 4-6 CDRs, more preferably 5-6 CDRs and especially there are 6 CDRs. Preferably the V-min variable domain is derived from an antibody although other ligand binding members of the immunoglobulin superfamily such as T-cell receptors are also contemplated.

Preferably the CDRs substantially reproduce the spatial orientation of CDRs from a parent antibody and substantially retain the ligand binding specificity and affinity of the parent antibody.

The term "linker sections" includes sequences from any protein (for example foreign sequences) such as for example those on the Brookhaven Protein Data Bank (abbreviated PDB herein: the relevant address is Protein Data Bank, Chemistry Department, Building 555, Brookhaven National Laboratory, Upton Nyl1973, USA and; a journal reference is Bernstein, F.C. et al. J. Mol. Biol., (1977) 112, 535-542).

The term "ligend binding" includes antigen-antibody type binding recognition and also ligand binding in the sense of catalytic functions of antibodies (see Jacobs, J.W. in Bio/Technology 9: 1991, 258-262; and Tramontano, A. in Methods in Enzymology 178, 531-568).

Preferably in the V-min the framework integers (a), (b) and (d) per se have upto 180 amino acids. The framework region is defined as any part of the variable domaim other than the CDRs.

Preferably the central β-barrel and outer β-sheet segments are from the same antibody (i.e. parent antibody) which may or may not be the parent antibody from which the CDRs are derived. In some circumstances it is advantageous to reduce immunogenicity, for example for therapeutic use in humans. To date human antibodies have proved difficult to produce for therapeutic use, therefore alternative approaches have been sought. One such approach is to "humanise" antibodies of non-human origin. For example CDRs from a mouse parent antibody may be grafted onto a human parent antibody framework region using known techniques (see EP 239400) thus reducing antigenicity in humans compared with the complete mouse parent. Another example is to graft a complete mouse variable region onto a human constant domain (see US 4 816 567). Another option is "antibody veneering" where surface residues of an antibody are altered to mimic a human antibody (George Mark presentation at 2nd Annual IBC Conference on Antibody Engineering, December 16 - 18, 1991, San Diego, USA and: EP519596 , Merck/NIH, Padlan et al).

Preferably the V-min possesses an affinity for ligand of at at least 10E-5M; more preferably the V-min possesses an affinity for ligand of at least 10E-6M; more preferably the V-min possesses an affinity for ligand of at least 10E-7M, more preferably the V-min possesses an affinity for ligand of at least 10E-8M, more preferably the V-min possesses an affinity for ligand of at least 10E-9M, more preferably the V-min possesses an affinity for ligand of at least 10E-10M and especially the V-min possesses an affinity for ligand of at least 10E-11M. In general, when a V-min is based on a parent antibody, the V-min preferably has an affinity for ligand at least about the same as that of the parent antibody. Modification of V-min to give increased affinity compared with a parent antibody is contemplated.

Preferably the V-min has less than 2 covalent linkages between the heavy and light chains.

Preferably there is provided a V-min as hereinbefore described in the form of one continuous polypeptide. This aspect simplifies recombinant production of the V-min. However chemical synthesis of V-mins is also contemplated.

Preferably there is provided a V-min as hereinbefore described comprising the following core segments: light chain residues 21-55; light chain residues 84-104; heavy chain residues 21-60 and; heavy chain residues 88-109. This aspect has the advantage of preserving the tight β-turns in the heavy and light chains around residue 40.

Preferably there is provided a V-min as hereinbefore described wherein the linker sections essentially consist of sequences from the antibody used as the source of the central β-barrel. This aspect has the advantage of substantially avoiding the introduction of foreign sequence into the V-min. Thus the modelling process is simplified since conformation of foreign linkers does not have to be modelled; furthermore immunogenicity is reduced in the host species used to provide the antibody framework.

Preferably there is provided a V-min as hereinbefore described having less than about 180 amino acids in the framework regions. Preferably the V-min has less than about 175 amino acids, in the framework regions, more preferably the V-min has less than about 170 amino acids in the framework regions, more preferably the V-min has less than about 165 amino acids in the framework regions, more preferably the V-min has less than about 160 amino acids in the framework regions, more preferably the V-min has less than about 150 amino acids in the framework regions, more preferably the V-min has less than about 140 amino acids in the framework regions, more preferably the V-min is less than about 130 amino acids in the framework regions and especially the V-min has less than about 120 amino acids in the framework regions.
Chothia C. and Lesk A.M. in Journal of Molecular Biology, 1987, 196, 901-917 define the canonical CDRs as:

| | | |
|---|---|---|
| L26-32 | L50-52 | L91-96 |
| H26-32 | H53-55 | H96-101 |

which means that the canonical Framework regions are:

| | | | | |
|---|---|---|---|---|
| L1-25 | L33-49 | L53-90 | L97-107 FV | 91 residues |
| H1-25 | H33-52 | H56-95 | H102-112 Fv | 96 residues |
| | | | | total 187 residues |

This compares with one exemplified canonical V-min framework described in the examples set out below:

| H87-95 | H102-109 | H15-25 | H33-52 | H56-H64 | L1-7 | L21-25 | L33-49 | L53-63 | L79-90 |
|---|---|---|---|---|---|---|---|---|---|
| 9 | 8 | 11 | 20 | 9 | 7 | 5 | 17 | 11 | 12 |

| L97-104 | | | | | | | | | |
|---|---|---|---|---|---|---|---|---|---|
| 8 | | | | | | | | | |
| | | | | | | total 117 residues | | | |

i.e. a reduction of 37% (calculated from a reduction of 70 residues from the original 187 residues). Molecular Design and Modelling Concepts and Applications are described in Methods in Enzymology, volumes 202 and 203, Academic Press.

Minimally sized antibodies are desirable for certain applications such as for example where improved tissue penetration or fast clearance from the body is important (Friedman, P.N. et al., Cancer Research 53, 334-339: 1993). Improved tissue penetration is important in imaging applications and fast clearance from the body is important in clearance of toxic drugs from the circulation and in antibody directed enzyme prodrug therapy. Ward et al. in Nature, 341, 1989, 544-546 have described the binding activity of single VH domains called dAbs. DAbs have the disadvantage of only being able to present 3 CDRs and exposure of hydrophobic amino acid residues to polar environments gives rise to handling problems such as non-specific binding.

Further advantages of size minimalisation are that simpler structures may lend themselves to more specific chemistry or a wider range of chemistries during for example conjugation and the simplest/most compact structure may be more amenable to oligomerisation of a series of binding elements either in a homo or a hetero oligomer.

According to another aspect of the present invention there is provided a multivalent ligand binding moiety comprising at least one V-min as hereinbefore described. The term "multivalent" means that one mole of binding moiety can bind more than one mole of ligand; said ligand comprising one ligand (i.e. monospecific) or more than one ligand (e.g. bispecific). Preferably the multivalent ligand binding moiety is bivalent. For example one mole of bivalent ligand binding moiety can bind 2 moles of ligand and comprises either 2 ligand binding V-min domains of the present invention or one ligand binding V-min domain of the present invention and one other ligand binding moiety such as Fab, Fv or single chain Fv.

According to another aspect of the present invention there is provided a conjugate comprising a V-min as hereinbefore described or a multivalent ligand binding moiety defined as hereinbefore described and an effector molecule.

The term "effector molecule" refers to a molecule capable of producing an effect or a signal. Preferably the effector molecule is an enzyme, catalytic antibody, toxin, drug or radionuclide and especially the effector molecule is an enzyme or catalytic antibody for antibody directed enzyme prodrug therapy (1). The effector molecule may be covalently and/or non-covalently linked to the V-min. Known techniques for covalent linkage include heterobifunctional linkers and protein fusion. A known technique for non-covalent linkage is via leucine zipper motifs and the like (Kostelny, S.A. et al., J. Immunol. 148 1992, 1547 - 1553 and Pack, P. et al., Biochemistry 31 1992, 1579-1584). In some cases a mixture of both covalent and non-covalent linkage is used. For example a polylysine moiety may be covalently linked to a V-min and the positive charge on the polylysine moiety may non-covalently link a complementary charged moiety such as nucleic acid. Another example is covalent linkage of a chelate to a V-min; the chelate then non-covalently binding a moiety such as a radionuclide.

According to another aspect of the present invention there is provided a polynucleotide sequence encoding a V-min as hereinbefore described or a multivalent ligand binding moiety as hereinbefore described.

According to another aspect of the present invention there is provided an expression vector comprising a polynucleotide sequence as hereinbefore described. The expression vector may direct secretion of translated polypeptide chain for facilitating folding of the chain into a ligand binding conformation. Conveniently the expression vector directs expression of translated polypeptide for intracellular accumulation, especially as inclusion bodies.

According to another aspect of the present invention there is provided a host cell comprising an expression vector as hereinbefore described. Preferably the host cell is procaryotic and especially the host cell is E. coli.

According to another aspect of the present invention there is provided a method of producing a V-min as hereinbefore described or a multivalent ligand binding moiety as hereinbefore described comprising culture of a host cell as hereinbefore described. Methods for production and analysis of single chain antibody molecules as well as general antibody engineering techniques have been set out in the following publications:
1. BEDZYK, W.D.: J. Biol. Chem., (1990), 18615-18620
2. HUSTON, J.S.: Proc. Natl. Acad. Sci., (1988), 85, 5879-5883
3. BIRD, R.E.: Science, 242, (1988), 423-426
4. WHITLOW, M.: Methods, A Companion to Methods in Enzymology, 2(2), (1991), 97-105
5. WARD, E.S.: Antibody Engineering, A Practical Guide, C.A.K. Borraebaeck (Ed), W.H. Freeman New York (1992), Ch 6: 121-137
6. ANAND, N.N.: J. Biol. Chem., 266(32), (1991), 21874-21879
7. SKERRA, A.: Current Opinion in Immunology, 5, (1993), 256-262
8. PLUCKTHUN, A.: Bio/technology, 9, (1991), 545-551
9. GLOCKSHUBER, R.: Biochemistry, 29, (1990), 1362-1367
10. SKERRA A.: Bio/technology, 9 (1991) 273-278
11. PLUCKTHUN, A.: Nature, 347, (1990), 497-498
12. FORD, C.F.: Protein Expression & Purification, 2, (1991), 95-107
13. PLUCKTHUN, A.: Immunological Reviews, 130, (1992), 151-188
14. HOLLINGER, P.: Current Opinion in Biotechnology, 4 (1993), 446-449
15. BRINKMANN, U: Proc. Natl. Acad. Sci. USA, 90 (1993), 7538-7542.
16. Methods in Enzymology, vol 178.

Analogous methodology can be applied to V-mins described herein. Inspection of databases has indicated over 100 publications at present on single chain antibody molecules covering many different antibodies thus indicating the general applicability of modelling concepts across this family of molecules.

According to another aspect of the present invention there is provided a pharmaceutical composition comprising a conjugate as hereinbefore described an active ingredient in association with a pharmaceutical excipient or carrier. Conveniently the composition comprises an effective amount of the active ingredient.

According to another aspect of the present invention there is provided a radioimaging composition comprising a conjugate as hereinbefore described as an radioimaging ingredient in association with a pharmaceutical excipient or carrier. Conveniently the composition comprises an effective amount of the radioimaging ingredient.

The reader is referred to Comprehensive Medicinal Chemistry, Volume 5 (Biopharmaceutics), Hansch (Editor), Pergamon Press 1990; especially chapters 25.2 (formulation), 25.3 (Dosage Regimes) and 25.5 (Drug Targetting). Section 25.5.5.1 therein discusses immunotoxins and Section 25.5.5.3 discusses antibody-radionuclide conjugates for imaging and radiotherapeutic purposes. Radioimaging is described in Larson (1987). Radiotherapy 165, 297. A reference work on toxins is "Genetically Engineered Toxins" edited by A.E. Frankel, Marcel Dekter Inc. 1992. Immunotoxin Preparation is discussed in chapters 20 and 21 of Methods in Enzymology, Vol 93, Academic Press 1993.

According to another aspect of the present invention there is provided a method of treatment of a tumour comprising the administration to a human or animal in need of such treatment of a pharmaceutically effective amount of a conjugate as hereinbefore described.

According to another aspect of the present invention there is provided an immunotoxin pharmaceutical composition comprising a V-min as hereinberore defined in association with a pharmaceutical excipient or carrier.

According to another aspect of the present invention there is provided a radioimaging composition comprising a V-min as hereinbefore defined in association with a pharmaceutical excipient or carrier.

According to another aspect of the present invention there is provided an antibody-enzyme pharmaceutical composition for ADEPT comprising a V-min as hereinbefore defined. Many cytotoxic compounds have been discovered which are of potential use in cancer chemotherapy. Nitrogen mustards form one important family of such cytotoxic compounds. The clinical use of cytotoxic compounds in general and nitrogen mustards in particular has been limited because of the poor selectivity in the cytotoxic effect between tumour cells and normal cells. One approach to overcome this problem has involved the development of so-called pro-drugs which are derivatives of the cytotoxic drug, often a relatively simple derivative, whose cytotoxic properties are considerably reduced compared to those of the parent drug. Proposals have been made for the administration of such pro-drugs to patients under regimes whereby the pro-drug is only converted to the cytotoxic drug in the region of the intended site of action. One approach involves linkage of a cytotoxic parent nitrogen mustard with an amino acid to form a pro-drug which can be converted to the parent nitrogen mustard at the site of intended action under the influence of an enzyme. This approach can be put into practise by the utilisation of an antibody/enzyme conjugate in association with a pro-drug. The antibody/enzyme conjugate is formed from an antibody selective for tumours and an enzyme that will convert the pro-drug to the cytotoxic drug. In clinical practice, the antibody/enzyme conjugate is first administered to the patient and is allowed to bind to the tumour. After a suitable period of time, to allow clearance of the antibody/enzyme conjugate from the rest of the body, the pro-drug is administered to the patient. Conversion of the pro-drug, under the influence of the localised enzyme, to the cytotoxic drug takes place mainly in the region of the tumour. Such a system (Antibody Directed Enzyme Prodrug Therapy or ADEPT) is described in International Application PCT/GB88/00181 published as WO88/07378 and US Patent No 4,975,278.

According to another aspect of the present invention there is provided a method of treatment of a tumour comprising the administration to a human or animal in need of such treatment of a pharmaceutically effective amount of a pharmaceutical composition for ADEPT or an immunotoxin pharmaceutical composition.

According to another aspect of the present invention there is provided use of a radioimaging composition as hereinbefore defined to produce a radioimage as an aid to diagnosis of a condition in a human or animal.

The invention is illustrated, but not limited, by the following examples in which:
Figure 1 illustrates the Beta Strand Arrangement of Immunoglobulin Variable Regions in which structurally important residues Cysteines (L23-L88, H22-H92) indicated by C and Tryptophans L35 and H36 are represented by W. The upper section illustrates the outer β-sheets for each chain. These sheets overlay the inner sheets shown in the lower section of the diagram. Folding along the horizontal crease yields the β-sandwiches for the two chains. Juxtaposition of the inner sheets (fold along the vertical crease) generates the central β-barrel (highlighted). Folding along the horizontal crease and then rolling the sides of the paper to meet each other generates the inner central barrel surrounded by outer strands etc.
   The dotted lines connecting the strands represent hydrogen bonding between strands; straight dotted lines are H-bonds formed between antiparallel strands and wavy dotted lines are H-bonds formed between parallel strands.
Figure 2 illustrates the core segments as emboldened sequence.
Figure 3 illustrates the Beta Strand Arrangement of Immunoglobulin Variable Regions the V-min Construct 4m in which:
   | β-strands above paper
   : β-strands behind paper
   . vertical linkers
   - horizontal linkers
   # non-sequential links for V-min
      The emboldened sequence is the V-min construct.
Figure 4 illustrates alignment of FAB light chains for F19.9 & 1MCP Complementarity Determining Loops are underlined.
   Canonical Structure Type (reference 4) is indicated alphabetically Framework residues important to Loop conformations are indicated by *.
   Segments used in the V-min design are demarcated by < >.
   The order of appearance in the V-min sequence is indicated by s#.
Figure 5 lists: (a) amino acid sequence for F19.9 V-min constructs; (b) amino acid sequence for 1MCP V-min.
Figure 6 lists the initial nucleotide sequence for F19.9 V-min.
Figure 7 illustrates a V-min construct in bold line with redundant sequence in faint line.
Figure 8 shows the α-carbon diagram of a final V-min structure.

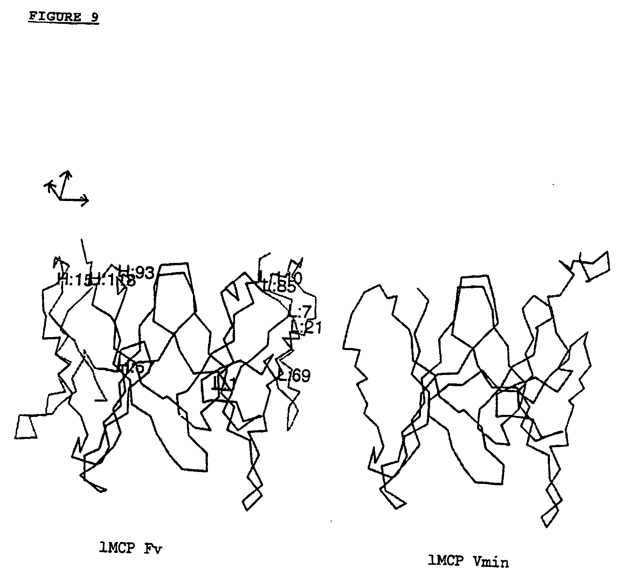

Figure 9 shows a V-min compared with a Fv structure. Faint line on the Fv represents sequence discarded to form the V-min. The bottom left corner of the V-min represents TAG sequence added.
Figures 10, 10A & 11 show that the environment of possible problematic polar groups in V-min structures have adequate bonding. Figures 10 and 10A show the environments for residues Glu55 and Lys97 in F19.9 V-min respectively. Figure 11 shows the environments for Lys101 and Gln108 in IMCP V-min.
Figure 12 is a diagrammatic representation of Fv, central β barrel and minimal V-min constructs.
Figure 13 illustrates the V_{L} β-strand arrangement.
Figure 14 illustrates the V_{H} β-strand arrangement.
Figure 15 illustrates the Fv β-sandwich arrangement with the heavy chain in continuous line and the light chain in broken line.
Figure 16 illustrates a conservative V-min construct including redundant-segments.
Figure 17 illustrates the conservative V-min construct of Fig 16 but excluding redundant segments.
Figure 18 illustrates a minimal V-min construct including redundant segments.
Figure 19 illustrates the minimal V-min construct of Fig 18 but excluding redundant segments.
Figure 20 illustrates construction of a MCP603 V-min gene from oligonucleotides.
Figure 21 is the peptide sequence of a MCP603 V-min construct including c-myc peptide tag.
Figure 22 illustrates pBluescript.
Figure 23 illustrates pMIN603.
Figure 24 illustrates construction of pICI0020.
Figure 25 illustrates a trp promoter.
Figure 26 illustrates construction of pTB344.
Figure 27/27A illustrates construction of pICI0042.
Figure 28 is a transcription terminator sequence.
Figure 29 illustrates construction of pLB013.
Figure 30 illustrates pICI123.
Figure 31 illustrates pICI1079.
Figure 32 illustrates construction of pICI1572.
Figure 33 illustrates construction of a F19.9 V-min gene using oligonucleotides.
Figure 34 illustrates a F19.9 V-min peptide sequence.
Figure 35 shows the sequence of pSW1-VHD1.3-VKD1.3-TAG1 from the start of the first pelB leader sequence to approximately 50 bases beyond the end of the TAG1. Numbered from the start of the pelB. Amino acid sequence of the pelB-VHD1.3 and pelB-VKD1.3-TAG1 genes are shown below the DNA sequence.
Figure 36 shows PCR generation of D1.3 V-min
Figure 37 shows products from first round PCR reactions
Figure 38 shows a plasmid map of pICI226
Figure 39 shows a plasmid map of pJFH3037
Figure 40 shows D1.3 V-min conservative
Figure 41 shows h65-115/h13-64/11-108 D1.3 V-min with Intact Light Chain
Figure 42 shows 161-107/113-60/h1-116 D1.3 V-min with Intact Heavy Chain
Figure 43 shows h87-115/hl3-64/11-7/121-61/176-108 D1.3 V-min minimal Construct
Figure 44 shows h67-125/h13-66/11-112 KOL V-min with Intact Light Chain.
Figure 45 shows 162-109/112-61/h1-125 KOL V-min with Intact Heavy Chain
Figure 46 shows h66-118/h13-65/11-112 PAC-1 V-min with Intact Light Chain
Figure 47 shows 166-111/112-65/h1-123 PAC-1 V-min with Intact Heavy Chain
Figure 48 shows binding data for a D1.3 V-min
Table 1 lists Cα-Cα distances for linker sections of the MPC Fv fragment
a) L55-84 and L104-107 versus L1-21
b) H60-88 versus L1-21
c) H60-88 and H108-113 versus H1-21
d) L55-84 versus H1-21
e) L55-84 versus L104-107 and H60-88 versus H108-113
f) Intra Section relationships
Table 2 lists oligonucleotides used in gene assembly

### EXAMPLE 1 MODELLING STUDIES

V-min proteins have been designed, modelled and analysed. One is based on a known (2) FAB X-ray structure, 1MCP (also referred to herein as McPC603), a mouse monoclonal which binds phosphatidyl choline. Another is based on F19.9 which lacks an experimental structure for the FAB fragment. We have therefore generated a FAB model for F19.9 by analogy with the known structure of 1MCP. This homology modelling approach (3) is a well established method for generating structural models of proteins that exhibit more than 30% identity to a protein whose 3D structure has been determined experimentally. The methodology set out below can be applied to any antibody and was used to generate the V-min structures in the examples herein.

### 1.1 Background

The variable Fv region of the FAB structure forms the basis for designing the V-min structure and, in the general case, a structural model of the Fv region is generated by homology modelling based on the most closely analogous known structure.

As indicated earlier, the central eight-stranded β-barrel of the Fv structure is retained in the V-min design. In the Fv structure, four β-strands are contributed by the light chain, and four by the heavy chain.

Four of the six complementarity determining regions (CDR's) appear as apical loops connecting these β-strands (L3, L2, H3 & H2 in Fig 1). The remaining two CDR's connect the N-terminal segments of each chain to the first barrel β-strand in that chain (L1 & H1 in Fig 1).

The arrangement of β-strands in the variable regions of immunoglobulin light and heavy chains is shown in Figure 1 using the canonical numbering of Chothia and Lesk (4).

In Figure 1, the upper section illustrates the outer β-sheets for each chain. These sheets overlay the inner sheets shown in the lower section of the diagram. Folding along the horizontal crease (dotted line) yields the β-sandwiches for the two chains.

Juxtaposition of the inner sheets (fold along the vertical crease) generates the central β-barrel (highlighted).

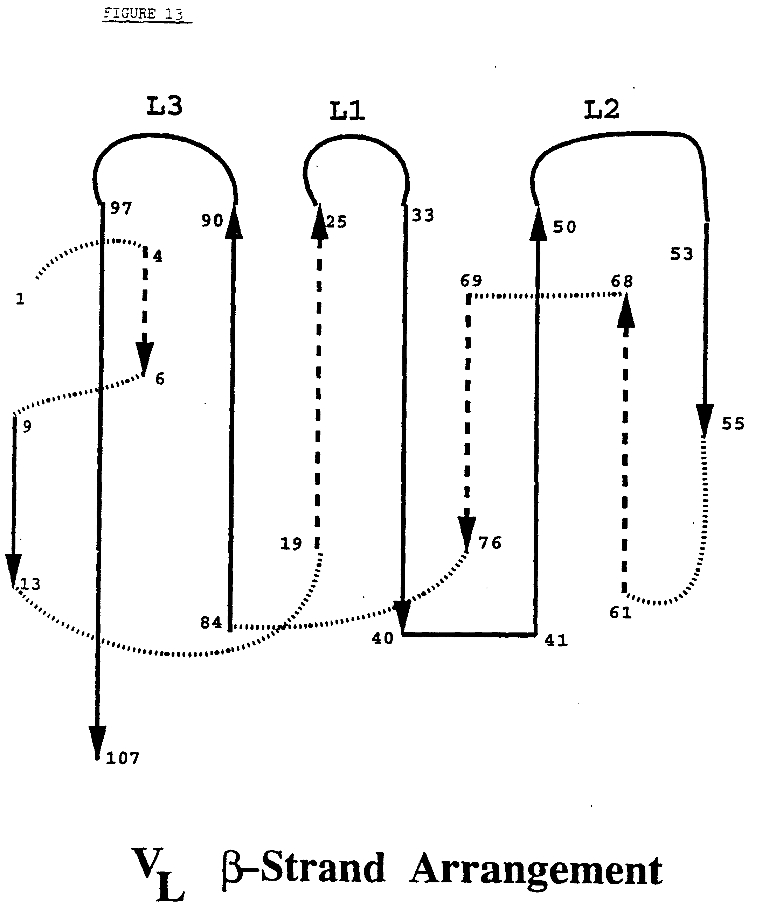

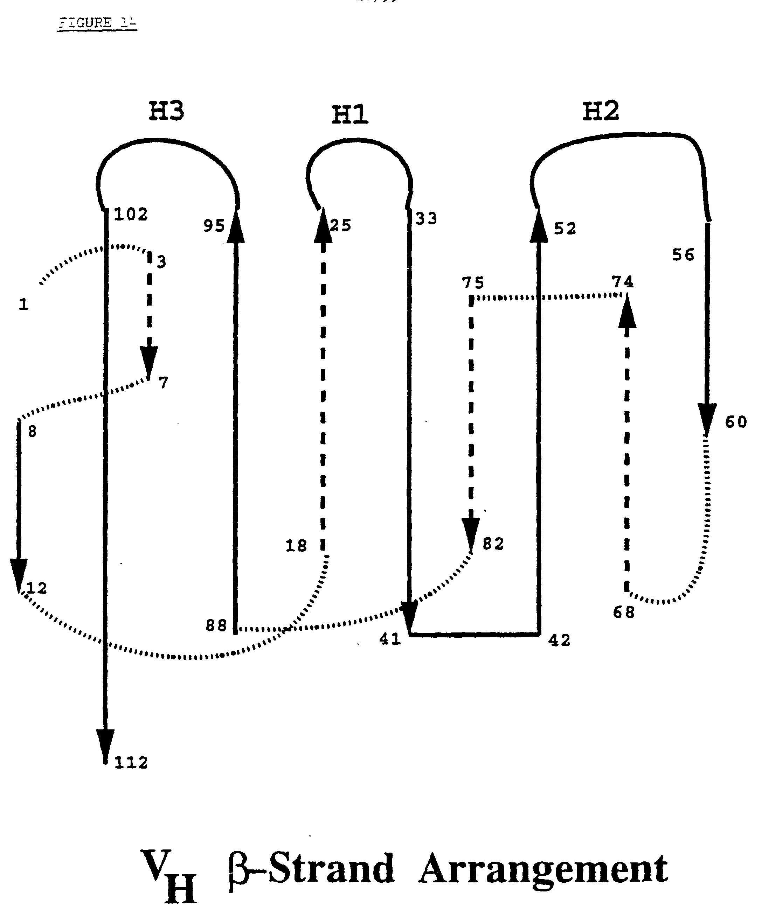

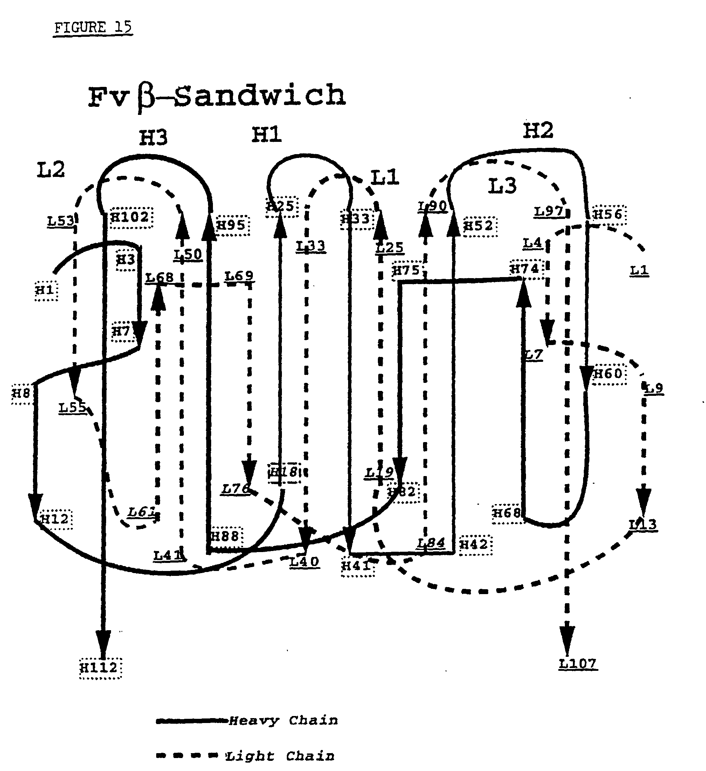

Figures 13, 14, and 15 also illustrate the arrangement of β-strands.

### 1.2 The Minimal Recognition Structure

In addition to the central β-barrel, four segments of β-strand (L19-25, L53-55, H18-25, H56-60) are required for the generation of CDR's-I and II in both chains.

Residues on either side, in sequence terms, of the CDRs are anchored in the central β-barrel. These three segments per chain are defined below:
Light Chain 21-39, 42-55, 84-104
Heavy Chain 20-40, 43-60, 88-109

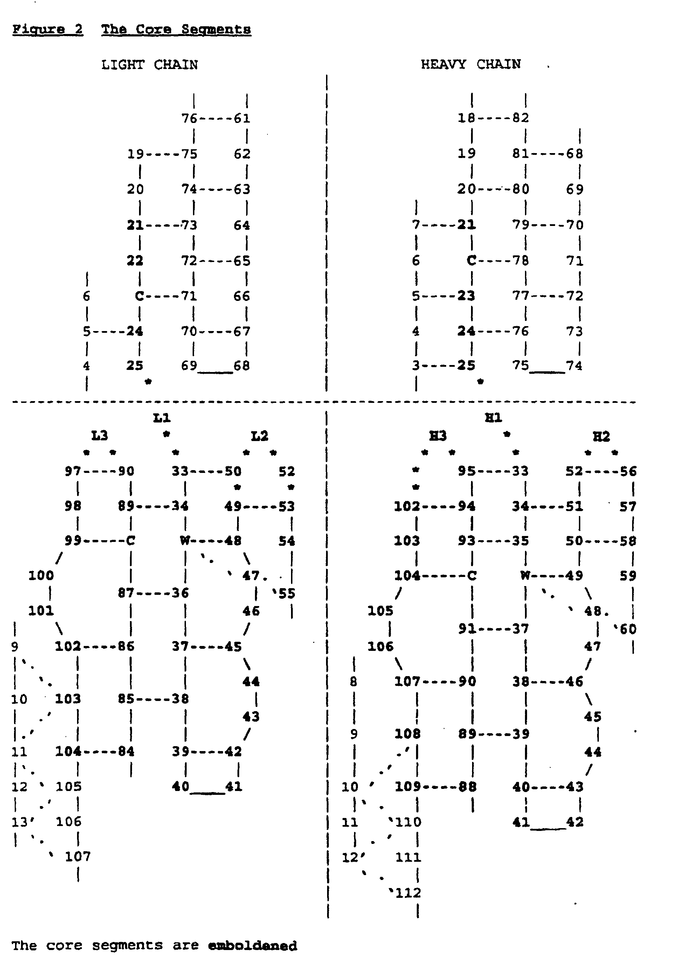

In order to avoid disrupting the two tight ßturns at L39-L42 and H40-H43 we have adopted the following four **core segment** model (see Figure 2):
Two core segments from the Light Chain
   L21-55 ie light chain residues 21-55 (contains CDR's LI and LII)
   L84-104 ie light chain residues 84-104 (contains CDR LIII)
Two core secrments from the Heavy Chain
   H21-60 ie heavy chain residues 21-60 (contains CDR's HI and HII)
   H88-109 ie heavy chain residues 88-109 (contains CDR HIII)

### 1.3 Single Chain Constructs

In order to produce a single chain species, these four core segments are linked together using spatially appropriate sections from the intact structure. These **linker sections** are indicated below (NB lower case is used to differentiate linker sections from the core segments).

| **Section** | **Chain** | **Residue Numbers** |
|---|---|---|
| 11-20 | Light | 1-20 |
| 156-83 | Light | 56-83 |
| 1105-107 | Light | 105-107 |
| h1-20 | Heavy | 1-20 |
| h61-87 | Heavy | 61-87 |
| h109-113 | Heavy | 109-113 |

(NB non-natural, that is foreign, linker sections could also be designed to do this such as for example using sequences extracted from the Brookhaven data bank as outlined in Genex US4946778).

The cyclic nature of the central β-barrel means that the four core segments can be combined in different orders. For example, by linking the C-terminus of a given segment to any one of the N-termini from the three other segments. There are thus potentially 24 different combinations. Their feasibility can be determined by means of the spatial relationships of the available elements of the intact structure (ie the linker sections) that can be used to join the core segments. The adjacency of these linker sections can be evaluated in terms of the distances between the α-carbons of the residues involved (see Table 1).

These distance tables can be used to produce an adjacency matrix of possible linkages. If any α-carbon in one linker section is sufficiently close to any of another we define the sections as adjacent. The cut-off distance that we have used is 15Å, since this allows for the reorientation of up to six residues in order to create a contiguous structure. In the following table, + indicates sections that are adjacent by the above definition, - indicates sections that are too distant to connect and 0 represents self with self.

| | l1-20 | l56-83 | l105-107 | h1-20 | h61-87 | h109-113 |
|---|---|---|---|---|---|---|
| l1-20 | 0 | + | + | - | + | - |
| l56-83 | + | 0 | + | + | - | - |
| l105-107 | + | + | 0 | - | - | - |
| h1-20 | - | + | - | 0 | + | + |
| h61-87 | + | - | - | + | 0 | + |
| h109-113 | - | - | - | + | + | 0 |

We can enumerate the possible combinations of linker segments via the adjacency diagram shown below, where double-headed arrows indicate two adjacent sections.

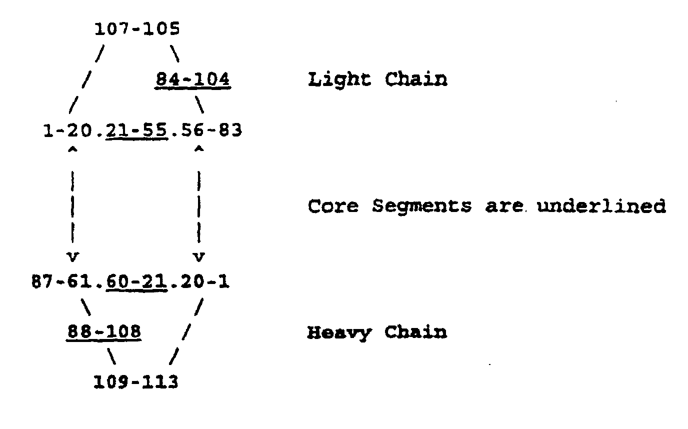

In the following, we use example residues to illustrate possible connections. Residues in the core segments (upper case) and linker sections (lower case) are separated by -. Where segments or sections are separated by : the sequence is continuous as for the native protein. Where / is used a novel linkage between segments is formed.

The lack of a direct connection between 1105-107 and h1-20 (or h109-113 and 11-20) means that the native sequence order can only be preserved by use of a foreign linker section (!!) :-
l1-20:L21-55:156-83:L84-104:l105-107/!!/h1-20:H21-60:h61-87:H88-108:h1 09-113
or vice versa.

Some of the proximal distances are non-productive in that they would connect a single segment in a closed fashion. For example; the distance between light chain residues 20 and 74 is 4Å but this merely connects linker sections 156-83 to 11-20, thus enclosing the core segment L21-55.

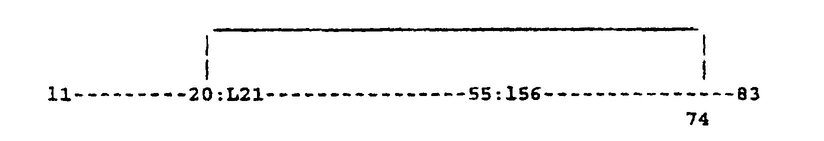

Exploiting the adjacency diagram, inter-chain connections involve:
11-20 <----> h61-B7, or h1-20 <----> 156-83 and the following four combinations may be possible :
1. L21-55:l56-83:L84-104/l1-20/h61-87:H88-108:h109-113/h1-20/H21-60
2. H21-60:h61-87:H88-108/h1-20/156-83:L84-104:l105-107/l1-20/L21-55
3. L84-104:l105-107/l1-20:L21-55:l56-83/h1-20:H21-60:h61-87:H88-108: h109-113
4. H88-108:h109-113/h1-20:H21-60:h61-87/l1-20:L21-55:156-83:L84-104: l105-107

In choosing specific residues from the linking sections to generate the connected sequences, we make use of the distance tables to identify candidates.

In construct 1, residues from the linker sections 11-20 and h61-87 are to be used to connect core segments L84-104 and H88-108. Analysis of the distance tables suggests that light chain residue 1 can connect to heavy chain residue 61, and that light chain residue 104 could link to light chain residue 13. The two candidates from l1-20, ie 13 and 1, also need to be connected. These two residues are connected in sequence l1-13, unfortunately, the direction would need to be reversed in this context.

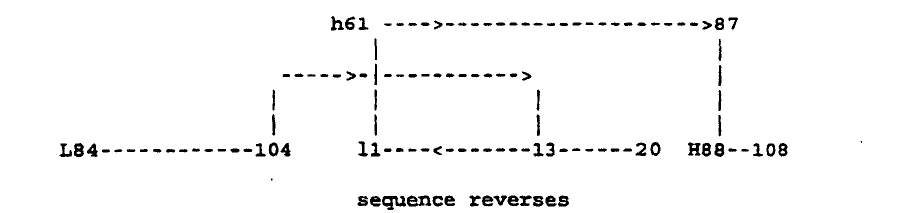

This process reveals that constructs 1 and 2 are unlikely unless foreign linkers (II) are used.
1. L21-104/L13/!!/L1/H61-113/H13-65
2. H21-108/H13/!!/H1/L57-107/L13-60
   Constructs 3 and 4 appear to be viable and represent generic constructs that produce single chain species in which the sequential order of the CDR's is changed and novel linkages using native residues are introduced.
   In construct 3, the sequential order of the light chain is reorganised to provide a connection to the intact heavy chain, whilst in construct 4 the heavy chain is reorganised and the light chain preserved. Choosing specific connecting residues by means of the distance tables yields the following exemplifications:
3a. L83-107/L13-57/H1-113
4a. H87-113/H13-64/L1-107

### Minimal Single Chain Constructs

The distance tables indicate that constructs 3 and 4, which consist of approximately 185 residues, may be reduced in size by:
a) Omitting sequence that spans key segments which are close in space. Thus in construct 3, section h1-20:H21-60 can be replaced by h1-6/H21-60 thereby saving fifteen residues. 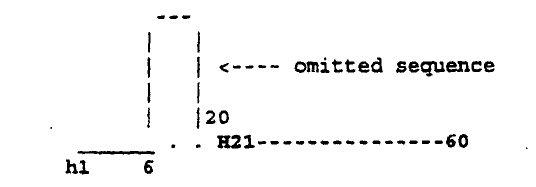
b) Splicing those candidate junctions which minimise the number of residues needed.

Thus in construct 4, h109-113/h13-20 can become h109/h15-20, a saving of six residues. 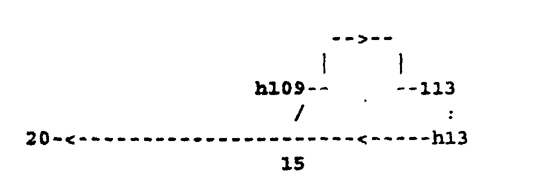 Such considerations lead to the following minimal single chain constructs of about 150 residues in which similar numbers of residues are contributed by the originating chains.
3m. L83-106/L15-57/H1-6/H21-60/H88-113
4m. H87-109/H15-64/L1-7/L21-63/L79-104

We now further illustrate the approach by adopting construct 4m (Figure 3) for further modelling analysis.

### 1.4 Alignment and V-min constructs

The alignment of F19.9 with 1MCP was performed within QUANTA (5) and topological consequences were checked using computer graphics

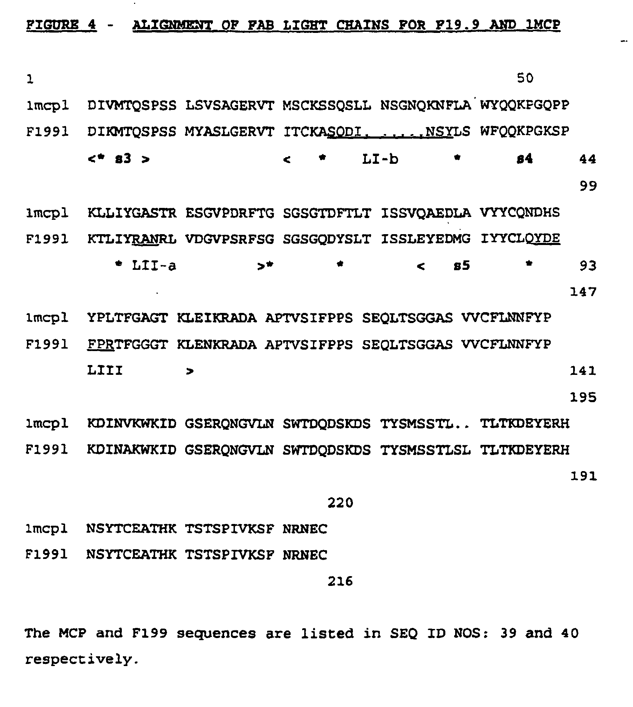

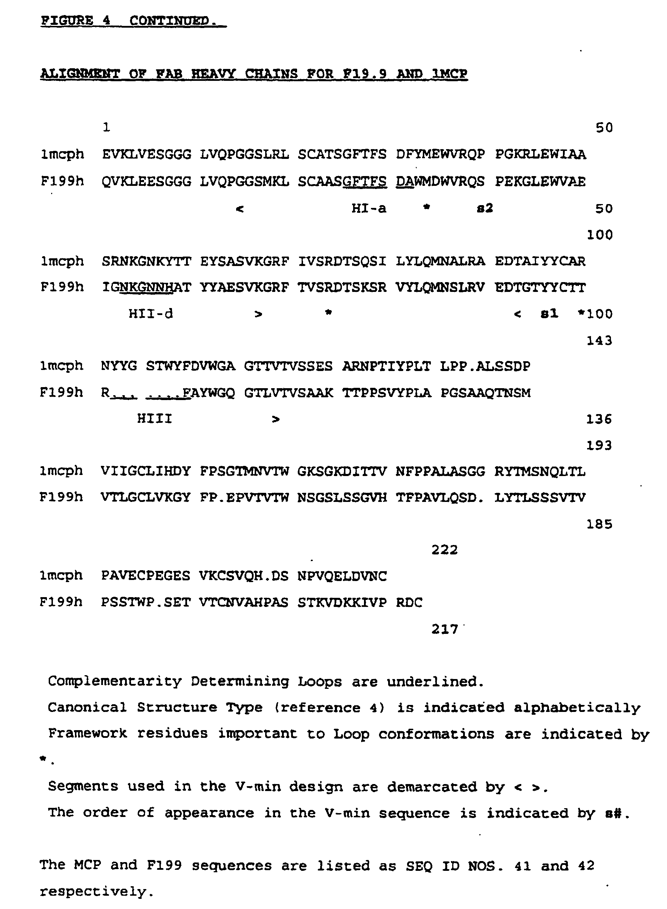

The F19.9/1MCP alignment is shown in Figure 4 and the V-min constructs are as follows :

| | s1 | s2 | s3 | s4 | s5 |
|---|---|---|---|---|---|
| Canonical | H87-H109 | /H15-H64 | /L1-L7 | /L21-L63 | /L79-L104 |
| 1MCP | H93-H118 | /H15-H67 | /L1-L7 | /L21-L69 | /L85-L110 |
| F19.9 | H93-H111 | /H15-H67 | /L1-L7 | /L21-L63 | /L79-L104 |

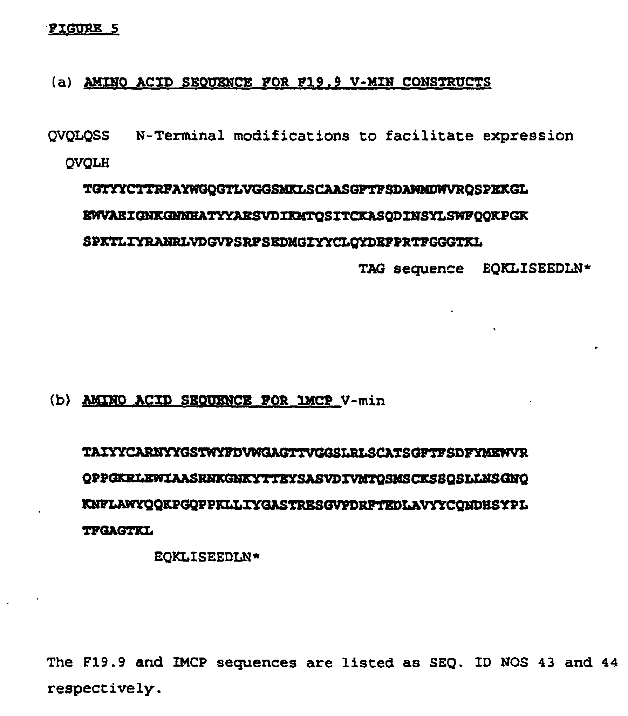

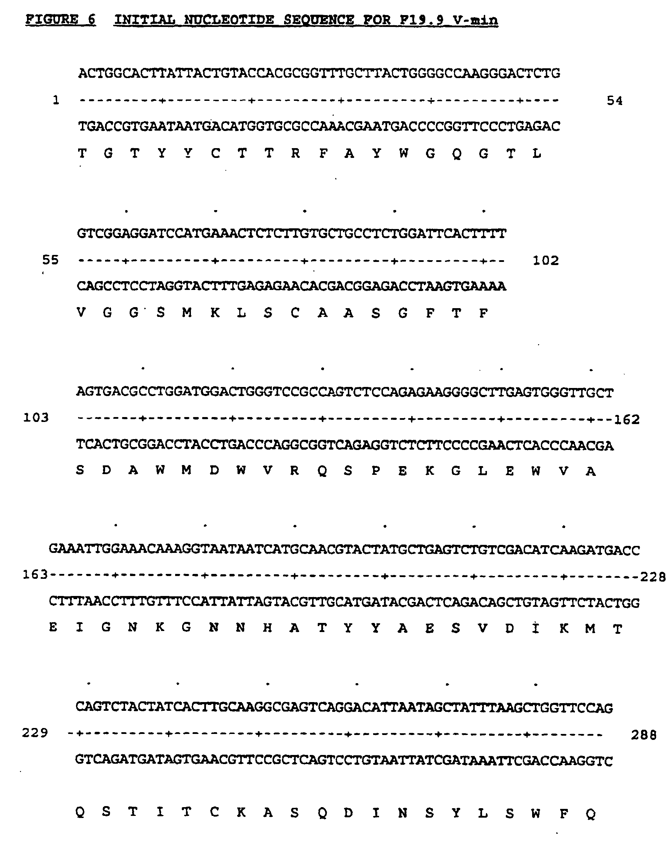

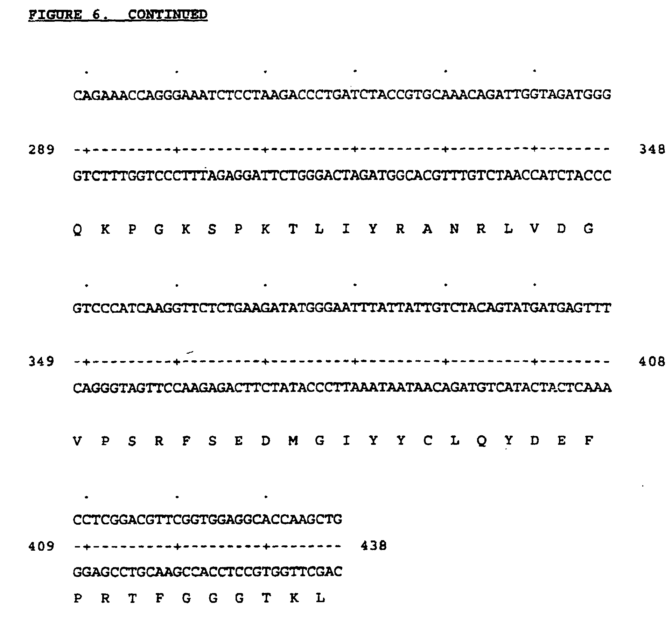

The resultant amino acid sequences are shown in Figure 5 with associated C-terminal TAG residues (used to aid subsequent detection in binding assays) and N-terminal modifications which may result from cloning into certain expression vectors. An initial nucleotide sequence for F19.9 V-min is shown in Figure 6.

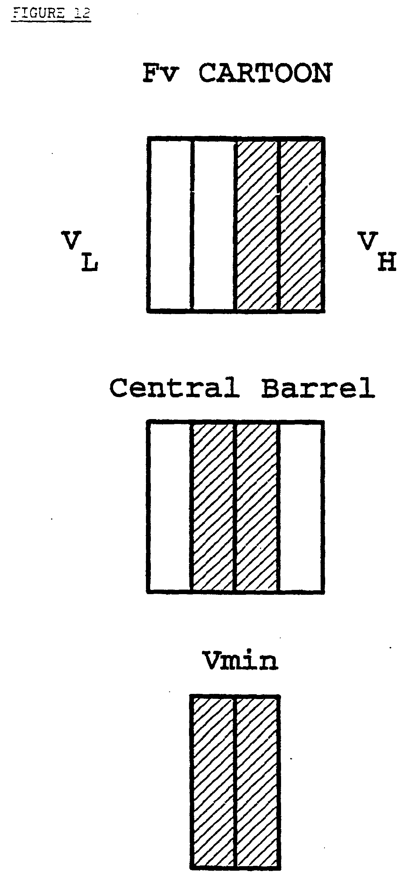

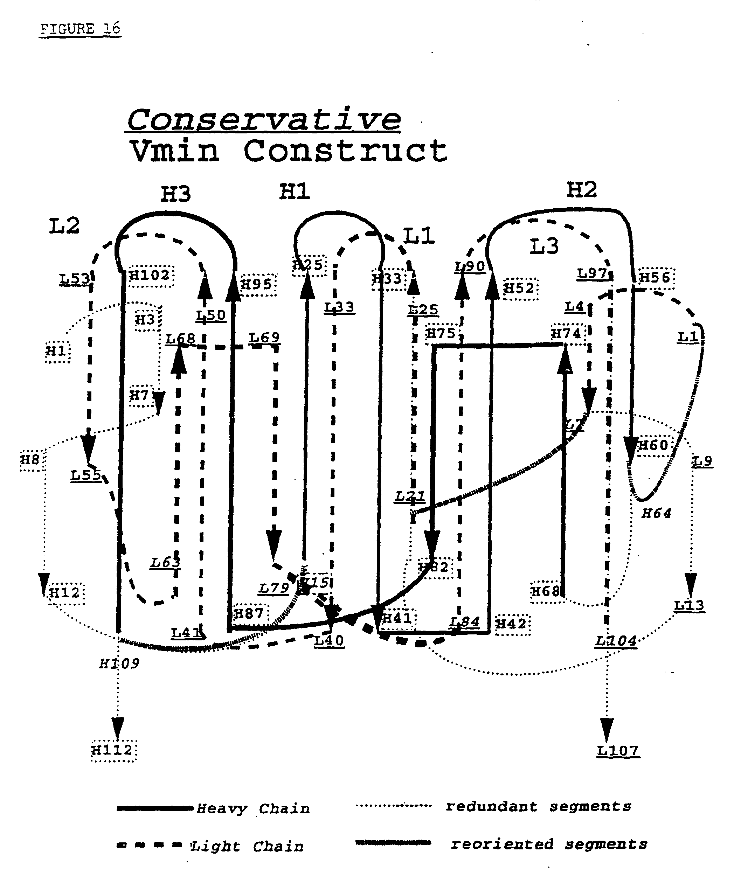

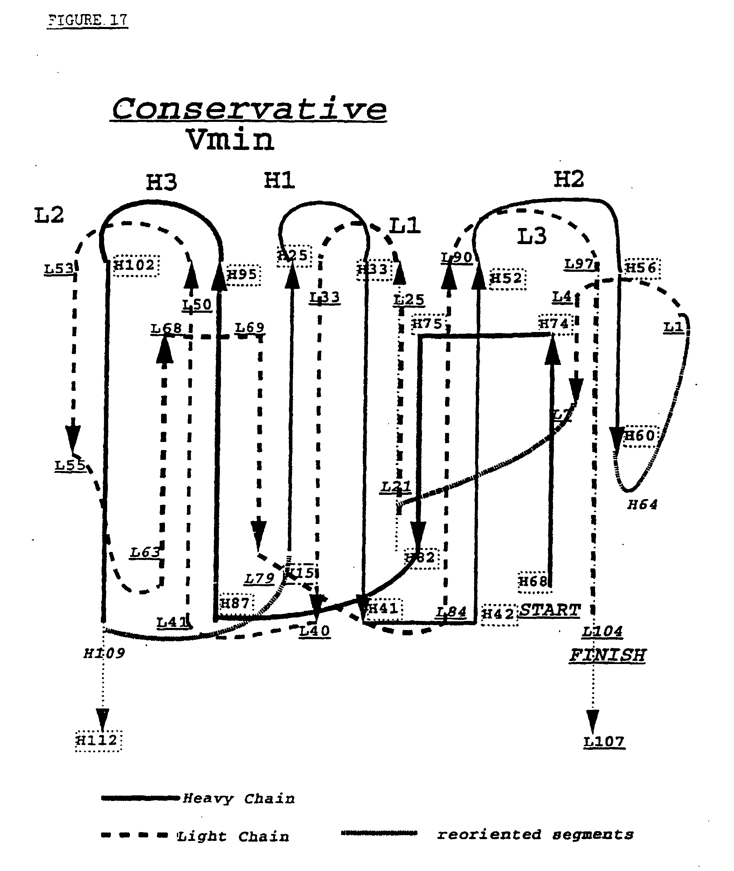

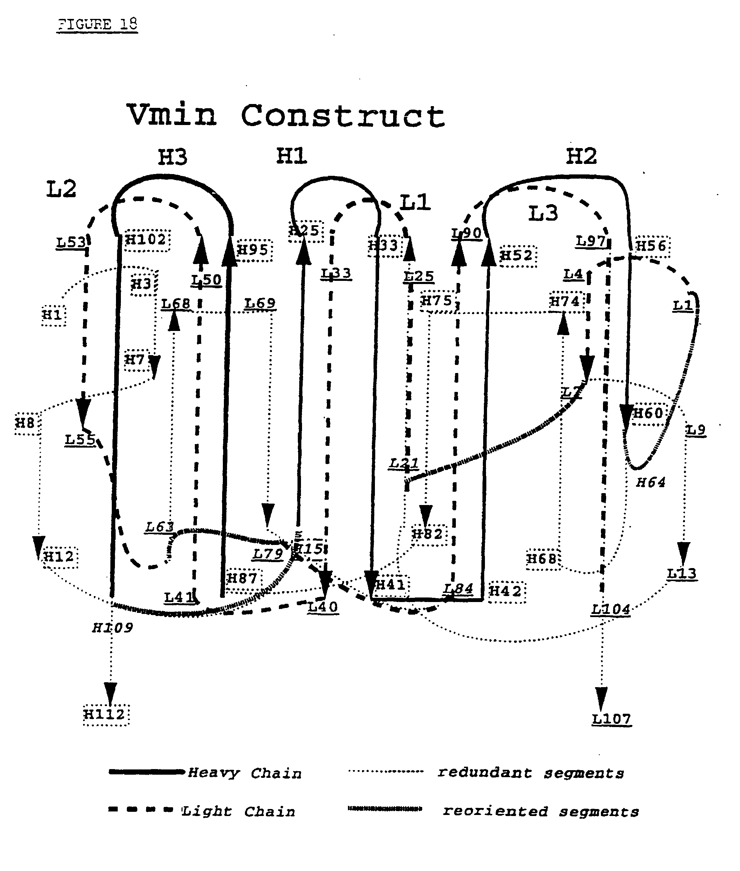

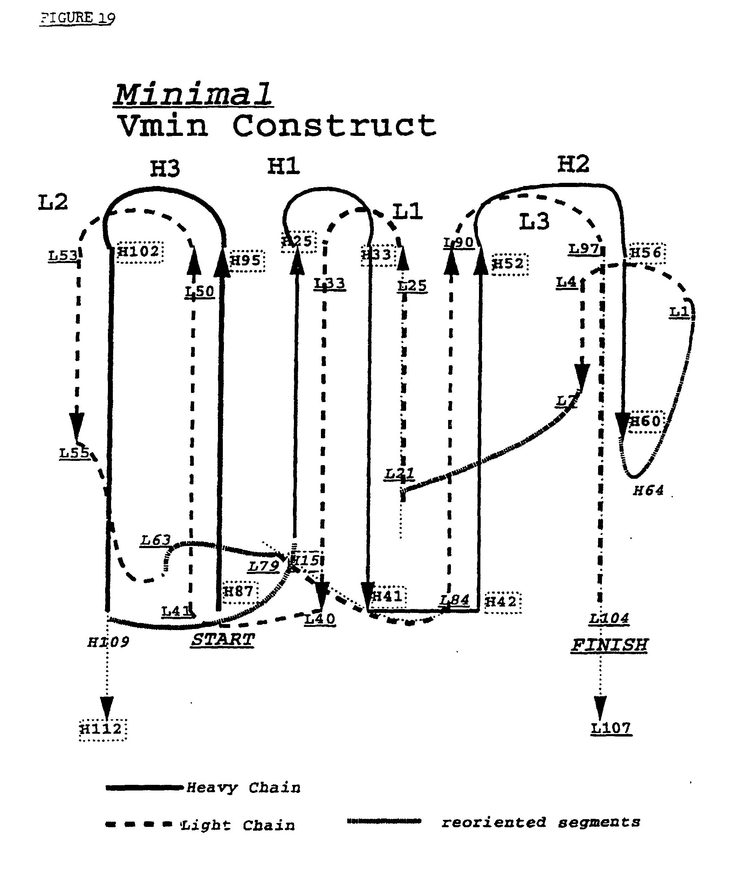

In Figure 4, the V-min segments, and the CDR's are indicated. In addition, certain framework residues which Chothia suggests (4) are important to CDR loop conformations are identified. The V-min construct shown above incorporates five of these residues, however three are omitted. Of these, L64 Gly (iMCP L70) (important to CDR LII) and L71 Tyr (IMCP L77 Phe) (important to CDR LI) could be restored by replacing s4 & s5 with L21-L104 (IMCP L21-L110).
Restoration of the remaining residue H71 Arg (H74 in F19.9 and 1MCP) which is important to the juxtaposition of CDR's HI and HII could be achieved by initiating the sequence earlier than H87, eg at H68 (H71 F19.9 and 1MCP). Alternatively, it may be possible to mutate eg H49 (H52 Asn in F19.9 and 1MCP) to reinforce the loop positions.
Further illustration of the V-min concept is provided in Figures 12 - 19.

### 1.5 Modelling the V-min Proteins

### 1.5.1 Introduction

The first step is to identify an X-ray structure of a Fab on which to base the model. In the case of 1MCP the X-ray structure has been determined. In general, the decision is based on the degree of identity between the sequence of known Fab structures and the target protein. Particular emphasis is placed on the relationship of the corresponding CDR loop sequences and their associated conformational determinants from the framework (4). Numerous Fab X-ray structures are available in the Brookhaven data base. The 1MCP Fab structure represents a viable starting point for the development of a homology model of F19.9. The Fv portions of the Fab structures provide useful reference points for evaluating the feasibility of the V-min models in terms of the residue environments etc.

### 1.5.2 1MCP V-min

The regions of the 1MCP structure which are not involved in the V-min construct were deleted using the QUANTA (5) utility. The remaining structure was output as a PDB file which was edited to the proposed V-min order. The resulting PDB file was imported into QUANTA as an initial V-min structure.

This structure required remodelling of the junctions between segments to create a connected structure. This was undertaken using an annealing protocol which can be generalised as follows:
Fix all atoms other than those in the annealing zone.
The annealing zone consists of ca four to six residues (eg two on either side of the proposed "join"). The size of this annealing zone is reflected in the 15 Â criterion for "adjacency" used earlier. Perform constrained optimisation using CHARMM (6) until residual gradient is less than 1 Kcal/Å
Check for torsion angle violations (eg cis peptides etc).
If necessary, incorporate appropriate dihedral angle constraints and re-optimise.

After all junctions were annealed, the resulting structure was energy relaxed in two stages, firstly, all side chain atoms were allowed to relax and this was followed by a full energy relaxation. The disulphide distances were constrained in these steps.

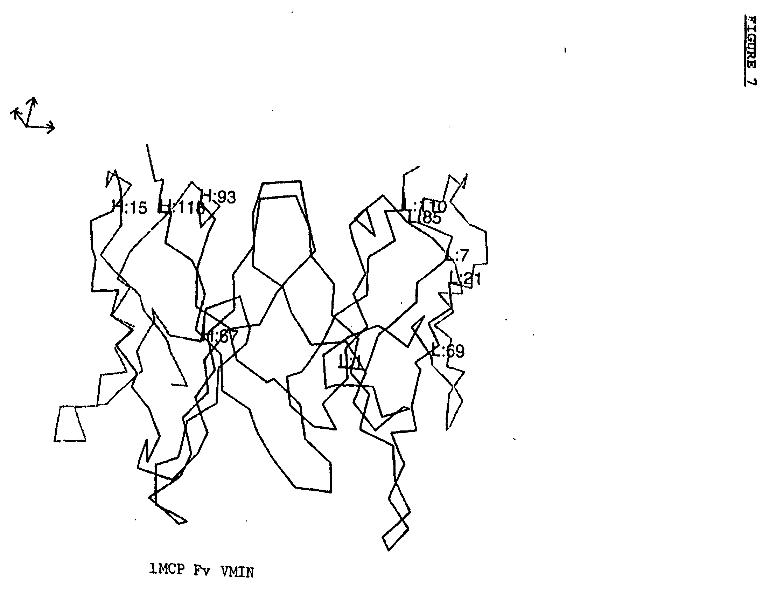

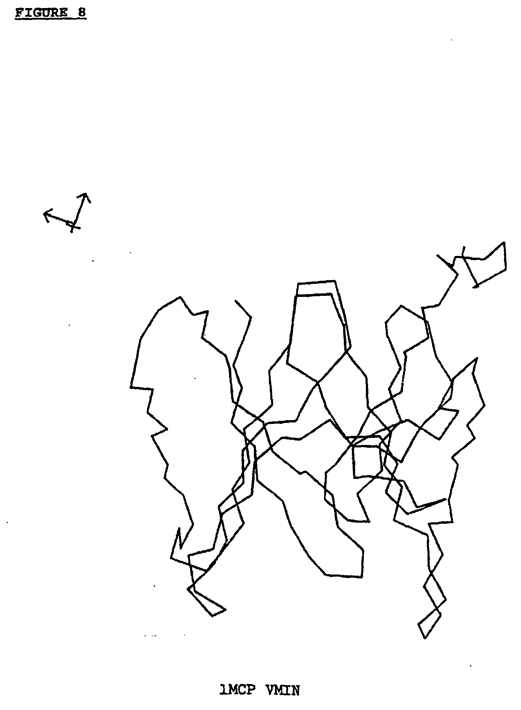

At this stage, the "tag" sequence was inserted after the C-terminal residue. The "tag" sequence plus the last two V-min residues formed an annealing zone and the protocol described earlier was followed. Additional distance constraints were applied to bring the non-polar residues in the tag sequence into contact.
A final full energy relaxation was applied and an α-carbon diagram is shown in Figure 8; a corresponding Figure 7 shows the V-min construct in bold and redundant sequence in faint line.

### 1.5.3 F19.9 V-min

The proposed amino acid sequence file for this construct + "tag" was generated and QUANTA utilities used to map the sequence onto the structure of 1MCP V-min + "tag" generated earlier. As can be seen in Figure 4, two deleted sections with respect to 1MCP occur in the CDR loops. Thus, two annealing steps were undertaken followed by energy relaxation of sidechains and, finally, of all atoms as described earlier for the 1MCP V-min model.

Two candidates for the N-terminal modification are indicated in Figure 5. The shorter candidate requires a glycine to be replaced by leucine. Initial inspection of the V-min model indicated that this could be problematic. Thus, the necessary mutations and insertions were accomplished using QUANTA and a zone consisting of the candidate sequence plus the next residue was annealed followed by energy relaxation.

### 1.5.4 Modelling The F19.9 FAB Structure

This model was required for comparative analysis with the F19.9 V-min construct. The alignment shown in Figure 4 formed the basis for mapping the F19.9 sequence onto the 1MCP structure using QUANTA. The deletion and insertion zones were annealed as described for the V-mins and the side chains were energy relaxed, followed by the full molecule also as described earlier.

### 1.5.5 Analysis of The V-min Models

The following criteria have been applied in assessing feasibility of the models, the residue numbering is that of the V-mins:

### 1.5.5 Potential Energies

The energy of the system should indicate a lack of steric clash, and internal strain. In the CHARMM force field using a distance dependent dielectric (4r), a general rule of thumb is that energies/residue should be in the range -4 ± 1 Kcal/mole.

| Potential Energy | | |
|---|---|---|
| | kcal/mole | kcal/residue |
| F19.9 V-min + tag | -556 | -3.6 |
| F19.9 V-min/tag/N-term | -543 | -3.4 |
| 1MCP V-min + tag | -689 | -4.1 |

### 1.5.5.2 Dihedral angles

Peptide omega angles (C'-N torsion) should be trans (180° +/- 20°). Cis peptides are allowed if they precede proline (7).
Main chain phi angles (N-Cα torsion) should be negative for L-amino acids. Positive values are acceptable for glycine residues (7).

| Positve Phi Angles | | |
|---|---|---|
| F19.9 V-min | 1MCP V-min | Comment |
| Gly 16 | Gly 23 | |
| Gly 21 | Gly 28 | |
| Gly 31 | Gly 38 | |
| Asn 62 | Lys 69 | positive in 1MCP Fab Xray structure |
| | Gln 100 | + in 1MCP; deleted in F19.9 |
| Gly 99 | Gly 112 | |
| Arg 108 | Gly 121 | + in 1MCP but G=>R in F19.9! |
| Gly 115 | Gly 128 | |
| Gly 142 | Gly 155 | |

| Cis Omega Angles | | |
|---|---|---|
| F19.9 V-min | 1MCP V-min | Comment |
| Phe 135 Pro 136 | Tyr 148 Pro 149 | cis in 1MCP Xray |

| Omega angles outside the range 180° +/- 20° | | | | |
|---|---|---|---|---|
| F19.9 V-min | w | 1MCP V-min | w | Comment |
| Asp 72 | 159 | Asp 79 | 159 | junction s2-s3 |
| Asp 123 | -152 | Asp 136 | -152 | junction s4-s5 |
| Leu 130 | 159 | Gln 143 | 159 | |
| Lys 144 | 153 | Lys 157 | 154 | junction s5-tag |
| | | | | |
| Glu 146 | 156 | Glu 159 | 156 | The following are |
| Ile 150 | 154 | lie 163 | 154 | residues from the |
| Ser 151 | 154 | Ser 164 | 154 | "tag" sequence and |
| Glu 153 | 152 | Glu 166 | 152 | less well modelled |

### 1.5.5.3 Hydrogen Bonds

For folded proteins main chain hydrogen bonds occur at an average frequency of 60 ± 20 for every 100 possible (8).

| H-Bonds/100 | | | |
|---|---|---|---|
| | All Main Chain | β-sheet | Turns |
| F19.9 V-min | 55 | 22 | 22 |
| 1MCP V-min | 57 | 25 | 21 |
| 1MCP Fab(Xray) | 59 | 31 | 15 |
| F19.9 Fab | 63 | 29 | 18 |

### 1.5.5.4 Exposed Non-Polar residues

In general the surface accessibility of non-polar residues should be less than half of that of a Gly-X-Gly tripeptide in the extended conformation (9). In addition, free energies of hydration for residues should be negative or zero.
For the present purpose, we report non-polar residues that are exposed and have positive hydration free energies (10), and which also differ significantly from the parent Fab model.

| F19.9 | Accessible Fraction | | | Hydration Free Energy (kcal/mole) | | | |
|---|---|---|---|---|---|---|---|
| | V-min | Fab | D | V-min | Fab | D | |
| **Val 19** | **0.1** | **0.0** | **0.1** | **0.6** | **0.0** | **0.6** | **junction s1-s2** |
| Val 53 | 0.2 | 0.0 | 0.2 | 0.3 | 0.0 | 0.3 | |
| Ile 56 | 0.4 | 0.0 | 0.4 | 0.3 | 0.0 | 0.3 | |
| **Val 71** | **0.5** | **0.0** | **0.5** | **0.7** | **0.0** | **0.7** | **junction s2-s3** |
| **Ile 79** | **0.8** | **0.0** | **0.8** | **0.1** | **0.0** | **0.1** | **junction s3-s4** |
| Ile 106 | 0.2 | 0.0 | 0.2 | 0.2 | 0.0 | 0.2 | |

| 1MCP | Accessible Fraction | | | Hydration Free Energy (kcal/mole) | | | |
|---|---|---|---|---|---|---|---|
| | V-min | Fab | D | V-min | Fab | D | |
| Val 19 | 0.5 | 0.1 | 0.4 | 0.7 | 0.2 | 0.5 | ?? |
| **Val 26** | **0.1** | **0.0** | **0.1** | **0.6** | **0.0** | **0.6** | **junction s1-s2** |
| **Val 78** | **0.6** | **0.0** | **0.6** | **0.8** | **0.0** | **0.8** | **junction s2-s3** |
| Leu 95 | 0.5 | 0.4 | 0.1 | 0.3 | 0.1 | 0.2 | |
| Leu 104 | 0.1 | 0.0 | 0.1 | 0.2 | 0.2 | 0.2 | |
| Ile 119 | 0.2 | 0.0 | 0.2 | 0.2 | 0.0 | 0.0 | |

### 1.5.5.5 Buried Polar Residues

If the accessible surface area of a polar residue is less than 10 Å² (ie that of a water molecule) (8), or if the free energy of hydration is positive, the environment must satisfy the hydrogen bonding requirements of the residue (11).

| F19.9 | Accessible Area Å² | | | Hydration Free Energy(kcal/mole) | | | |
|---|---|---|---|---|---|---|---|
| | V-min | Fab | D | V-min | Fab | D | |
| Thr 7 | 0 | 0 | 0 | 0.0 | 0.0 | 0.0 | |
| Asp 40 | 0 | 0 | 0 | 0.0 | 0.0 | 0.0 | |
| **Glu 55** | **2** | **1** | **1** | **2.6** | **-0.2** | **2.8** | **??** |
| Ser 92 | 0 | 1 | -1 | -0.1 | -0.1 | 0.0 | |
| **Lys 97** | **56** | **38** | **18** | **0.2** | **-1.2** | **1.4** | **??** |
| Ser 101 | 6 | 9 | -3 | -0.6 | -0.6 | 0.0 | |
| Gln 131 | 2 | 0 | 2 | -0.1 | 0.0 | -0.1 | |
| Arg 137 | 5 | 2 | 3 | -0.5 | -0.1 | -0.4 | |
| Thr 138 | 0 | 21 | -21 | 0.0 | 0.2 | -0.2 | |
| Thr 143 | 3 | 0 | 3 | -0.7 | 0.0 | -0.7 | |

| 1MCP | Accessible Area Å² | | | Hydration Free Energy (kcal/mole) | | | |
|---|---|---|---|---|---|---|---|
| | V-min | Fab | D | V-min | Fab | D | |
| Asn 9 | 1 | 0 | 0 | 0.0 | -0.1 | 0.1 | |
| Thr 14 | 0 | 0 | 0 | 0.0 | 0.0 | 0.0 | |
| Gln 47 | 0 | 0 | 0 | 0.0 | 0.0 | 0.0 | |
| **Lys 101** | **54** | **44** | **10** | **0.4** | **0.0** | **0.4** | **??** |
| **Gln 108** | **9** | **12** | **-3** | **-0.4** | **-1.3** | **0.9** | **??** |
| Gln 143 | 0 | 0 | 0 | 0.0 | 0.0 | 0.0 | |
| Asn 144 | 0 | 0 | 0 | 0.0 | 0.0 | 0.0 | |
| Asp 145 | 2 | 2 | 0 | -0.2 | -0.3 | 0.1 | |
| Thr 156 | 3 | 0 | 0 | 0.0 | 0.0 | 0.0 | |

### 1.5.5.6 Summary of Analysis

The potential energies and hydrogen bonding compositions of the V-min structures are in the expected range (8).
The only dihedral angle that may require further investigation is F19.9 V-min Arg 108 φ which is positive and is a Gly in 1MCP.
For both V-min structures, three non-polar residues are slightly more exposed than their Fab counterparts. These occur at junctions between sequence sections that have been connected in the modelling process.

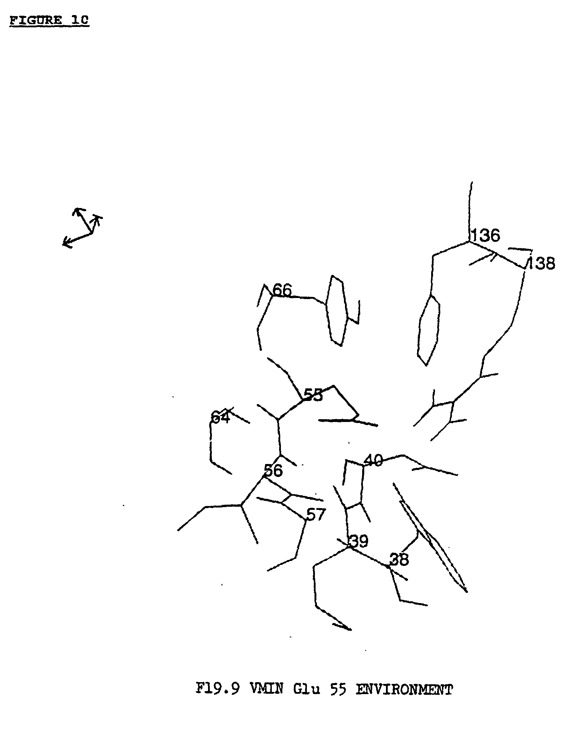

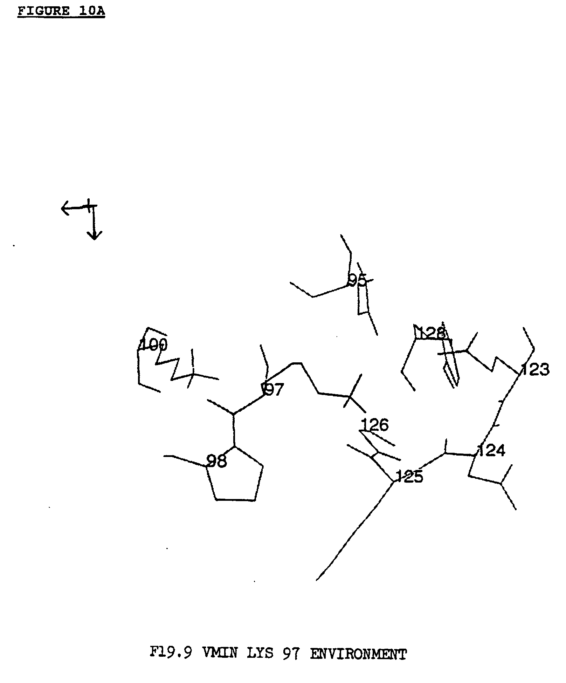

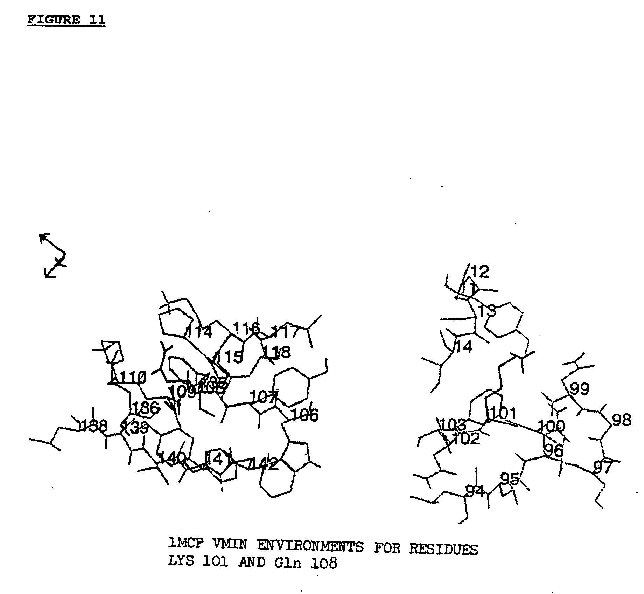

There are also slight distortions in peptide omega values in these regions. The deviations are relatively small and are unlikely to present major structural problems.
Similarly two polar residues have been identified in both V-min structures as possible problems, however inspection of their environments (Figures 10-11) suggests that the polar groups are adequately bonded.
In terms of these "modelling" criteria, both structures appear to be feasible.

### 1.6 Further V-min constructs

i) The restoration of potentially important framework residues was alluded to earlier and comprises a four segment construct:
   F!19.9 H71-111/H15-67/L1-7/L21-104
ii) If a cysteine were to be required at the base of the V-min in order to facilitate linkage to an effector protein, the presence of two disulphide bridges (F19.9 6-27, 81-129) may complicate purification, refolding etc. The disulphides are relatively buried and in an extended conformation which suggest the use of valine or alanine replacements.
iii) As indicated earlier, the core segments required to present the six CDRs can be chosen such that six core segments are used. This gives rise to additional combinatorial possibilities which can be evaluated as shown earlier for the four core segment approach.
iv) In addition it is possible to consider the generation of human Fv frameworks based on genetic families or clans (13). Mouse CDR's can then be grafted on taking into account the requirements for particular framework residues to control CDR loop conformation. The resultant Fv sequences can readily be converted to V-min constructs as shown earlier.
v) Minimal sized antigen combining molecules could be generated as two or more chains. The simplest such molecule would be of the type :
   Light Chain L83-106/L15-57 eg 67 canonical residues
   Heavy Chain H87-109/H15-64 eg 73 canonical residues for a total of 140.

### 1.7 Sequence for a conservative V-min based on D1.3 antibody

The Vmin invention involves the generation of a single-chain Fv-like recognition protein by altering the order of appearance of sequence segments that display the CDRs. By judicious paring of non-essential segments, the size of the protein can be reduced to as few as 160 residues. For a conservative construct, the aim is to conserve more of the original Fv and reduce the new splice junctions, thus resulting in a larger protein (for example upto 210 residues). One advantage of the conservative approach is the retention of framework residues which may influence CDR loop conformation or participate in induced fit responses to binding. With these considerations in mind analysis of the X-ray structure for D1.3 Fab resulted in the following conservative Vmin construct:-
H65-H115:H13-H64:L1-L108

The light chain segment remains intact and there are two splice-junctions where remodelling is required. A loop, taken from pseudoazurin (2paz 34-39) served as a template for the first junction and two loops (leucine binding protein 21bp 109-115, chymotrypsin inhibitor 2ci2 42-48) provided two possible conformations for the second junction. Thus two models have been generated, both are energetically and geometrically feasible and exactly correspond to the original Fv with the exception of the two splice-junction loops and the absent N-terminal residues from the heavy chain (H1-H12).

In comparison with the Fv, more non-polar surface is exposed (ca 130Å²), and much of this arises from the two leucines associated with the remodelled splice-junctions. There may be a case for replacing these leucines by alanine or serine to reduce the exposed non-polar surface (Figure 40). Example 4 herein sets out expression of a D1.3 V-min construct.

In sequence number 54 there is indicated on a D1.3 Vmin (conservative) an N-terminal secretion sequence (amino acids 1 to 22) as used by Winter et al (Nature 1989 341 544-546) in the successful cloning of Fv fragments used for crystallography (J.Mol.Biol. 1990 213 617-619). At the c-terminus there is included the c-myc tag sequence (amino acids 234 to 244) used previously. The changes to the splice junction loops are also indicated, but not the possible replacement of the leucines.

### EXAMPLE 2 GENERATION OF V-MIN EXPRESSION PLASMIDS

### 2.1 Design of an McPC603 V-min Synthetic Gene

We have designed an McPC603 V-min synthetic gene based on the protein sequence defined by the model. A major consideration was the use of E.coli preferred codons but it is possible to include other features without compromising this objective. The gene was designed for intracellular expression in E.coli using expression vectors set out herein but has inherent flexibilty for cloning into other systems such as secretion systems as set out in example 4 herein. Protease deficient strains of E. coli may be considered for expression.

### 2.1.1 Preparation of a V-min synthetic gene from antibody McPC603

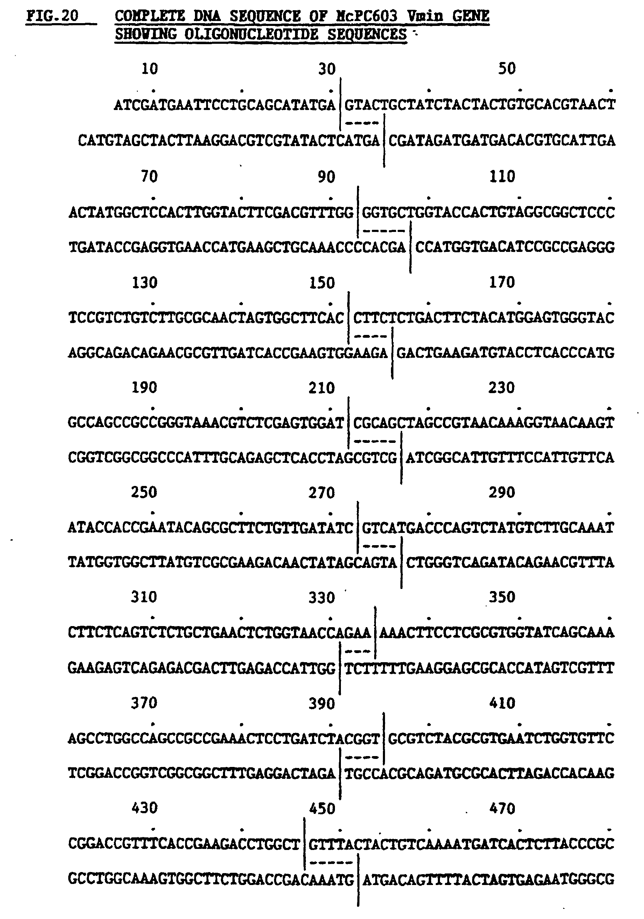

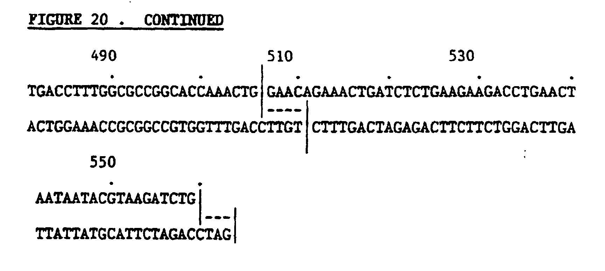

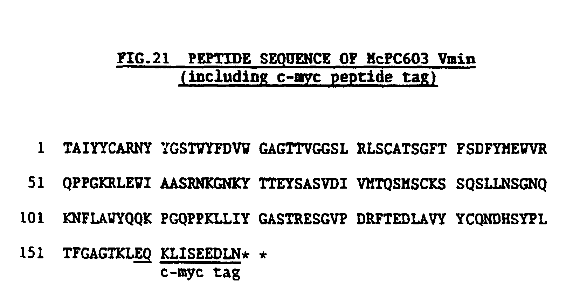

A DNA sequence (Figure 20) encoding the amino-acid sequence of the polypeptide of Figure 21 P1 which is based on the modelled structure for V-min was designed with the following features:
1) Codons normally chosen as those most commonly used in highly expressed E.coli genes.
2) A series of restriction endonuclease sequences flanking and throughout the gene to facilitate flexibilty in cloning and subsequent genetic manipulation.
3) Translation initiation and termination codons.
4) A sequence designed to minimise potential secondary structure within both component oligonucleotides and predicted messenger RNA.
5) Single-stranded cohesive termini to allow ligation at suitable sites within a plasmid.

### 2.1.2 Sequence compilation.

The amino-acid co-ordinates specified by the V-min model were used to compile the DNA sequences for the synthetic gene using existing DNA sequence data. The DNA/amino-acid sequence data for the Fv fragment of MCPC603 is published (Skerra et al, 1991, Bio/Technology 9, p273-278). The inclusion of a c-myc peptide tag (Ward et al, 1989, Nature 341, p544-546) at the C-terminus is useful for subsequent assay purposes. There are no antibodies available which would be capable of recognising the framework regions of a range of V-mins and any CDR specific antibodies would have restricted use and could interfere or be blocked in an assay.

The verified sequence was then checked for the presence of codons rarely used in E.coli ( according to Andersson and Kurland, 1990, Microbiol. Rev. 54, p198-210) and the relevant changes introduced.

### 2.1.3 Introduction of unique restriction sites.

To maximise the flexibility of the gene we attempted the introduction of unique restriction sites flanking each CDR region. This will allow the subsequent replacement/shuffling of the binding domains by a pair of complimentary oligonucleotides in the form of a restriction fragment. The premise was that the V-min framework may be the basis on which to build subsequent molecules with altered/alternate binding specificities. This could be in the form of modified or completely different CDRs. Such changes could be tested by protein modelling prior to any experimentation.
Using the MAP/SILENT programme in the University of Wisconsin GCG software package (Devereux et al, 1984, Nuc.Acids Res. 12 , p387-395, hereinafter refered to as "Wisconsin"), the V-min sequence was searched for potential restriction sites which could be introduced by codon changes but without causing amino-acid substitutions. From the results of all potential restriction sites a choice of unique sites flanking the CDR regions, which do not compromise the E.coli codon preference, was made.

Codon usage was again derived from this sequence and the relevant balance between the various codons as quoted in codon usage tables adjusted without compromising any of the introduced restriction sites.

### 2.1.4 Cloning strategy.

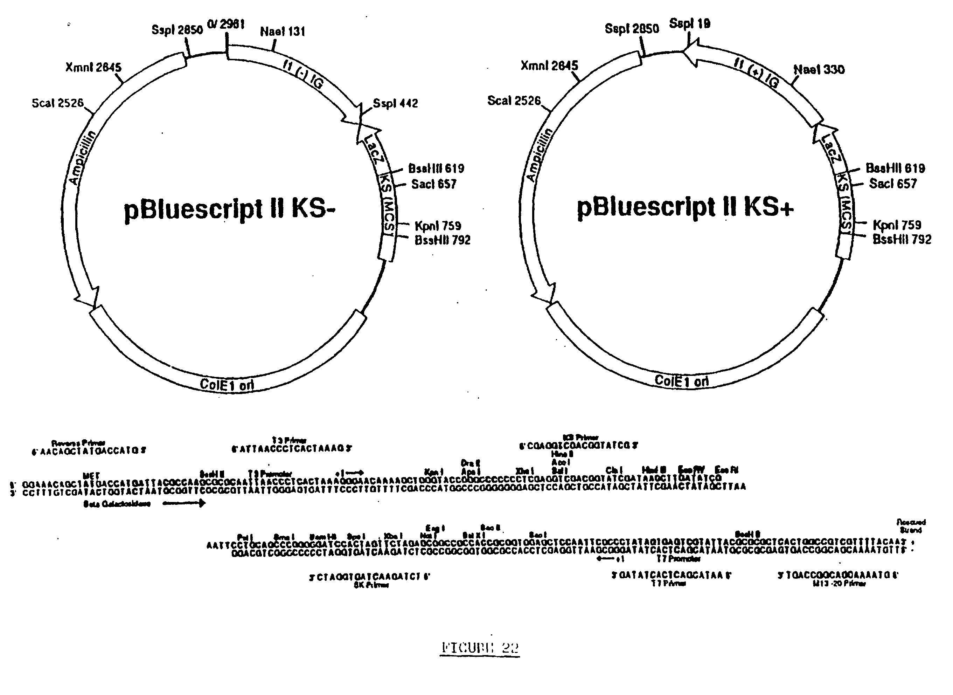

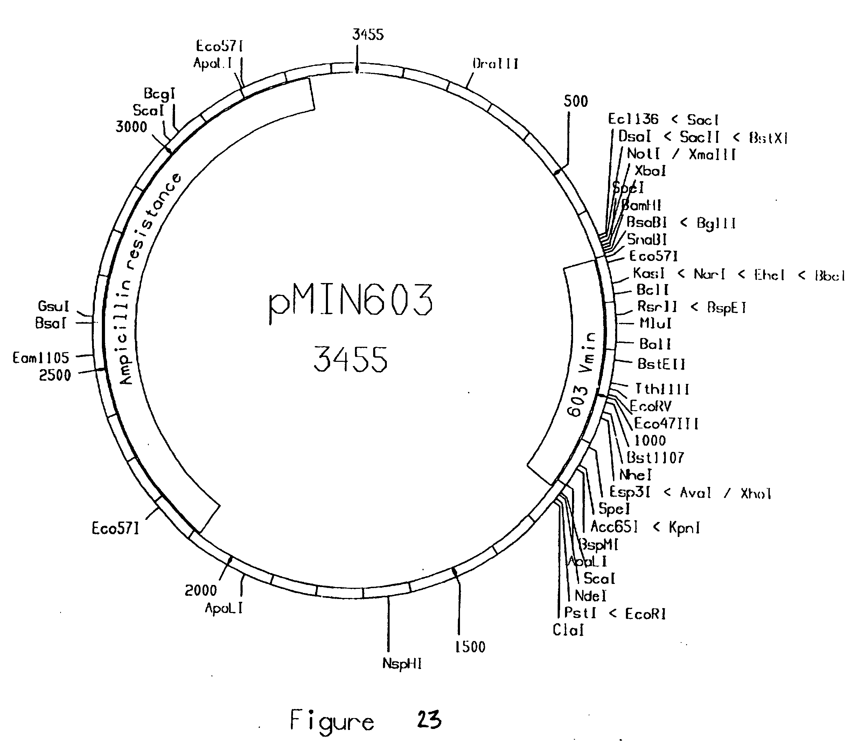

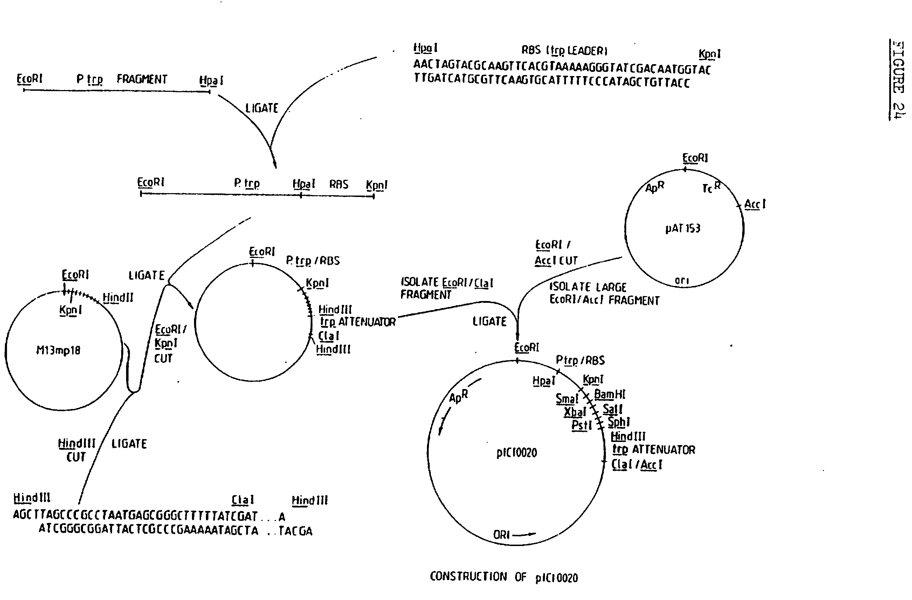

The phagemid vector pBluescript KS- (Short et al, 1988, Nuc.Acids Res. 16, p7583 and available from Stratagene) was chosen as the initial cloning vector to provide a permanent source of the gene. The complete nucleotide sequence of pBluescript KS- is available in the GenBank Genetic Sequence Data Bank, Bilofsky et al, 1986, Nuc.Acids Res. 14, p1-4, filename VecBase(3):BlueKSm, accession number VB0077 (Fig 22). This vector has the advantages of high copy number for ease of plasmid purification, colour selection for recombinants and the ability to generate single-stranded DNA which is useful for introducing subsequent manipulations via site-directed mutagenesis.

The manner of insertion into this vector can be achieved in non-expressing orientation.

The strategy allows for insertion of a synthetic V-min gene fragment between the kpnI and BamHI sites of the vector. By including additional unique restriction sites on the synthetic fragment which flank the gene, high flexibility for subcloning from this source is possible. After cloning into pBluescript, the majority of restriction sites within the gene remain unique.

### 2.1.5 Subcloning into expression vectors.

The gene can be cloned into pICI expression vectors (described below) on a ScaI (blunt-end) to XbaI restriction fragment between the KpnI (T4 polymerase treated to create a blunt end) and XbaI sites. The gene can be subsequently transfered to vectors with different promoters by simple restriction fragment swap such as for example Trp vectors.

The gene design also allows subcloning into any vector which makes use of an NdeI site to create the ATG initiation codon. In this case a single N-terminal amino-acid addition (Ser) would be created.

Cloning into E.coli secretion vectors would be useful in that any sequence modifications could be readily assessed in a binding assay. This would allow rapid generation and screening of several constructs. N-terminal amino-acid substitutions would result from cloning into the lac promoter/pelB leader vectors used for antibody fragment expression vectors (Gussow et al, 1989, Cold Spring Harbor Symp.Quant.Biol LIV, p265-272). These substitutions have been modelled and are not considered to significantly alter the folding of the molecule.

### 2.1.6 Translation initiation considerations.

A DNA sequence file was compiled for the V-min gene in trp and lambda_{PL} promoter vectors with two different ribosome binding site (RBS) sequences. In each case, the programme FOLD in Wisconsin was used to predict mRNA secondary structure over the first 100 bases of message. Whilst not entirely predictive, this programme can be employed to verify translation initiation problems and can alert the researcher to any potential problems with a given sequence. After running this programme minor changes were made to the 5' region of the gene to remove a potential stem-loop which reduced the availabilty of the AUG initiation codon. These changes did not compromise the previous design features. The new stem-loop predictions have a low free energy and allow good access to the initiation codon.

### 2.1.7 Secondary Structure Considerations

The gene was divided into the 20 oligonucleotides (Seq.ID No.1 - 20) shown in Figure 20/Table 2 on an arbitrary basis. The Wisconsin programme STEMLOOP was then used to identify potential secondary structures within the gene. These secondary structures can cause problems in the correct annealing of complementary oligonucleotide pairs during gene synthesis. Any significant stem-loop structure occuring within a particular oligonucleotide sequence was destabilised by base changes which did not compromise the main design considerations.

### 2.2 Preparation of oligonucleotides

The oligonucleotide sequences shown in Table 2 were prepared using known methods such as on an Applied Biosystems 380A DNA synthesiser from 5'dimethoxytrityl base-protected nucleoside-2-cyanoethyl-N,N-di-isopropyl- phosphoramidites and protected nucleosides linked to controlled-pore glass supports on a 0.2 µmol scale, according to protocols supplied by Applied Biosystems Inc. Alternative apparatus includes Applied Biosystems 380B or 394 DNA synthesisers using 5'dimethoxytrityl base-protected nucleoside-2-cyanoethyl-N,N-di-isopropylphosphoramidites and protected nucleosides linked to controlled-pore glass supports on a 0.2 µmol scale, according to protocols supplied by Applied Biosystems Inc.
Alternatively, the oligonucleotides can be prepared manually by the methods described by Atkinson and Smith in "Oligonucleotide Synthesis, a manual approach" (M.T.Gait, editor, IRL Press, Oxford, Washington DC, pages 35-81).

The purification of the oligonucleotide sequences subsequent to automated synthesis was as follows:
Each synthesised oligonucleotide, after cleavage from the solid support and removal of all the protecting groups, was dissolved in double distilled water (1ml). An aliqout (400µl) of each oligonucleotide was taken and the DNA precipitated by the addition of 50µl of 3M sodium acetate, pH6.5 and 1ml absolute ethanol. After incubation on ice or at -20°C for a minimum of 15 minutes, the precipitated DNA was pelleted by centrifugation at 13,000rpm for 10 minutes. The supernatant from each pellet was discarded and the pellet washed in 1ml of 70% ethanol (in water), re-centrifuging for 5 minutes. Pellets were then dried under vacuum before resuspending in 20µl distilled water and 5µl of a formamide/dye mixture (10mM NaOH, 0.5mM EDTA, 0.01% each of bromophenol blue and xylene cyanol and 80% formamide). The samples were then electrophoresed on a 10% polyacrylamide gel containing 8.3M urea in 100mM Tris-borate buffer, 2mM EDTA (pH8.3).

After electrophoresis, the full-length oligonucleotide product was excised from the gel after visualisation by UV-shadowing (Narang et al, 1979, in Methods in Enzymology, 68, pp90-98). This was generally the major product of the synthesis although products of intermediate size were also visible. The DNA was electro-eluted from the acrylamide in a dialysis bag containing 400µl of 10mM Tris-borate buffer, 0.2mM EDTA (pH8.3) at 300v for 2 hours. The eluate was collected and the DNA recovered by ethanol precipitation as described above. Alternatively, recovery of the DNA can be achieved by the use of proprietary kits such as Spin-X filters (Costar) with the protocols recommended by the manufacturers. The concentration of each purified oligonucleotide solution was estimated by scanning a diluted sample from 300 to 220nm in a UV spetrophotometer and using an extinction coefficient of 1OD₂₆₀=20µg/ml.

### 2.3. Gene Assembly

Oligonucleotides Seq.ID 2-19 (100-400pm of each) were phosphorylated with T4 polynucleotide kinase (50 units, Pharmacia Biosystems Ltd.) in a solution containing ATP (8µM), g-³²P ATP (50pM, New England Nuclear, 6000Ci/mmol), spermidine (100µM), MgCl₂ (20mM), Tris-HCl, pH9.0 (50mM) and EDTA (0.1mM) at 37°C for lhr. The effectiveness of the phosphorylation reaction was assessed by electrophoresing a 2µl sample of each reaction on a 10% polyacrylamide gel containing 8.3M urea in 100mM Tris-borate buffer, 2mM EDTA (pH8.3) followed by autoradiography of the gel. The reactions containing oligonucleotides seq.ID 10 and 20 were incubated at 70°C for 10 minutes to inactivate the T4 polynucleotide kinase thus preventing premature phosphorylation of the complementary partner upon mixing.

The phosphorylated oligonucleotides were then mixed in complementary pairs as shown in table 1 to produce the duplexes A to J. The two 5' oligonucleotide sequences (seq.ID 1 and 11) were used unphosphorylated (200pm in 25µl) to prevent concatomerisation of the final or intermediate gene products. The mixed oligonucleotide pairs were then ethanol precipitated as previously described. Each pair was resuspended in 100µl of distilled water and the complementary sequences annealed by heating to 100°C for 1 minute before gradually cooling to room temperature over several hours or overnight.

The annealed pairs were then mixed into 3 sets as shown in Table 2. The volume of sets II and III was increased to 400µl by the addition of 100µl of distilled water. Protein contamination was then removed from each set by treatment with Strataclean resin (Stratagene) in accordance with the manufacturer's instructions. Alternatively, this can be achieved by extraction against an equal volume of phenol. The DNA was again recovered by ethanol precipitation and the pelleted DNA dried under vacuum.

The DNA in each set was then ligated together by resuspension in 30µl of a solution containing T4 DNA ligase ( 1 unit; Bethesda Research Laboratories), 50mM Tris-HCl, pH7.6, 10mM MgCl₂, 5% w/v polyethylene glycol 8000, 1mM ATP and 1 DTT (BRL, Focus, 8, Winter 1986) at 15°C for a minimum of 1.5 hours. The ligated DNA from each set was again recovered by ethanol precipitation, resuspended in 20µl distilled water and 5µl formamide dye mix and each set electrophoresed on a 10% polyacrylamide/8.3M urea gel. Bands of the appropriate length (163-217 bases) were identified by autoradiography, excised from the gel and recovered by electroelution and ethanol precipitation. These products were then annealed, the sets pooled, the DNA recovered by ethanol precipitation and then ligated to generate the complete gene sequence in a repeat of the above processes.

Recovery of the intact gene sequence can be achieved by electrophresis of the ligation products on a 4% polyacrylamide/8.3M urea gel, autoradiography, excision of the appropriate bands, electroelution and ethanol precipitation. The recovered DNA then requires annealing to produce the double-stranded gene fragment. Alternatively, a sample (1µl) can be added to a polymerase chain reaction (PCR) containing oligonucleotide seq.ID Nos. 1 and 11 (0.25µM of each), dATP, dCTP, dGTP and dTTP (0.2mM of each), KC1 (50mM), Tris-HCl, pH8.3 (10mM), MgCl₂ (1.5mM), gelatin (0.01% w/v) and Taq polymerase (2.5 units, Cetus Amplitaq) and incubating at, for example, 94°C for 1.5 minutes, 55°C for 1 minute and 72°C for 1.5 minutes for 25 cycles plus 72°C for 5 minutes in a programmable heat controller (Techne PHC-1). The resulting single, amplified product can be purified from excess by spun column chromatography using a Miniprep spun column (Sephacryl S-400, Pharmacia Biosystems Ltd.) or other proprietary equipment in accordance with the manufacturer's instructions and recovered by ethanol precipitation.

Prior to cloning the isolated gene must be phosphorylated (as described for the individual oligonucleotides) to allow ligation with a suitably digested cloning vector.

### 2.4 Cloning the Synthesised Gene

The synthetic gene was designed with cohesive termini which allow insertion into a general cloning vector digested with the restriction enzymes KpnI and BamHI such as the phagemid pBluescript (see "Cloning Strategy" above and fig 21) or any other suitable phagemid or plasmid vector. Cloning into the KpnI site does not result in regeneration of a restriction site (GGTACA) whereas the BamHI site is retained. Additional restriction sites are included in the synthetic gene both 5' and 3' to the actual coding sequence to allow flexibilty in sub-cloning for expression.

A sample of pBluescript KS- (5µg) was digested with the enzymes KpnI (25 units, Bethesda Research Laboratories) and BamHI (24 units, Amersham International) in a 100µl solution containing 10mM Tris-acetate buffer, pH7.5, 10 mM magnesium acetate and 50mM potassium acetate (provided in 10x concentration as One-Phor-All buffer PLUS, Pharmacia Biosystems Limited). The reaction was incubated at 37°C for 1.75 hours. Control samples of the phagemid (0.5µg) were digested with each enzyme individually in a 10µl volume under identical conditions. After digestion, samples of each restriction digest (0.1 - 0.5µg) are electrophoresed on a 0.8% agarose gel in a solution containing 20mM Tris-acetate, pH8.0, 0.5mM EDTA at 100V for 30 minutes followed by staining with ethidium bromide (1µg/ml) and visualisation under UV illumination. Digestion with each enzyme in the double digest was confirmed by the presence of both major (2896bp) and minor (65bp) bands and by complete linearisation with each enzyme individually.

The double digest was then treated with Strataclean resin (Stratagene) to remove proteins using the protocol provided by the supplier and the DNA recovered by ethanol precipitation. The purified DNA pellet was resuspended in 25µl of distilled water and 5µl of a solution containing 25% sucrose, 5mM sodium acetate, 0.1% sodium dodecyl sulphate and 0.5% bromophenol blue before electrophoresis on a 3% NuSieve GTG agarose gel (FMC Bioproducts) in 20mM Tris-acetate, pH8.0 and 0.5mM EDTA at 80V for 1 hour. The DNA was visualised under UV illumination after staining the gel in ethidium bromide solution (1µg/ml), the major vector band excised from the gel and the DNA recovered by phenol, phenol/ chloroform and chloroform extractions by the procedures provided by the suppliers of the agarose. Alternative methods for isolation and purification of DNA fragments are provided in "Molecular Cloning - a laboratory manual", Maniatis, Fritsch and Sambrook, published by CSH laboratory, second edition 1989 and hereinafter referred to as "Maniatis" (Chapter 6, p22-35). After recovery, the DNA was resuspended in 10µl of distilled water.

Alternatively, isolation of the synthetic gene via PCR results in a product which can be cloned into a vector digested with a restriction enzyme which creates blunt ends such as SmaI digested M13mp10 (Messing, J., 1983, Methods in Enzymol.101, p20) available from Amersham International. M13mp10 can be purchased (from Amersham International) already digested with SmaI and de-phosphorylated with alkaline phosphatase to prevent re-circularisation. Alternatively any suitable vector can be digested with a restriction enzyme generating blunt ends and then treated with calf-intestinal or bacterial alkaline phosphatase using protocols provided by the supplier of the appropriate enzyme.

Cloning of the isolated synthetic gene was achieved by ligation with a sample (50-100ng) of the KpnI and BamHI digested pBluescript KS-vector in a solution containing T4 DNA ligase ( 1 unit; Bethesda Research Laboratories), 50mM Tris-HCl, pH7.6, 10mM MgCl₂, 5% w/v polyethylene glycol 8000, 1mM ATP and 1mM DTT which is incubated at 15°C for a minimum of 2 hours. Alternatively the PCR generated synthetic gene is ligated with the SmaI cut and dephosphorylated M13mp10 under similar conditions. After ligation, the DNA can be used to transform competent E.coli cells such as strain DH5α (a Dlac derivative of strain DH1, Hanahan, D., 1983, J.Mol.Biol. 166, p557-580) for pBluescript and strain JM109 (Yanish-Perron et al, 1985, Gene 33, p103-11 for M13mp10. The competent cells can be purchased from a number of suppliers such as Bethesda Research Laboratories or Stratagene. Alternatively, procedures for the generation and transformation of competent cells are provided in Maniatis, Chapter 1 p74-84 and Chapter 4 p33-38. The vectors used can transform E.coli cells to ampicillin resistance (100µg/ml) or in the case of M13mp10 to generate plaques due to growth inhibition of infected cells.

Having selected ampicillin resistant colonies or plaques, these can be propagated and DNA purified for analysis. With both vectors, double stranded phagemid or replicative form (RF) DNA can be recovered from cultured cell pellets. Alternatively single stranded DNA can be prepared by rescue of phage particles using helper phage (such as R408 described by Russell et al, 1986, Gene 45, p333-338) from phagemid containing cells or directly from the supernatants of cells infected with M13mp10. Standard protocols for the purification of double- and single-stranded DNA are provided in Maniatis (Chapter 1, p21-52 and Chapter 4, p26-32). Double-stranded DNA can be used for analysis by restriction enzyme digestion to identify clones with inserts of the appropriate size. Both types of DNA can be used for DNA sequence analysis by the dideoxy chain termination method of Sanger (Proc.Nat.Acad.Sci. USA 74, 1977, p5463) using a proprietary sequencing kit such as the Sequenase kit supplied by United States Biochemical Company and used in accordance with the protocols provided and analysed by autoradiography after high resolution polyacrylamide gel electrophoresis (Sanger and Coulson, 1978, FEDS lett.87, p107).

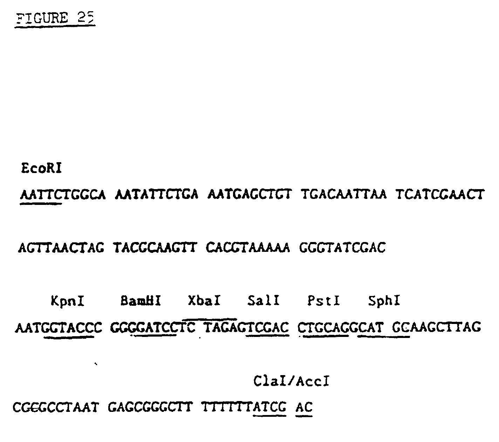

One plasmid clone identified as containing the gene sequence was named pMIN603. The construction of this plasmid is outlined in fig 23.

### 2.5 Construction of E.coli expression vectors

The V-min gene can be cloned into a number of E.coli expression vectors for intracellular accumulation. Such vectors can allow constitutive expression from a trp promoter (Bennet and Yanofsky, 1978, J.Mol.Biol., 121, p179-192) or regulable expression from a lambda P_{L} promoter under the control of a temperature sensitive CI857 repressor gene (Remaut et al, 1986, Methods in Enzymology 19, p366-383). Suitable alternative promoters can also be employed for this purpose.

Variants of these vectors can be prepared in which the only difference is in the DNA signal for translation initiation site or ribosome binding site (RBS). These variants can be compared for optimal accumulation level which can vary with different RBS sequences.

### 2.5.1 pICI0020

Plasmid vector pICI0020 is a pAT153 based plasmid vector in which the 651bp EcoRI-AccI region is replaced by a 167bp EcoRI-ClaI fragment consisting of:-
1) a synthetic E.coli trp promoter and trp leader ribosome binding site
2) a translation initiation codon
3) a multiple restriction enzyme recognition sequence derived from M13mp18 containing sites for KpnI, BamHI, XbaI, SalI, PstI, SphI and HindIII.
4) a synthetic transcription termination sequence.

The construction of a plasmid vector containing a synthetic trp promoter sequence is published (Windass et al Nuc.Acids Res. 10 p6639-6657, 1982). A promoter fragment was isolated from such a vector after digestion with the enzymes EcoRI and HpaI and purification of the appropriate band from an agarose gel by electro-elution (in Maniatis, Chapter 6 p28).

A pair of complementary synthetic oligonucleotides were prepared which would ligate to the HpaI end of the promoter fragment providing the natural trp leader ribosome binding site, a translation initiation codon and a 3' KpnI cloning site. These oligonuleotides were mixed in equimolar concentrations and allowed to anneal by heating to 100°C followed by slowly cooling to room temperature.

The promoter fragment and annealed oligonucleotides were then ligated and the appropriate band isolated from a polyacrylamide gel by electroelution. This fragment was then ligated with an M13mp18 vector derivative containing the trp attenuator sequence (generated from synthetic oligonucleotides) cloned into the HindIII site and introducing an additional ClaI restriction site 3' to the attenuator. The ligated DNA was transfected into E.coli strain JM109 (Yanisch-Perron et al Gene, 33, p103, 1985) made competent by the CaCl₂ method (Maniatis, chapter 1p82). After plating out and incubation of the plates, plaques were screened by the method of Benton and Davies (Maniatis, chapter 4p41) using a ³²P labelled probe generated by nick translation of the EcoRI-HpaI promoter fragment isolated previously. Single stranded DNA was prepared from positively hybridising plaques by a standard method (Maniatis, chapter 4p29) and sequenced using the M13 universal primer and the Sanger dideoxy chain termination method as provided in kit form by a number of suppliers eg. Sequenase (United States Bioscience).

RF DNA was prepared from one isolate in which the promoter/ribosome binding site/attenuator sequence had been confirmed. This DNA was digested with EcoRI and Clal and the appropriate fragment isolated from a polyacrylamide gel as above. Plasmid pAT153 (Twigg and Sherratt, 1980, Nature 283, p216-218) was digested with the enzymes EcoRI and AccI and ligated with the isolated promoter fragment. Ligated DNA was transformed into competent E.coli HB101 (Bethesda Research Laboratories) and ampicillin resistant colonies selected.

Plasmid DNA from several clones was prepared and DNA sequence derived from the region between the EcoRI and ClaI sites. One clone confirmed as containing the correct promoter/attenuator region was named pICI0020. This construction is outlined in fig. 24.

### 2.5.2 Preparation of Plasmid pCH101

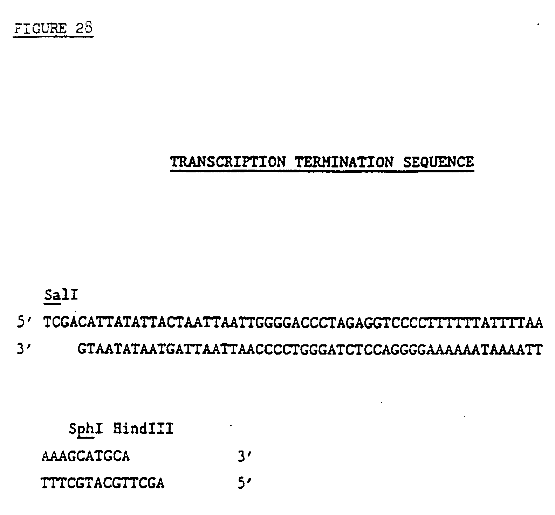

The plasmid pCH101 corresponds to pICI 0020 except that the EcoRI-SalI fragment is replaced by a fragment consisting of the SEQ ID No 37 (see Figure 25) and the interferon α₂ gene sequence as described by Edge M.D. et al, Nucleic Acids Research 1983, Vol11, p6419-6435.
In this regard the 3'-terminal ATG codon of SEQ ID No 37 immediately precedes the TGT codon which codes for cysteine (amino acid 1) in the interferon α₂ sequence of the above-mentioned Edge M.D. et al Nucleic Acids Research reference. The 5' nucleotide sequence GATCCATG and the complementary 3' nucleotide sequence GTAC are thus omitted from the nucleotide sequence of the aforementioned reference.

### 2.5.3 pICI0042

pICI0042 is a plasmid in which the antibiotic resistance markers of pAT153 have been replaced by a single, inducible tetracycline resistance gene from the plasmid RP4 (encoded by the gene tetA and regulated by the product of the tetR gene). These genes have been characterised by Klock et al (J.Bacteriol. 161 p326-332, 1985). A plasmid stability function (cer) has also been incorporated. This obviates the requirement for β-lactam antibiotics in cultures of cells bearing this plasmid. Because the new resistance marker is only expressed in the presence of antibiotic, the tetA gene product will not be a potential contaminant in cultures where the plasmid is stabily maintained in the absence of tetracycline.

The initial stage in the generation of this vector was to produce a derivative of pAT153 from which the gene encoding tetracycline resistance had been completely removed. A complementary pair of synthetic oligonucleotides were designed to replace the EcoRI-AvaI fragment from pAT153 with a short sequence containing several unique restriction endonuclease sites for subsequent cloning.

pAT153 plasmid DNA was digested with the enzymes EcoRI and AvaI and the 2.175Kbp plasmid DNA fragment isolated from a 0.7% agarose gel using Geneclean (Bio 101, California) in accordance with the manufacturers instructions. The 1.425Kbp fragment containing the tetracycline resistance gene is thus removed.

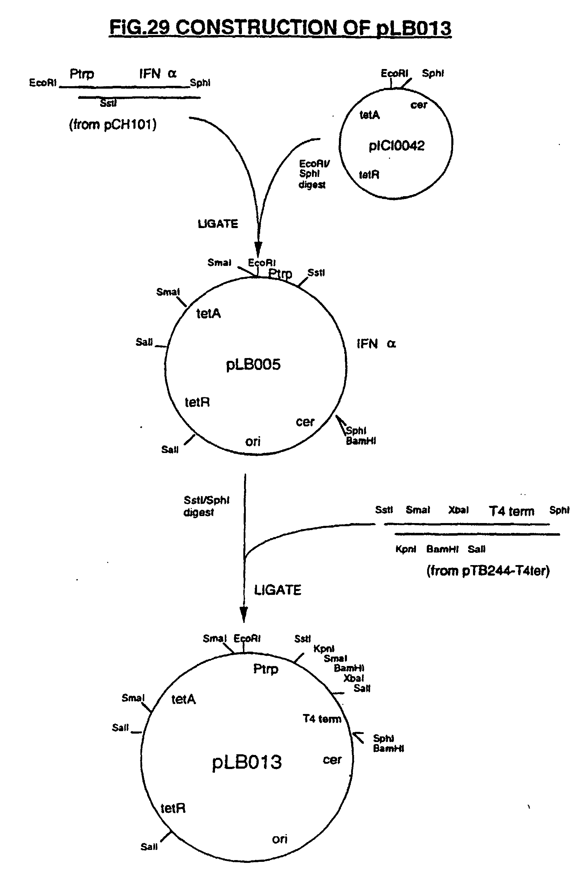

The oligonucleotides (fig 27/27A) were phosphorylated using T4 polynucleotide kinase and equimolar amounts annealed together. A sample of the annealed oligonucleotides was then ligated with the plasmid fragment from pAT153. Ligated DNA was transformed into competent E.coli HB101 (BRL) and ampicillin resistant colonies selected.

Several colonies were picked for small-scale plasmid DNA preparation by the method of Birnboim and Doly (as specified in Maniatis, chapter 1p25) and the desired construction identified by restriction analysis with suitable enzymes eg. EcoRI, AvaI and BamHI. The structure of 3 isolates identified as having the correct restriction pattern was confirmed by DNA sequence analysis using a pBR322 EcoRI site clockwise primer (New England Biolabs). One isolate was named pICI0019.

RP4 plasmid DNA was isolated from extant stocks by the method of Holmes and Quigley (Maniatis, chapter 1p29). This DNA was cut to completion with BglII and then partially with XmaI (at 25°C for up to 35min) taking samples at various timepoints until a 2.45 Kbp fragment containing the tetR and tetA was clearly identifiable. A sample of pUC8 DNA (Yanisch-Perron et al, 1985, Gene 33, p103-119 and available from Amersham International) was digested to completion with BamHI and XmaI. Ligations were performed to insert the tetracycline resistance genes into the pUC8. Ligated DNA was transformed into E.coli C600 (Appleyard, R.K. Genetics 39 p440, 1954) made competent by the CaCl₂ method (Maniatis, chapter 1p82) and tetracycline resistant colonies selected. Plasmid DNA was prepared from 8 clones (Holmes and Quigley) and the presence of the RP4 tetR and A genes confirmed by restriction analysis. One of these isolates was named pTB344.

The tetracycline resistance genes were then inserted into pICI0019 (described above) by replacement of an EcoRI/PstI fragment from pICI0019 with the corresponding fragment from pTB344. This results in replacement of the majority of the ampicillin resistance gene in pICI0019 with the tetracycline resistance genes. After digestion and ligation of the plasmid DNAs, followed by transformation into E.coli C600, colonies were selected on the basis of phenotype ie. Tc^{R} and Ap^{S}. Plasmid DNA was prepared from 4 such clones and digested with a combination of enzymes eg. BamHI/PstI/SstI, EcoRI/SalI, SmaI, StyI/SalI and AvaI/PstI. All 4 clones produced restriction patterns consistent with the desired construct. One of these was designated pTB351.

Summers and Sherratt (Cell 36 p1097-1103, 1984) have shown that the instability of plasmids derived from ColEI (eg. pAT153) is due to the loss of a 283bp sequence, cer, present in the parent plasmid. This sequence helps prevent the formation of plasmid oligomers, the latter appearing to disrupt plasmid partitioning in some as yet undefined way. The cer sequence (Summers, D. et al MGG 201, p334-338, 1985) was kindly provided by Prof.D.Sherratt in the form of a fragment cloned into pUC18 (pKs492). pKS492 plasmid DNA was digested with BamHI and TagI to release a 289bp cer-containing fragment. Plasmid pTB351 DNA (isolated from the dam host E.coli GM48 - Arraj, J.A. and Marinus, M.G. J.Bact. 153 p562-565, 1983) was digested to completion with BamHI and ClaI and ligated with the digested pKS492 DNA. After transformation of ligated DNA into competent E.coli C600, tetracycline resistant colonies were selected. Restriction analysis of plasmid DNA from putative clones with the enzymes AvaI, MluI and PvuI was used to confirm the presence of cer. One isolate with the correct structure was named pICI0042. This construction is outlined in figs. 26 and 27/27A.

### 2.5.4 Insertion of an Expression Cassette into pICI0042

An expression cassette consisting of the trp promoter, a ribosome binding site and the interferon α₂ gene was isolated from plasmid pCH101 (see 2.5.2 above) on an EcoRI to SphI restriction fragment. This was ligated into the vector pICI0042 (see above) similarly cut with EcoRI and SphI. This DNA was used to transform a competent culture of E. coli C600 and tetracycline resistant colonies were isolated. A few of these were tested by plasmid DNA preparation and restriction analysis for the acquisition of the SstI restriction site carried on the expression cassette. Clones positive in this respect were further tested by restriction mapping to check that the expected construct was correct. They were also checked for the conferred capacity to produce interferon α₂ protein as analysed on a polyacrylamide-SDS gel stained with Coomassie blue. One such confirmed clone was designated pLB005.

### 2.5.5 Insertion of T4 transcription terminator into pTB244

The T4 transcription terminator sequence in the form of the SalI to HindIII fragment (see SEQ ID No. 38 and Figure 28) was inserted into the multicloning site of an intermediate vector pTB244 (described in European Patent Publication No. 237,269) between its Sall and HindIII sites. Clone analysis was used to confirm the structure of this construct (pTB244-T4 ter). From this vector, an SstI to SphI fragment containing most of the multicloning site and the T4 terminator was then isolated. This was inserted into pLB005 similarly cut with SstI and SphI thereby substituting the interferon α₂ gene but leaving a cassette consisting of the trp promoter, multicloning site and T4 terminator. This construct was confirmed by clone analysis and the plasmid designated pLB013 (Figure 29).

### 2.5.6 Substitution of the multicloning site

The multicioning site in pLB013 is not ideal for this vector in several respects: the SalI, BamHI and SmaI sites are not unique, existing elsewhere on the plasmid. This fragment was therefore excised by cutting with SstI and XbaI (both unique) and synthetic oligonucleotides with the sequence of SEQ ID No. 40:- were inserted in its place. Clones were analysed for acquisition of the new restriction sites and then confirmed by sequencing. One such plasmid was designated pLB014. The new cloning sites inserted in this way are: NdeI, KpnI, BglII, XhoI and Scal with the previous XbaI and Sail following them.

### 2.5.6.1 Further modification

It was discovered that the adjacent SstI and NdeI sites in pLB014 could not be cut by both these restriction enzymes either simultaneously or sequentially presumably because of their close proximity. An additional sequence was therefore inserted between them. This was done by cutting pLB014 with SstI and KpnI and then inserting the synthetic oligonucleotide of SEQ ID No. 41:-

Clones were analysed for acquisition of an extra PvuII or PstI site and then confirmed by sequencing. One such plasmid was designated pLB015 (= pICI0080). This plasmid, unlike pLB014, is efficiently cut by SstI and KpnI or NdeI. This is to provide a place to insert a variety of ribosome binding site sequences correctly positioned with respect to the upstream trp promoter and by digesting with KpnI and T4 DNA polymerase treatment to generate a blunt end or by digesting with NdeI, designed to provide the ATG start codon of the gene to be expressed.

### 2.5.7 Alternative promoters

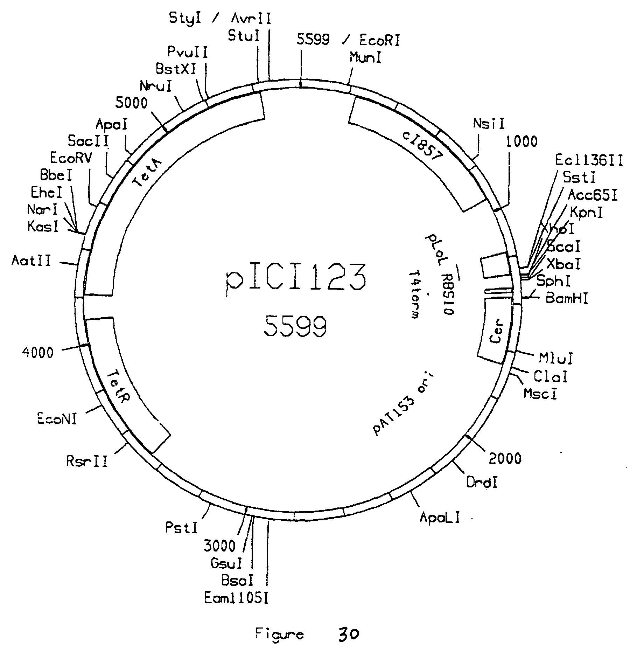

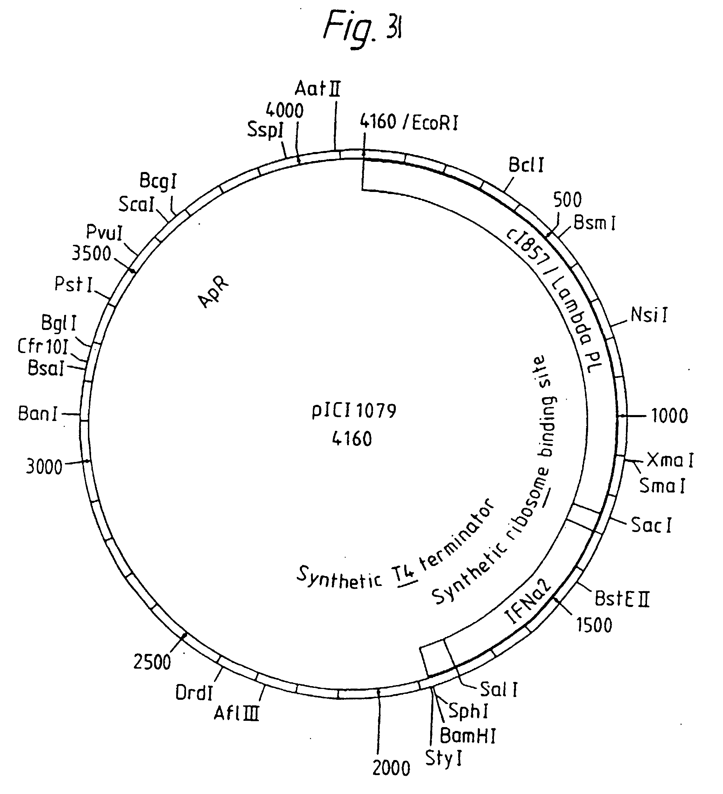

The trp promoter in pICI0080 is excisable on an EcoRI to SacI restriction enzyme fragment and can be replaced by a suitable alternative promoter element such as the regulable lambda P_{L}/cI857 element from plasmid pICI1079 (Figure 31) which has been deposited under the Budapest Treaty at the National Collections of Industrial and Marine Bacteria Limited (NCIMB), 23 St. Machar Drive, Aberdeen, AB2 1RY, Scotland, U.K. (NCIMB No. 40370, date of deposit 19 February 1991). A version with lambdaP_{L} promoter is called pICI123 (Fig 30).

Other E.coli promoter elements available on a compatible restriction fragment could also be substituted.

### 2.6 Sub-cloning the Synthetic V-min Gene into an expression vector

The cloned synthetic gene in pMIN603 contains a number of unique restriction sites which can be used for sub-cloning the V-min coding sequence into an E.coli (or yeast) expression system. The enzyme ScaI digests at the 5' end of the gene to generate a blunt-ended fragment which starts at the N-terminal amino-acid codon (ACT=thr). This can be fused to an expression plasmid which provides transcription and translation signals (promoter and ribosome binding site) and can be blunt-ended immediately after a translation initiation codon (ATG=met). Examples of such vectors are the pICI expression vectors described above. Alternatively, the unique NdeI site can be used to create a cohesive 5' terminus which can be cloned into an identical site of an expression vector such as pICI0080 described above. In this case the translation initiation codon is provided on the synthetic gene fragment and expression results in an N-terminal extension (AGT=ser) on the V-min protein.

E.coli expression vectors which allow fusion to a secretory leader sequence may also be used such that the expressed V-min protein is directed to the E.coli periplasm ( as described by Skerra et al, 1991, Bio/Technology 9, p273-278). In addition, secreted protein can be fused to the C-terminal portion of a bacteriophage coat protein (as described by Hoogenboom et al, 1991, Nuc. Acids Res. 19, p4133-4137) to allow the generation of phage particles which display the V-min protein in active form on their surface as an aid to selection. These approaches may require the modification of 5' and 3' restriction enzyme sites by, for example, PCR as described in the cited examples. A review of the expression of antibody fragment molecules in E.coli has been published by Pluckthun, 1991, Bio/Technology 9, p545-551.

The synthetic gene contains unique restriction sites for SnaBI, BglII, BsaBI and BamHI after the translation termination codon (TAA) to allow flexibility for cloning the 3' terminus into a range of expression vectors. In addititon, pMIN603 has additional cloning sites for XbaI, NotI, BstXI, SacI and BssHII 3' to the gene to further improve flexibility.

Expression vector pICI123 was digested with KpnI (Bethesda Research Laboratories) in a solution containing 20mM Tris-HCl, pH7.4, 5mM MgCl₂ and 50mM KCl at 37°C for 2 hours. The KpnI digested DNA was then treated with T4 DNA polymerase (1 unit/µg DNA Amersham International) in the above solution containing 0.1mM each of dATP, dCTP, dGTP and dTTP at 12°c for 15 minutes. This treatment makes use of the 3' to 5' exonuclease activity of the T4 polymerase to remove the 4 base 3'extension generated by KpnI digestion resulting in a blunt end immediately after the translation initiation codon. The reaction is then treated with Strataclean resin (Stratagene) to remove protein and the DNA recovered by ethanol precipitation. The DNA was then digested with XbaI (Bethesda Research Laboratories) in a solution containing 6mM Tris-HCl, pH8.0, 6mM MgCl₂, 150 mM NaCl and 1mM dithiothreitol (DTT) at 37°C for 2 hours followed by treatment with Strataclean resin to destroy enzyme activity and recovery of the DNA by ethanol precipitation. The treated DNA was resuspended in 10µl of distilled water.

The plasmid pMIN603 was digested with ScaI (Pharmacia Biosystems Limited) in a solution containing 20mM Tris-acetate, pH7.5, 20mM magnesium acetate and 100mM potassium acetate at 37°C for 2 hours.
The reaction was then treated with Stratclean resin and the DNA recovered by ethanol precipitation. The purified, ScaI digested DNA was subsequently digested with XbaI as described for pICI123 above. The protein is removed by Strataclean resin treatment and the DNA recovered by ethanol precipitation. The digested DNA was then eletrophoresed on a 3% NuSieve GTG agarose gel and a DNA band corresponding to the MCPC603 V-min fragment (523bp) isolated from the gel as described previously for the isolation of KpnI/BamHI digested pBluescript KS-. The purified DNA fragment was resuspended in 10µl of distilled water.

A sample of the KpnI/T4 polymerase treated/XbaI digested pICI123 (approx. 50ng) was ligated with the ScaI/XbaI digested V-min gene fragment (50-100ng) as described previously under "Cloning the Synthesised Gene". Ligated DNA was used to transform competent E.coli strain CGSC 6300 to tetracycline resistance. Plasmid DNA was prepared from selected tetracyline resistant colonies according to the methods in Maniatis previously refered to. Restriction digestion analysis is used to identify plasmids which contained a cloned insert consistant with the size of the McPC603 V-min gene. The cloning of the gene was then confirmed by DNA sequence analysis.

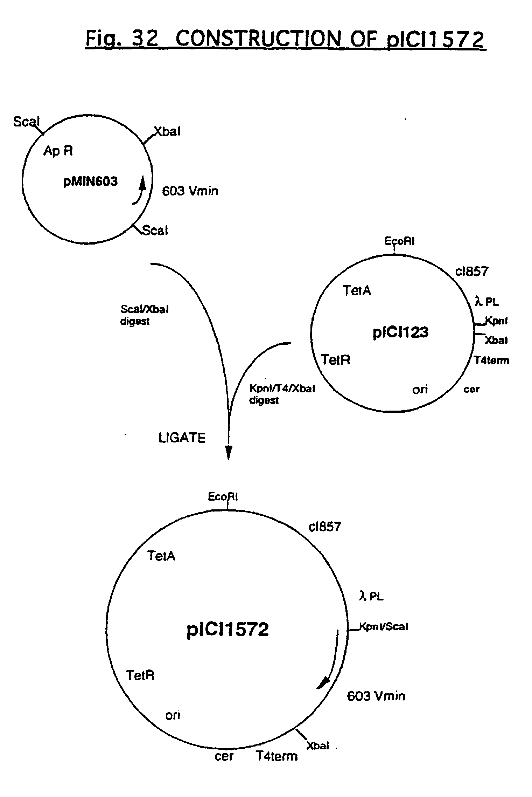

One cloned plasmid with the correctly inserted V-min gene sequence is named pICI1572. The construction of this plasmid is outlined in fig.32.
Alternatively, similar techniques can be used to clone the V-min gene into other vectors for intracellular accumulation, secretion to the periplasm or for display on the surface of a bacteriophage.

### 2.7 Expression studies

Plasmid pICI1572 is used in expression studies. It is used to transform a range of E.coli host strains, some of which are protease deficient, to assess accumulation levels in different genetic backgrounds.

Cultures (75ml) are grown in M9 medium (12.8g/l di-sodium hydrogen orthophosphate septahydrate, 3g/l potassium di-hydrogen orthophosphate, 0.5g/l sodium chloride, 1.0g/l ammonium chloride) plus 0.2% (w/v) glucose and 0.02% (w/v) casein hydrolysate, in the presence of tetracycline (15 µg/ml), shaking at 37°C for 7-16 hours.
Expression is induced by increasing the growth temperature to 42°C for 2 hours prior to harvesting the cells. The OD₅₄₀ of the culture is measured in a spectrophotometer, the cells pelleted by centrifugation at 1000x g for 5 minutes and the cell pellet is resuspended in a solution containing 62.5mM Tris-HCl, pH6.8, 1% (w/v) sodium dodecyl sulphate, 10% sucrose, 50mM DTT and 0.05% (w/v) bromophenol blue to OD₅₄₀=10 and incubated at 100°C for 15 minutes. A 20µl aliquot of each sample is then used for analysis by sodium dodecyl sulphate-polyacrylamide gel electrophoresis (SDS-PAGE) analysis as described in Maniatis (Chapter 18, p47-55).

Expression of the McPC603 V-min gene is detected by the presence of an additional band in cells containing clone pICI1572 compared to cells containing the parent vector (pICI123) on Coomassie-blue stained gels. Alternatively, the incorporation of ³⁵S methionine into newly synthesised proteins can be used to identify additional proteins expressed by pICI1572 clones compared to non-expressing control clones. The identity of additional proteins can be confirmed by electrophoretic transfer of proteins from SDS-PAGE to a nylon or nitrocellulose membrane using a Pharmacia Multiphor electro-blotting apparatus in accordance with the manufacturer's instructions followed by Western blot analysis using an anti-c-myc tag antibody (9E10 as described below) and detection by standard procedures (Maniatis, Chapter 18, p69-75) or Auroprpbe BL+ (Amersham International) using the protocols provided with these reagents.

### 2.8 Recombinant V-min Extraction and Isolation

Recombinant V-min expressed in E. coli is produced in
either soluble form (particularly by manipulation of expression construct and growth conditions) or as insoluble inclusion bodies. Secretion methodology can also be used (see Example 4).

Purification of soluble forms of the protein is accomplished for example by cell lysis (sonication or high pressure homogenisation), centrifugation and then separation techniques known in the art in a variety of combinations. Known separation techniques include the following:
ion-exchange chromatography; gel permeation chromatography; ammonium sulphate precipitation; acid precipitation and;
immobilised antigen affinity chromatography (for example, for McPC603 Vmin see reference : Glockshuber, R. et al, 1990, Biochemistry 29, 1362-1367, and references contained therein).

Renaturation and purification of insoluble V-min is accomplished by cell lysis (sonication or high pressure homogenisation), centrifugation, washing of the pellet fraction with buffer or mild chaotropes, solubilisation in chaotropic agents (eg. 6 M guanidine HCl, 8 M urea, detergents etc with or without reductant being present) followed by the controlled removal of the chaotropic agent (eg. by dilution or dialysis) and formation of the disulphide bonds by oxidation reactions (eg. using dissolved air, glutathione redox buffers, etc). The resulting solubilised, renatured V-min is purified as above.

For example, E. coli paste, containing McPC603 Vmin inclusion bodies, was resuspended in lysis buffer (15 % sucrose, 50 mM (ethylenediaminetetraacetic acid, hereafter abbreviated to EDTA, 50 mM Tris(hydroxymethyl) aminomethane hydrochloride, pH8.0) to an optical density of 50 (550 nm wavelength). Lysozyme was added to a final concentration of 1 mg/ml and the suspension was left on ice for 30 minutes. The sample, on ice, was sonicated in 4 x 45 second bursts and the resulting lysed cell suspension centrifuged at 25000 g for 30 minutes. The pellet, containing the Vmin inclusion bodies, was resuspended in phosphate buffered saline and recentrifuged at 25000 g for 30 minutes.

The washed inclusion bodies were dissolved in a urea solution (8 M urea/50 mM potassium phosphate/50 mM beta mercaptoethanol, pH 7.4) and the resulting solution clarified by centrifugation. The clarified solution was dialysed against 50 mM Tris (hydroxymethyl) aminomethane hydrochloride, pH 10.5 overnight at 4oC. The resulting solution was clarified by centrifugation and the supernate, containing the solubilised Vmin, further dialysed against phosphate buffered saline and then purified utilising methods outline above.

### 2.9 Activity assays

General details of activity assays are given in 3.2 below. In the assay for McPC603 V-min with c-myc tag, binding material suitable for the McPC603 antibody is used in place of the Colo205 cells used for 19.9 V-min. An example of a binding material suitable for McPC603 V-min assay is a phosphorylcholine-protein conjugate (17). In this case steps 1, 2 and 3 of the 19.9 V-min assay are replaced by:-1. 100µl of a phosphorylcholine-protein conjugate solution (typically 10µg/ml but depending on the particular conjugate) in 0.05M carbonate/bicarbonate buffer pH9.6 is added to each well and incubated at 4°C for 16 hours in a damp atmosphere.

An alternative assay procedure, provided appropriate tyrosine residues are maintained within the Vmin structure, utilises the change in fluorescence observed on the binding of the phosphocholine hapten to the Vmin as described for McPC603 antibody fragments in reference : Glockshuber et al, 1990, Biochemistry 29, 1362-1367 and references contained therein.

An alternative rapid qualitative assay for McPC603 binding to phosphocholine is provided by the selective binding of Vmin protein to immobilised phosphocholine matrices as described in reference : Glockshuber et al, 1990, Biochemistry 29, 1362-1367.

### 2.10 Generation of Vmin genes using Polymerase Chain Reaction (PCR)

All or part of the Vmin gene sequence may be generated by the use of suitable primers and DNA sequence amplification using Polymerase Chain Reaction. Two primers are required for each part of the Vmin gene generated by PCR. Other regions of the gene can be generated by chemical synthesis, as detailed elsewhere in this document, by restriction enzyme isolation of suitable gene fragments, or by other standard molecular biology techniques, as deemed most appropriate for the particular Vmin gene sequence under consideration. The individual fragments of the Vmin gene may then be joined together by further rounds of PCR reactions, or by other standard molecular biology techniques.

For example, in a typical Vmin construct consisting of two sequences derived from the Vh region of the heavy immunoglobulin chain of a specific antibody, joined to a sequence derived from the Vl of the same antibody, all or any of the three Vmin regions may be derived by PCR. The specific sequence of the Vmin having been determined by molecular modelling, as detailed elsewhere in this document, and the Vh and Vl nucleic acid sequence of the original antibody being available, in a molecular biology vector suitable for PCR amplification. In the case where the whole Vmin is to be generated by PCR, then primers are designed for the amplification reactions. For a three region Vmin construct six primers are required, two for each domain. In addition other primers may be required if it is neccessary to alter the native DNA sequence of the original antibody in the Vmin regions, in order to change specific codons as dictated by the peptide sequence of the modelled Vmin, or to change the codon usage to be compatible with the final Vmin expression system. Other functions or DNA sequence may be added to the Vmin sequence, for example restriction sites or a peptide leader or tag, either during the construction of the gene or during or by cloning into a succession of molecular biology vectors for maintenance, sequencing or expression of the cloned nucleic acid sequence.

Primer design for the PCR of the Vmin regions is of particular importance. Primers are made to be complementary with the DNA sequence on either side of junction of the domain regions of the Vmin. Primers complementary to the ends of the Vmin may be extended if necessary to give features to aid the cloning of the Vmin sequence into the chosen molecular biology vector. Thus a Vh sequence having four DNA domains, A-BC-DE-FG-H, and a Vl having three domains, I-JK-LM-N, which give a Vmin consisting of the three domains E-FC-DK-L, in that order, where A-B for example is a 5' to 3' DNA sequence from the 5' end of the Vh, and A and B are sequences of about 20 nucleotides from each end of A-B. Thus the three pairs of primers to generate the three domains of the Vmin, with suitable overlaps, are : -
1. E (with 5' addition for cloning if required) and the complement of FC, to generate E-FC by PCR from the Vh,
2. FC and the complement of DK, to generate FC-DK by PCR from the Vh,
3. DK and the complement of L (with 5' addition for cloning if required), to generate DK-L by PCR from Vl.
The three PCR fragments are then purified by standard Molecular Biology techniques, and may then be used to generate the Vmin, or intermediates by further rounds of PCR.

For example :-
1. E-FC mixed with equimolar FC-DK, and primers E and complement of DK, in PCR will generate intermediate E-FC-DK. This when purified can be mixed with equimolar DK-L and primers E and complement of L, in PCR to generate the complete Vmin, E-FC-DK-L. After purification this may be cloned into a suitable vector.
2. It is possible that the Vim may be generated by mixing E-FC, FC-DK and DK-L together in equimolar amounts with primers E and complement of L in a PCR.
3. It is also possible that the Vmin may be generated by mixing all of the initial components together in a PCR. That is, Vh and Vl approximately equimolar, with primers E, FC, DK and complements of L, DK and FC.

It may be necessary to test several combinations of the possible PCR reactions and to vary PCR conditions and component concentrations to find the best reaction parameters for any specific Vmin generation and set of primers.

Any of the PCR generated Vmin fragments may be substituted by chemically synthesised double stranded DNA or restriction fragments or DNA fragments obtained by other molecular biology techniques, in order to generate the specific Vmin by PCR or other suitable molecular biology technique.

### EXAMPLE 3

### 3.1 Design Synthesis and Cloning of a Synthetic V-min Gene for Antibody 19.9

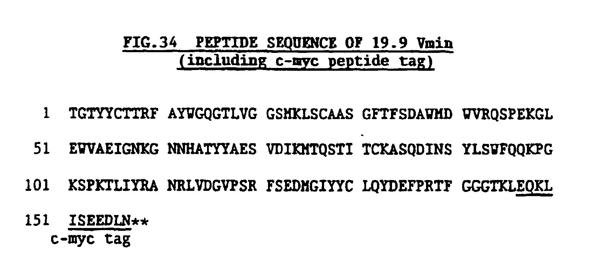

The amino-acid sequence for a V-min protein modelled for the antibody 19.9 is shown in fig.34. We have previously determined the complete DNA sequence of 19.9 heavy and light chain cDNAs (Tonge, D.W. et al, (1993), The Year in Immunology, vol 7, eds. Terhorst, C., Malavasi, F. and Albertini, A., published by Karger AG, Basel, Switzerland:
Proceedings of Biotech RIA 92 - New Generation of Monoclonal Antibodies in Diagnosis and Therapy, Genoa, Italy, April 12 - 15, 1992, published by Karger AG (Basel, Switzerland)) and used this information to compile a DNA sequence capable of encoding the amino-acid sequence shown.

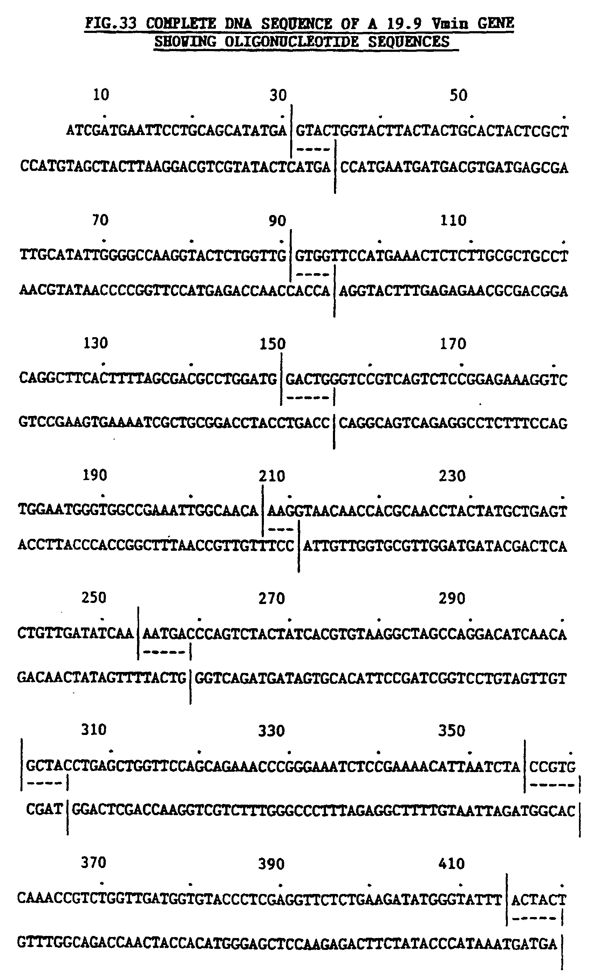

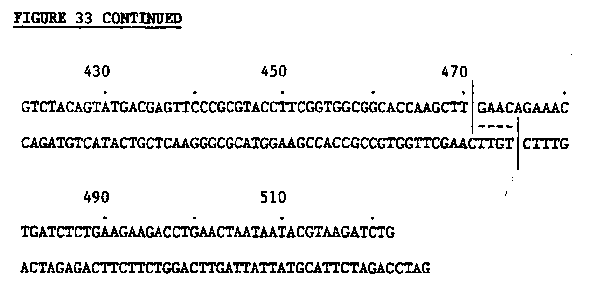

A synthetic V-min gene for antibody 19.9 was designed according to the principles described for the McPC603 V-min gene. The cloning regions and tag sequence which flank the coding sequence were identical to those used for the McPC603 synthetic V-min gene to maintain standard cloning strategies. The oligonucleotide sequences (ID No.21-36) used to construct the gene are shown in fig.33.

Gene synthesis, cloning steps and expression studies were performed according to the protocols stated for the McPC603 V-min gene.

### 3.2 Activity assays.

### 3.2.1 V-min detection and assay

V-min is detected by antibody reaction to a specific epitope, on the V-min per se, or on a peptide tag added to the V-min during the cloning procedures. Detection is in the form of immunoassay, by Western blot, dot blot, Elisa or other antibody based detection system. An estimate of V-min concentration may then be made by comparison to standard material detected by the same system.

For example, V-min is detected in bacterial supernatant by Western blot. Following separation of the protein components by polyacrylamide gel electrophoresis, and electrophoretic transfer to a suitable membrane (for example nitro-cellulose), the presence of V-min is detected by first blocking further non-specific binding to the membrane with a suitable protein solution (for example casein), and then reaction with a V-min specific antibody (for example a monoclonal antibody specific for a peptide tag on the V-min). The presence and location of the V-min is identified by a label on the V-min specific antibody (for example an enzyme reaction with a suitable chromophore) or by a sandwich detection system employing a labelled antibody or other binding molecule reactive with the first V-min specific antibody.

A similar competition system is used to estimate V-min concentration. V-min is bound to microtitre plates, excess binding sites blocked with a suitable protein, and the binding of a V-min specific antibody (either epitope or tag reactive) to the bound V-min competed by the unknown concentration V-min solution (for example bacterial extract), at suitable dilutions. Standard solutions of V-min are also used to generate assay curves. A reduction in antibody binding is detected by a label on the V-min specific antibody (for example an enzyme reaction with a suitable chromophore) or by a sandwich detection system using a labelled antibody or other binding molecule reactive with the first V-min specific antibody. Examples of the methods may be found in the laboratory manuals of Sambrook et al (14) or Harlow and Lane (15).

### 3.2.2 Identification and estimation of V-min binding to 'antigen'

The binding of V-min to the antigen of the antibody from which the V-min CDR sequences were derived, or binding to a suitable derivative or substitute for that antigen (for example a peptide sequence identified from a protein antigen, or a glyco-lipid identified from a cellular antigen, or a mimotope) is determined by immunoassay (14,15) or direct physical methods (for example using the Pharmacia Biacore apparatus or McPC603 Vmin fluorescence).

For example 19.9 V-min with a c-myc C-terminal peptide tag sequence (EQKLISEEDL) is estimated in its binding to antigen in the form of the colo-recta carcinoma cell line, Colo205 (ATCC CCL 222), by reaction with the monoclonal antibody 9E10 (16). In a typical assay : -
1. 10⁵ Colo205 cells (grown in serum free medium) in 100µl of PBS are added to each well of a microtitre plate and left at room temperature for 10 minutes.
2. The plates are then centrifuged at about 400g for 5 minutes at 10°C, and left to equilibrate at room temperature for 30 minutes.
3. 100µl of 0.1% glutaraldehyde is added to each well to fix the cells, and the plates left at room temperature for 10 minutes.
4. The plates are washed 3 times with PBS, leaving the wells full for five minutes for each wash. The final wash is emptied and the plates banged dry on paper towelling.
5. Excess binding sites in each well is blocked by the addition of 200µl of a 1% BSA solution in PBS and incubation for one hour at room temperature.
6. The plates are washed 3 times with PBS, leaving the wells full for five minutes for each wash. The final wash is emptied and the plates banged dry on paper towelling.
7. 100µl of 19.9 V-min solution, of unknown concentration or standards, at a suitable range of dilutions in PBS is added to the wells and incubated in a damp atmosphere at 4°C for typically 16 hours.
8. The plates are washed 3 times with PBS, leaving the wells full for five minutes for each wash. The final wash is emptied and the plates banged dry on paper towelling.
9. 100µl of 9E10 hybridoma supernatant at a suitable dilution in PBS, typically 1/30, is added to each well and incubated for 2 hours at 4°C.
10. The plates are washed 3 times with PBS to which 0.05% Tween 20 may be added, leaving the wells full for five minutes for each wash. The final wash is emptied and the plates banged dry on paper towelling.
11. 100µl of an anti-mouse IgG labelled with horse-radish peroxidase, at a suitable dilution (for example Sigma Chemicals Co. product A9044 at a dilution of 1/2000) is added to each well and incubated at 4°C for 2 hours. This second antibody is tested for its non-reactivity with V-min.
12. The plates are washed 3 times with PBS to which 0.05% Tween 20 may be added, leaving the wells full for five minutes for each wash. The final wash is emptied and the plates banged dry on paper towelling.
13. To generate a suitable chromophore, the contents of one capsule of phosphate-citrate buffer with sodium perborate (Sigma p4922) are dissolved in 100ml of water according to the manufacturers instructions and 30mg of o-phenylenediamine dihydrochloride (OPD) added and dissolved. 100µl of this solution are added to each well, and incubated in the dark for up to 30 minutes, until the colour develops.
14. 50µl of 25% sulphuric acid is added to each well to stop the reaction, and the absorbance of each well is read at 490nm.
15. Standard curves are then prepared, and the binding concentrations of unknowns is estimated.

Similar methods may be found in the laboratory manuals of Sambrook et al (14) or Harlow and Lane (15).

### EXAMPLE 4 Generation of D1.3 V-min (conservative) using PCR

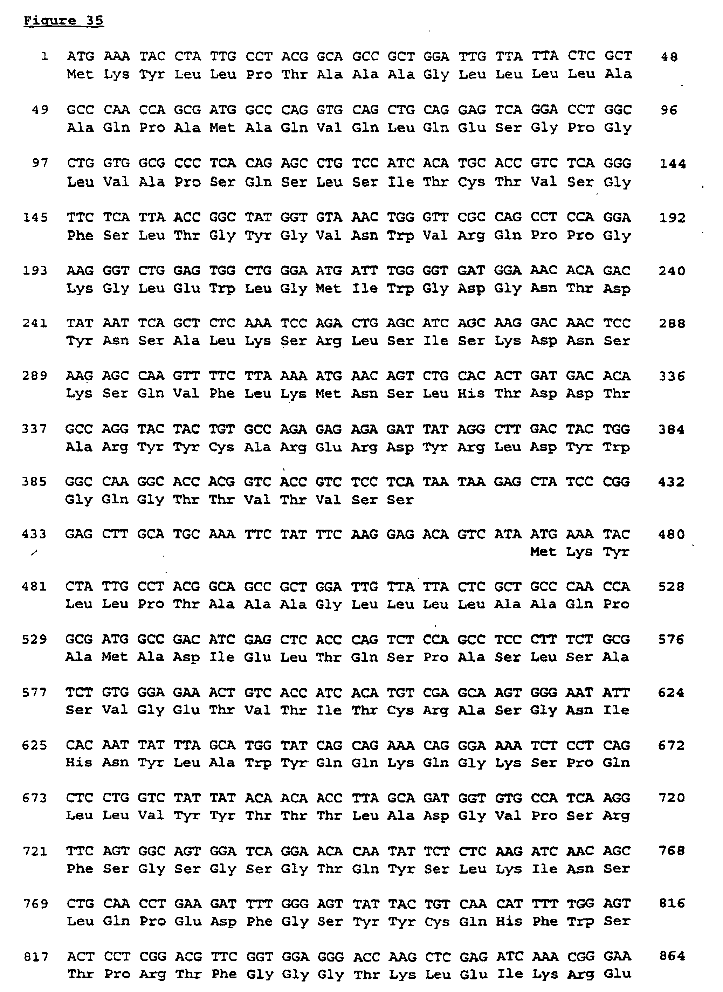

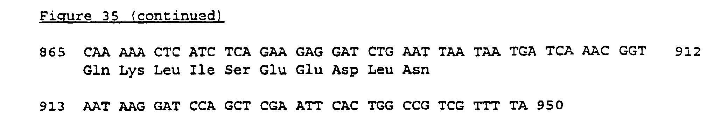

A Vmin peptide sequence for the anti-lysozyme antibody D1.3 (Amit, A.G., Mariuzza, R.A., Phillips, S.E.V. & Poljak R.J. Science 233, 747-754 (1986)), was generated by molecular modelling, based upon known DNA and peptide sequence generated from the plasmid pSW1-VHD1.3-VKD1.3-TAG1 (Ward, S.E., Gussow, D., Griffiths A.D., Jones, P.T. & Winter G. Nature 341, 544-546 (1989)) by standard molecular biology DNA sequencing techniques. The peptide sequence of the Vmin is shown in sequence 45, and the Vmin consists of three domains from the D1.3 antibody, amino acids 65 to 115 from the D1.3 heavy chain (1 to 51 in sequence 45), amino acids 13 to 64 from the D1.3 heavy chain (52 to 103 in sequence 45) and amino acids 1 to 108 from the D1.3 light chain (104 to 211 in sequence 45).
The sequence of the D1.3 insert in pSW1-VHD1.3-VKD1.3-TAG1 as determined from the start of the first pelB through to approximately 50 nucleotide bases after the TAG1 sequence is shown in Figure 35. It can be seen that the sequence after the TAG1 differs slightly from the published sequence (Ward, S.E., Gussow, D., Griffiths A.D., Jones, P.T. & Winter G. Nature 341, 544-546 (1989)) between the EcoR1 restriction site and end of the TAG1. It is the sequence as shown in Figure 35 that was used for the choice and sequence of the PCR primers. The specific choice of primers for Vmin gene construction will depend on the particular sequences to be included in the final construct.

The sequences of the chosen primers are shown in sequences 46 to 51. All primers are chosen to have about 20 bases complementary to the DNA strand on which they are priming at their 3' end, and if possible to end in a GC rich sequence. The single stranded DNA primers are used in pairs in three PCR reactions with pSW1-VHD1.3-VKD1.3-TAG1 double stranded DNA :-

### 1. Primer Sequence 46 with primer sequence 49.

Sequence 46 primer bases 22 to 39 are the same as bases 259 to 276 in Figure 35, except that the codon for Arg (262-264) is changed from AGA to CGT because of E.coli preferred codon usage (Andersson, S.G.E. & Kurland C.G. Microbological Reviews 54, 198-210 (1990)). Bases 1 to 21 of sequence 46 code for the seven amino acids Asp Tyr Pro Gly Ser Ser Gly and have a SmaI restriction site (GGGCCC) at bases 7 to 12. The SmaI site was included to allow blunt end cloning of the Vmin into an E.coli plasmid secretion vector. Additional codons were added and modelled at the start of the Vmin gene, because of the Arg at amino acid 2 in the Vmin sequence 45. It is known that charged amino acids at the start of a gene to be secreted into E.coli periplasm can block generation of the protein product (Geller B., Zhu H-Y., Cheng S., Kuhn A. & Dalbey R.E. Journal of Biological Chemistry, **268,** 9442-9447 (1993)), and the Gly Ser Ser Gly leader was designed to overcome this.

Sequence primer 49 bases 25 to 45 are complementary to bases 391 to 411 in Figure 35, except that the codon for Val (400-402) is change to Leu to fit with the molecular model sequence of D1.3 Vmin (sequence 45). Bases 1 to 24 are complementary to bases 103 to 126 in Figure 35.

### 2. Primer sequence 47 with primer sequence 50.

Sequence 47 primer bases 22 to 45 are the same as bases 103 to 126 in Figure 35. Primer bases 1 to 21 are the same as bases 391 to 411 in Figure 35, except that the codon for Val (400-402) is change to Leu to fit with the molecular model sequence of D1.3 Vmin (sequence 45).

Sequence 50 primer bases 24 to 46 are complementary to bases 236 to 258 in Figure 35. Primer bases 1 to 23 are complementary to bases 538 to 560 in Figure 35 except that the codons for Glu and Leu (544-449) are changed to Gln and Met to fit with the molecular modelling of D1.3 Vmin (Sequence 45).

### 3. Primer sequence 48 with primer sequence 51.

Sequence 48 primer bases 24 to 46 are the same as bases 538 to 560 in Figure 35 except that the codons for Glu and Leu (544-449) are changed to Gln and Met to fit with the molecular modelling of D1.3 Vmin (Sequence 45). Primer bases 1 to 23 are the same as bases 236 to 258 in Figure 35.

Sequence 51 primer bases 1 to 24 are complementary to bases 922 to 945 in Figure 35.

The PCR reactions were performed using a thermocycler of 25 cycles of 94°C for 1.5 minutes, 55°C for 1.5 minutes and 72°C for 2 minutes followed by a single period of 9.9 minutes at 72°C. Reactions were 20 microlitre total volume containing a concentration of 1 microMolar of the primers, 0.1ng of the pSW1-VHD1.3-VKD1.3-TAG1 double stranded plasmid DNA, dNTPs each at a concetration of 200 microMolar and amplification buffer and Taq DNA polymerase as recommended in 'Molecular Cloning - a laboratory manual, Sambrook, J., Fritsch, E.F. & Maniatis T. Published by Cold Spring Harbor Laboratory Press, second Edition 1989' or the material supplier's data sheets. PCR conditions may need to be optimised as recommended in the book.

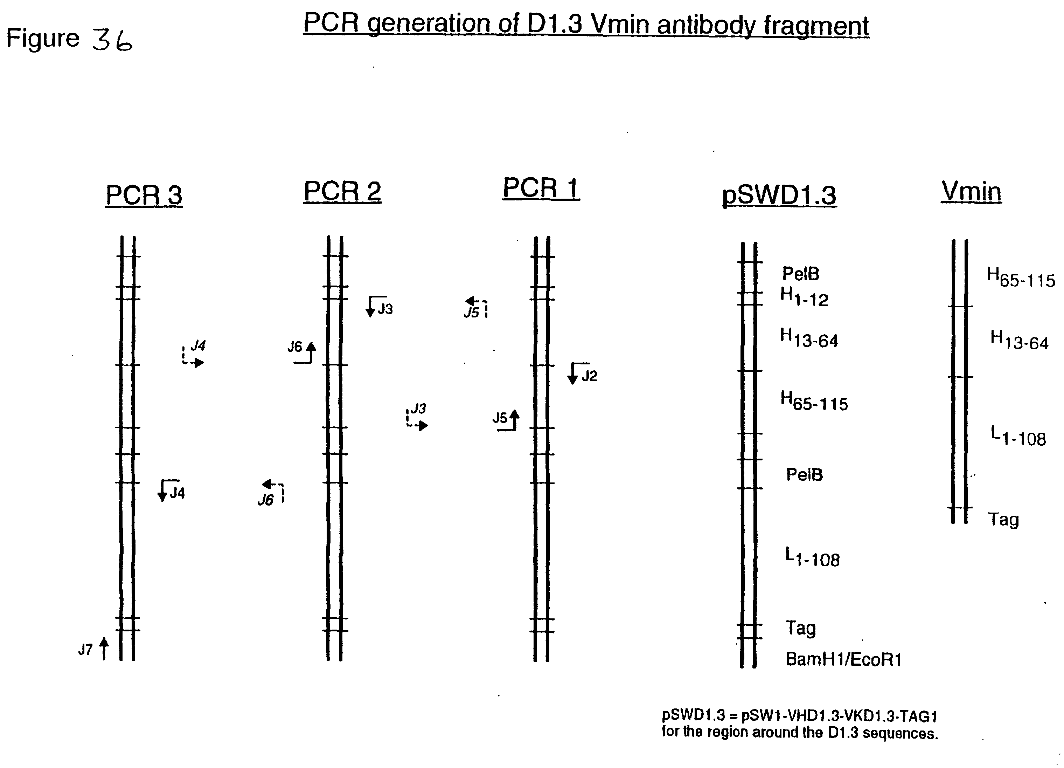

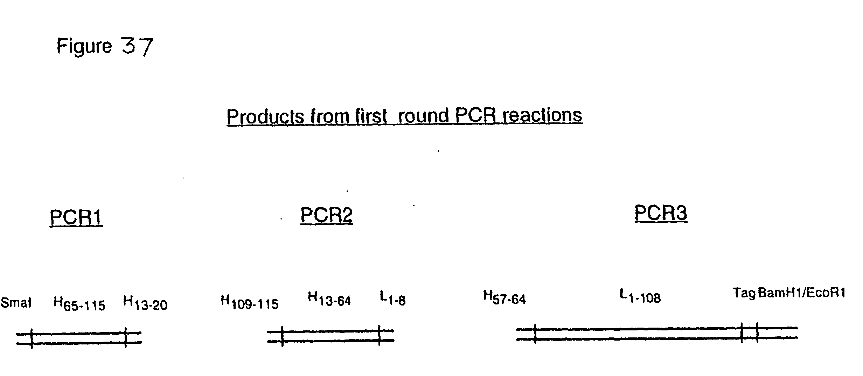

The structure of the D1.3 Vmin, the D1.3 insert in pSW1-VHD1.3-VKD1.3-TAG1 double stranded DNA, and the primers for these PCR reactions are summarised diagramatically in Figure 36. The products of the PCR reactions are summarised diagramatically in Figure 37. All numbering in these diagrams is based upon the amino-acid sequence of the D1.3 antibody.

The major product of each PCR reaction, with the correct molecular size, (approximately 200 base pairs for PCRs 1 and 2 and 400 for PCR3) was then band purified after running on a 1% agarose gel using standard molecular biology techniques. The isolated DNA from each band was dissolved in water to a final concentration of 1ng/microlitre.

The next stage in the generation of the Vmin is the joining of the PCR products from the first round of PCRs. This could be attempted in a number of ways, as described above, but in this case it was found that a two stage PCR containing all three products of the first round of PCRs gave a satisfactory yield of the Vmin gene product.

The first part of the PCR consisted of 10 cycles in a thermocycler of 94°C for 1 minute and 63°C for 4 minutes, with a hot start (reaction mixture heated to 94°C prior to the addition of the Taq polymerase). The reaction mix contained each of the Vmin gene fragments isolated from the first round of PCRs at a concentration of 4ng per 80 micolitres reaction volume together with dNTPs each at a concetration of 200 microMolar and amplification buffer and Taq DNA polymerase as recommended in 'Molecular Cloning - a laboratory manual, Sambrook, J., Fritsch, E.F. & Maniatis T. Published by Cold Spring Harbor Laboratory Press, second Edition 1989' or the material supplier's data sheets. PCR conditions may need to be optimised as recommended in the book. This first part of the reactions should generate some of the complete Vmin sequence because of the complementary overlaps at the ends of the Vmin fragments from the first PCRs.

The second part of the PCR consisted of 25 cycles of 94°C for 1.5 minutes followed by 55°C for 2 minutes and 72°C for 2 minutes with a final hold of 72°C for 9.9 minutes. Prior to these cycles the reaction volume was made up to 100 microlitres by the addition of the two end primers, seqences 46 and 51, to a concentration of ImicroMolar and fresh dNTPs each to a concetration of 200 microMolar and amplification buffer and fresh Taq DNA polymerase as recommended in 'Molecular Cloning - a laboratory manual, Sambrook, J., Fritsch, E.F. & Maniatis T. Published by Cold Spring Harbor Laboratory Press, second Edition 1989' or the material supplier's data sheets. PCR conditions may need to be optimised as recommended in the book.
The major product of the two part PCR, with the correct molecular size, (approximately 800 base pairs) was then band purified after running on a 1% agarose gel using standard molecular biology techniques. The isolated Vmin DNA from the band was dissolved in water to a final concentration of 1ng/microlitre.

### 4.1 Cloning of the PCR generated Vmin gene.

The Vmin gene product from the PCRs was the restriction enzyme cut to render it compatible with the plasmid expression vector. The Vmin gene was cut with enzymes SmaI and EcoRI, following standard molecular biology techniques, the suppliers recommendations, and the methods detailed in 'Molecular Cloning - a laboratory manual, Sambrook, J., Fritsch, E.F. & Maniatis T. Published by Cold Spring Harbor Laboratory Press, second Edition 1989'. The gene product was cleaned of small restriction fragments by standard techniques (for example Geneclean II, from Bio 101 Inc., PO Box 2284, La Jolla, California 92038, USA).

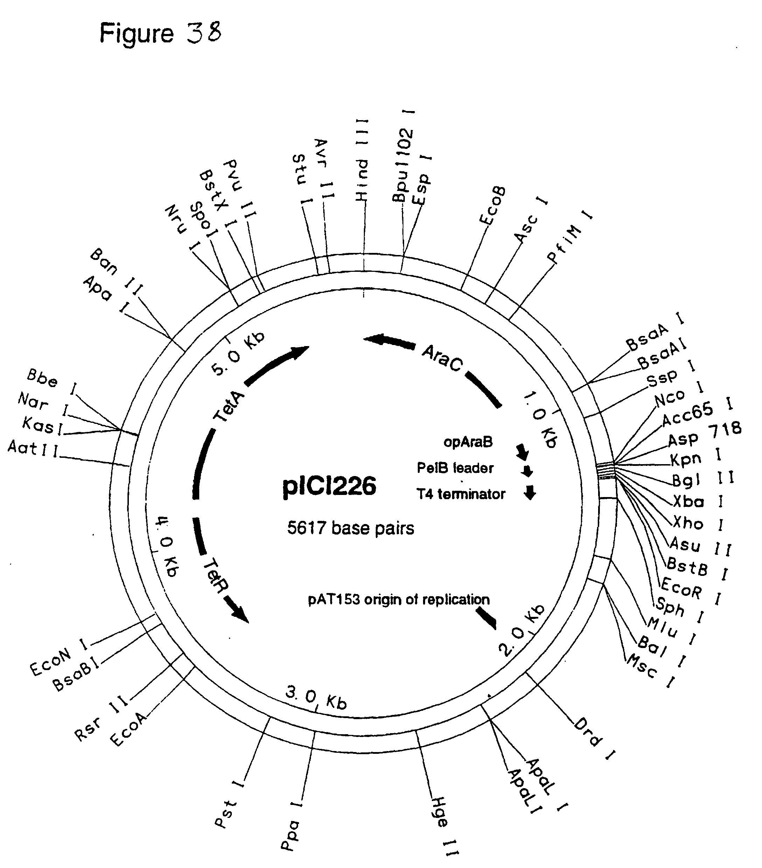

The E.coli plasmid vector selected for periplasmic secretion expression of the Vmin gene was pICI266. This vector (pICI266) with a single pelB leader sequence has been deposited under the Budapest Treaty at the National Collections of Industrial and Marine Bacteria Limited (NCIMB), 23 St. Machar Drive, Aberdeen, AB2 1RY, Scotland, U.K. The date of deposit was 11th September 1993 and the accession number is NCIMB 40589. A plasmid map of pICI266 is shown in Figure 38.

Plasmid DNA was generated by standard molecular biology techniques, using methods detailed in 'Molecular Cloning - a laboratory manual, Sambrook, J., Fritsch, E.F. & Maniatis T. Published by Cold Spring Harbor Laboratory Press, second Edition 1989'. Plasmid DNA was enzyme digested with restriction enzyme KpnI, using the manufacturer's recommended conditions. The cut plasmid DNA was then treated with Bacteriophage T4 DNA polymerase in the presence of high concentration of dNTPs (2milliMolar) at 12°C (details in 'Molecular Cloning - a laboratory manual, Sambrook, J., Fritsch, E.F. & Maniatis T. Published by Cold Spring Harbor Laboratory Press, second Edition 1989') to remove the protruding 3' termini generated by KpnI, and making compatible with the blunt end of the SmaI cut Vmin. The cut plasmid product was cleaned by standard techniques (for example Geneclean II, from Bio 101 Inc., PO Box 2284, La Jolla, California 92038, USA) and then enzyme digested with restriction enzyme EcoRI, using the manufacturer's recommended conditions. The cut plasmid product was cleaned of small restriction fragments by standard techniques (for example Geneclean II, from Bio 101 Inc., PO Box 2284, La Jolla, California 92038, USA).

The Vmin gene (treated with SmaI and EcoRI) and the pICI226 (treated with KpnI, T4 polymerase and EcoRI) were then ligated together using T4 DNA ligase under standard molecular biology conditions (details in 'Molecular Cloning - a laboratory manual, Sambrook, J., Fritsch, E.F. & Maniatis T. Published by Cold Spring Harbor Laboratory Press, second Edition 1989').

The ligation mix was then transformed into a suitable Ara⁻ E.coli strain such as that known locally as MSD213 (strain MC1000 of Casadaban, Journal of Molecular Biology 138, 179ff (1980)),(F⁻ ara delta (ara-leu) delta lacX74 galv galK strR) using calcium chloride treated cells as detailed in 'Molecular Cloning - a laboratory manual, Sambrook, J., Fritsch, E.F. & Maniatis T. Published by Cold Spring Harbor Laboratory Press, second Edition 1989'. Colonies were selected on L agar plates containing 10micrograms/ml of tetracycline.

### 4.2 Identification of transformants expressing active D1.3Vmin.

Individual colonies from the transformation were picked into numbered 1.5ml Eppendorf tubes containing sterile 200microlitres of L broth plus 0.1% arabinose plus 10micrograms/ml tetracycline. The tubes were well sealed and shaken upside down at approximately 200rpm at 30°C for about 16 hours. Cells in the tubes were precipitated by centifugation for 5 minutes in a bench top microfuge at room temperature. The supernatant was used immediately in ELISAs, and the cell pellet kept at 4°C until positive clones had been identified and could be picked and streaked on to fresh L agar with tetracycline plates.

For the ELISA, polystyrene microtitre plates were prepared by the addition of 100microlitres of a 3mg/ml solution of Chicken Egg White lysozyme (SIGMA L6876) in 0.05M carbonate/bicarbonate buffer pH9.6 (SIGMA C3141) to each well. The plates were incubated at 4°C for about two hours. The wells were washed three times, with a 5 minute soak between, in excess phosphate buffered saline (PBS) (SIGMA P4417). The plates were then banged dry onto tissues, and 150microlitres of a solution of a 1% Bovine serum albumin (SIGMA A7888 for example) solution in PBS added to each well. The plates were incubated at 4°C for about 2 hours. The wells were washed three times, with a 5 minute soak between, in excess phosphate buffered saline (PBS) (SIGMA P4417). The plates were then banged dry onto tissues, and 100microlitres of the bacerial supernatants added to the wells, keeping careful note of numbers and positions so that individual clones can be identified.
The plates were incubated at 4°C for about 16 hours. The wells were washed three times, with a 5 minute soak between, in excess phosphate buffered saline (PBS) (SIGMA P4417). The plates were then banged dry onto tissues, and 100microlitres of 9E10 hybridoma supernatant (Evan, G.I., Lewis G.K., Ramsay G. & Bishop J.M. Mol. Cell Biol., 5, 129-136 (1985)) at a suitable dilution (typically about 1 in 30) added to each well. The plates were incubated at 4°C for about 2 hours. The wells were washed three times, with a 5 minute soak between, in excess phosphate buffered saline (PBS) (SIGMA P4417) containing 0.05% Tween 20 (SIGMA P1379). The plates were then banged dry onto tissues, and 100microlitres of a suitable dilution of an anti-mouse IgG labelled with horse-radish peroxidase (for example SIGMA A0944 at a dilution of 1 in 2000) added to each well. The plates were incubated at 4°C for about 2 hours. The wells were washed three times, with a 5 minute soak between, in excess phosphate buffered saline (PBS) (SIGMA P4417) containing 0.05% Tween 20 (SIGMA P1379). The plates were then banged dry onto tissues, and 100microlitres of a solution of 30mg o-phenylenediamine dihydrochloride (OPD) dissolved in phosphate-citrate buffer with sodium perborate (SIGMA P4922) added to each well. The plates are then incubated in the dark at room temperature until the colour develops. 50microlitres of a solution of 25% sulphuric acid is added to each well to stop colour development. The absorbance of each well is read at 490nm. Positive wells are identified as having colour development more than twice background.

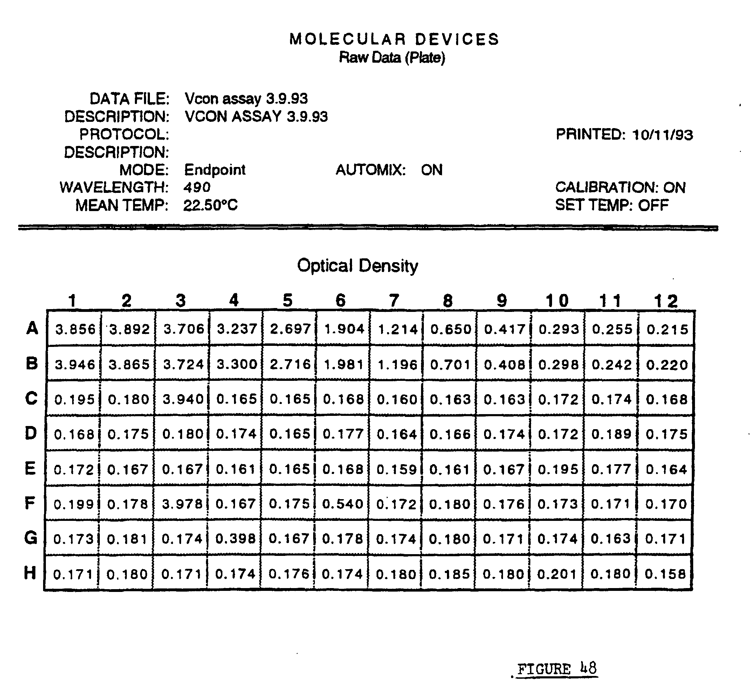

The results of an assay are shown in Figure 48 as raw data from the plate reader. On this plate rows A and B were duplicate doubling dilutions of a positive D1.3-Fab-cmyc construct, which was also included as sample numbers 3 and 34 (positions C3 and F3 on plate). Buffer blanks are included at positions C1, E10, F1 and H10. Bacterial controls are included at positions E11, E12, H11 and H12. Picked colonies, numbered 1 to 64 (including positive controls 3 and 34) are placed in the other wells starting at C2 and going from left to right and down the plate to H9. Thus the two positive identified clones from this plate, F6 (OD 0.540) and G4 (OD 0.398), correspond to colonies 37 and 47. The purified sequenced plasmid from 37 was later designated pJFH3037 (see below).

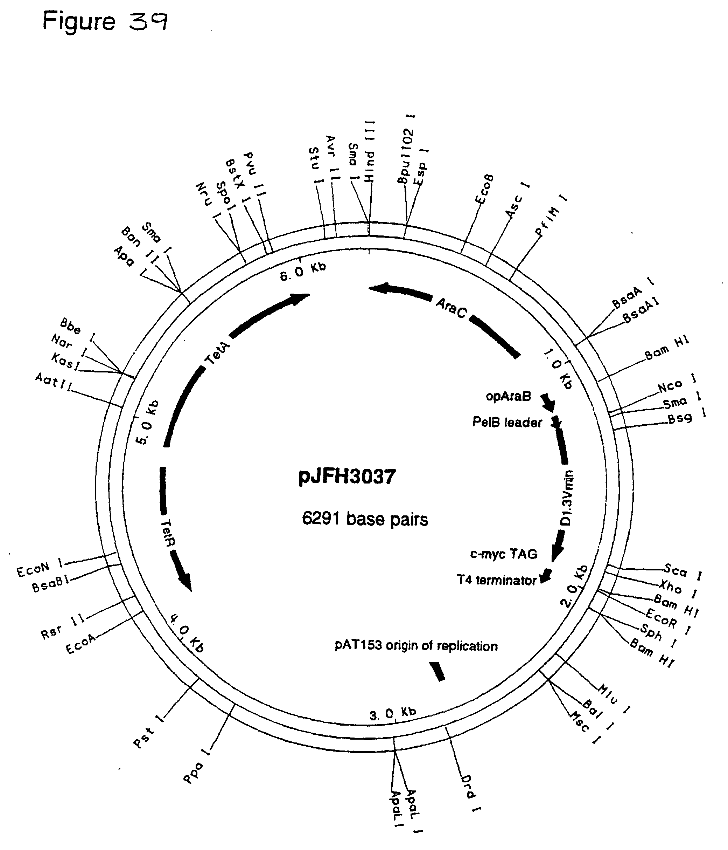

A number of positive wells were identified and the clones which led to them were grown and used to generate plasmid DNA by standard molecular biology methods (details in 'Molecular Cloning - a laboratory manual, Sambrook, J., Fritsch, E.F. & Maniatis T. Published by Cold Spring Harbor Laboratory Press, second Edition 1989'). The purified DNA was sequenced around the D1.3 Vmin insert, and a number of them were found to have the Vmin sequence shown in Sequence 53. One of these plasmids was designated pJFH3037, and a plasmid map is shown in figure 39.

### 4.3 Generation of E.coli cell pastes containing D1.3 V-min

E.coli strain MSD 213 was transformed with plasmid pJFH3037. The resultant strain MSD 215 (pJFH3037) was purified and maintained in glycerol stocks at -80oC. Aliquots of each culture were removed from stock and streaked onto agar plates of L-tetracycline to give separate single colonies after overnight growth at 37oC. A single colony of each culture was removed and suspended separately in 10ml L-tetracycline broth and 100ul immediately inoculated into each of fifteen 250 ml Erlenmeyer flasks containing 75 ml of L-tetracycline broth supplemented with 0.1% w.v L-arabinose. After growth for 16h at 30oC on a reciprocating shaker the bacteria were harvested in 1L centrifuge bottles in a Sorvall RC-3B centrifuge (7000 x g, 4oC, 30 min).

### 4.4 Purification and sequence identification of V-min D1.3 (conversative) with c-myc tag

Pellets (frozen at -80°; from 4.3) of E.coli cell paste were thawed, resuspended in lysis buffer (50mM Tris; 50mM EDTA; 15% Sucrose; 1mM Benzamidine, pH8.0) at 4° and 800µl of 2.5% sodium dodecyl sulphate (SDS) solution was added for every 25 ml of the suspension. The mixture was left on ice for 30 min with occasional agitation, deoxyribonuclease added, mixed and the suspension was left on ice for a further 30 min with occasional agitation. The cell suspension was divided into 45 ml aliquots which were sonicated on ice for 12 x 30s bursts with 30s intervals between bursts. Sonicated suspensions were centrifuged at 13,000 rpm for 40 min at 4° and the supernatant collected. Phenyl methylsulphonyl Fluoride (PMSF) was added to the supernatant pool to give a 1mM concentration followed by dialysis overnight at 4° into phosphate buffered saline (PBS), pH 7.3. The dialysed supernatant was filtered through a 0.22µm membrane, loaded on to an immobilised lysozyme column (CNBr-activated Sepharose 4B TM matrix based) equilibrated at 4° in PBS, pH 7.3. The column was washed with PBS and bound material was eluted by 50mM diethylamine, pH 9.5. The successful binding of the D.3 V-min to its immobilised antigen confirms maintenance of appropriate CDR conformations found in the parent D1.3 antibody. Eluted peak fractions were pooled, tri(hydroxymethyl)aminomethane (TRIS) added to give a 100mM concentration and pH adjusted to around pH 7.5 and sterile filtered. Further purification, utilising known methods such as ion exchange or gel permeation chromatography may be performed if required.

A sample of the material was analysed by SDS-PAGE using a 12% TRIS-Glycine gel which was electro-blotted on to a nitrocellulose membrane followed by Western blot analysis using an anti- c-myc tag antibody (9E10). The relevant band on a Coomassie stained nitrocellulose blot was sequenced by an automated protein/peptide sequencer using the Edman degradation technique. A positive match with the expected N-terminal sequence of D-Y-P-G-S-S-G-S-R-L was obtained (D-Y-P-G-?-?-G-?-R-L-) thus confirming identity between the isolated protein and gene sequences.

### EXAMPLE 5 Intra-cellular E.coli expression of D1.3 Vmin (conservative)

In a similar fashion to the generation of the secreted D1.3 Vmin gene detailed in Example 4, a Vmin gene was made by PCR for cloning into an inta-cellular E.coli expression vector. In this case the PCR primer shown in sequence 52 was used in place of the primer sequence 46, in the first round PCR reaction with primer sequence 49, and in the joining of the three first round PCR products together with primer sequence 51 in the second round PCR. The Vmin gene product then has the 5' sequence of primer sequence 52 at the start of the Vmin gene, and this provides an HpaI restriction site (GTTAAC) and sequence comprising RWWWWWWWW (where R is A or G, and W is A or T) preceding an ATG (methionine) start codon and the Vmin gene. The RWWWWWWWW generates a number of sequences that can act as ribosome binding sites, and effective constructs, which express the gene from a promoter in a suitable vector, can be selected by looking at expression levels.

This D1.3 Vmin gene for intra-cellular expression was restriction enzyme cut with Hpal and BamHI. An expression vector such as pICI0080 (described in section 2.5.6.1) is used for cloning of the Vmin. In this case the pICI0080 plasmid DNA is restriction cut with enzyme KpnI, and then treated with T4 DNA polymerase to remove the 3' protruding termini, in a similar manner to that described for plasmid pICI266 (section 4.1). After cleaning the KpnI cut/T4 DNA polymerase treated pICI0080 is restriction digest cut with enzyme BglII and the plasmid DNA cleaned as described. Restriction enzymes BglII and BamHI generate identical cohesive ends, and the D1.3Vmin gene (BamHI and HpaI cut) is then ligated with pICI0080 (KpnI/T4 DNA polymerase and BglII treated), as described. This inserts the Vmin gene with a variety of potential ribosome binding sites under the control of the pTrp promoter of the pICI0080 vector.

The ligation mix is used to transform a range of E.coli host strains, some of which are protease deficient, to assess accumulation in different genetic backgrounds. The methods for expression studies are essentially the same as those presented in section 2.7, except that the 42°C induction is not required in the pTrp promoter system of pICI0080. Protein accumulation may be affected by the temperature of the growth of the culture, and so a range of temperatures are tested for each isolate. Isolates found to give positive bands in the Coomassie-blue stained protein gels and/or by Western detection of the c-myc TAG sequence using antibody 9E10, are then used to generate plasmid DNA. This is sequenced around the Vmin gene to check and confirm the correct nucleic acid sequence of the gene insert, and to identify the specifc ribosome binding site sequence, in one example AAAATATTT (from the RWWWWWWW possibilities). Vmin produced by the intra-cellular expression system may then be extracted, isolated and renatured as described in section 2.8, and the activity tested in systems as described in section 4.2 for an ELISA binding to lysozyme.

### EXAMPLE 6 Vmin Constructs for D1.3 (1fdl)

### Anti-Lysozyme antibody

Fischmann T.O., Bentley G.A., Bhat T.N., Boulot G., Mariuzza R.A., Phillips S.E.V., Tello D., Poljak R.J.
Journal of Biological Chemistry 1991 266 12915.

### 6.1 a) h65-115/h13-64/11-108 Intact Light Chain

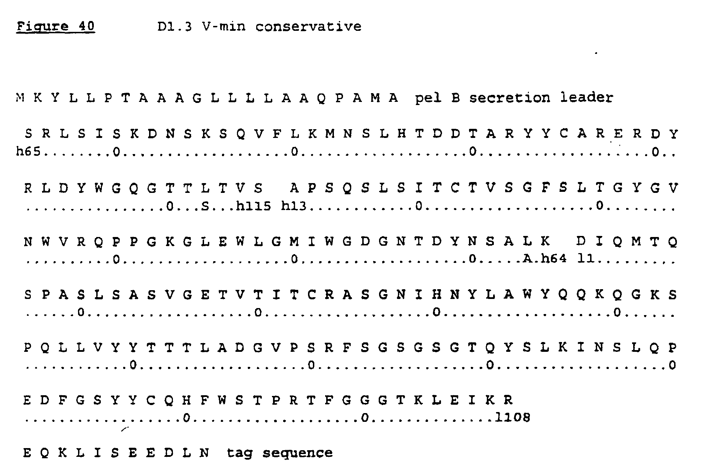

Utilising methods set out hereinbefore the V-min construct in Figure 41 was produced.

### 6.2 b) 161-107/113-60/h1-116 Intact Heavy Chain D1.3

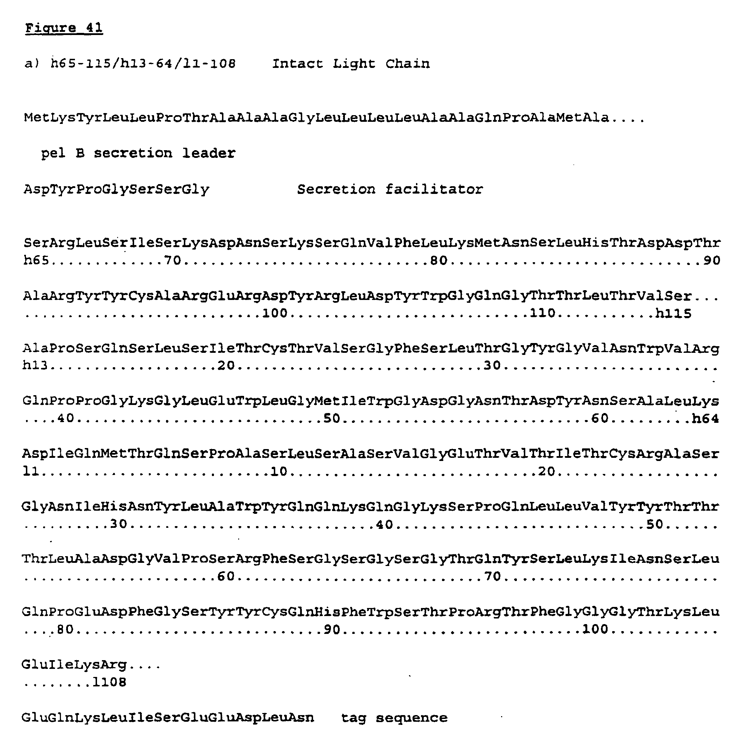

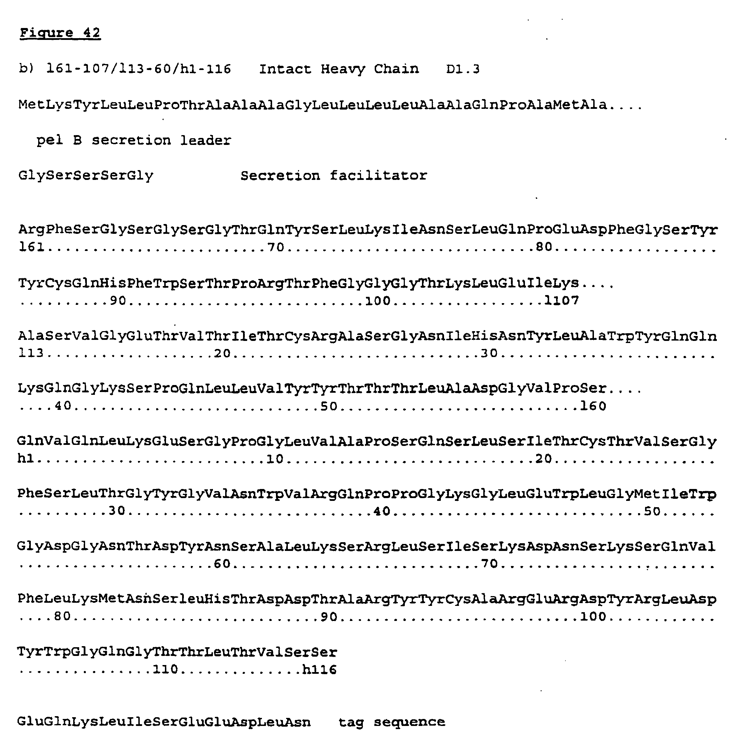

Utilising methods set out hereinbefore the V-min construct in Figure 42 was produced.

### 6.3 c) h87-115/h13-64/11-7/121-61/176-108 Minimal Construct D1.3

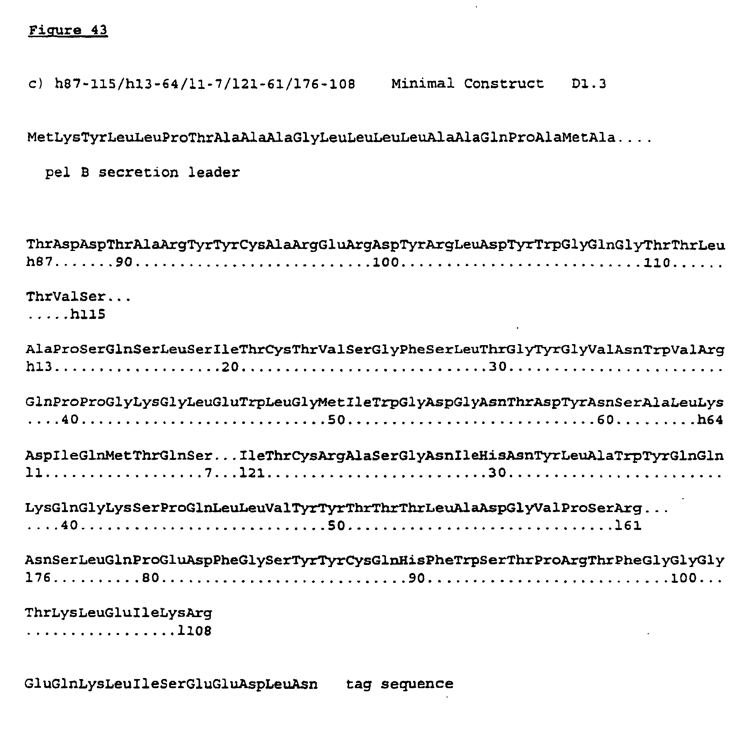

Utilising methods set out hereinbefore the V-min construct in Figure 43 was produced.

### EXAMPLE 7 Vmin Constructs for KOL (2fb4)

Antibody from Human Myeloma patient KOL

Kratzin H.D., Palm W., Stangel M., Schmidt W.E., Friedrich J., Hilschman N.

Biological Chemistry Hoope-Seyler 1989 370 263.

Marquart M., Deisenhofer J., Huber R., Palm W.
Journal Of Molecular Biology 1980 141 369.

### 7.1 a) h67-125/hl3-66/11-112 Intact Light Chain KOL

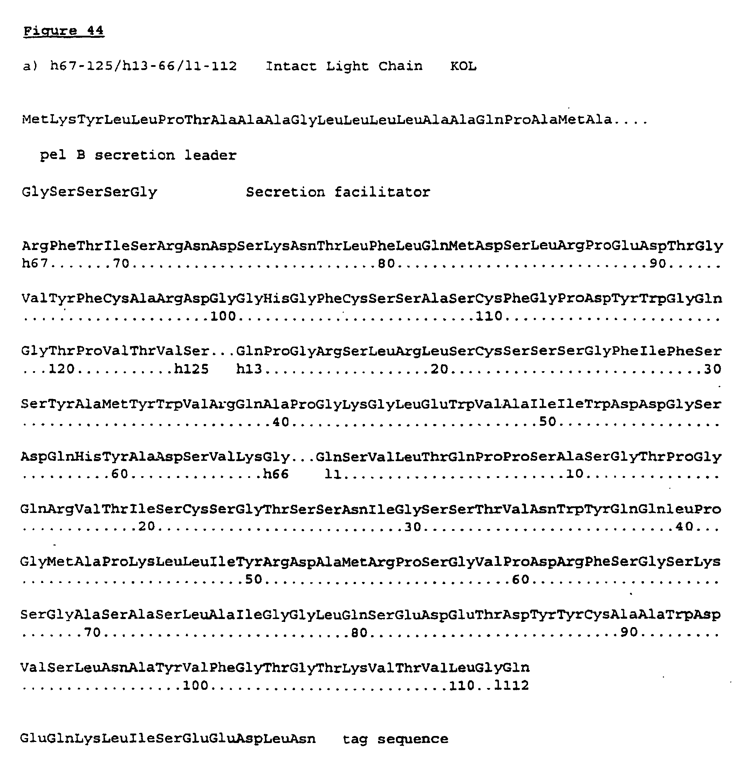

Utilising methods set out hereinbefore the V-min construct in Figure 44 was produced.

### 7.2 b) 162-109/112-61/h1-125 Intact Heavy Chain KOL

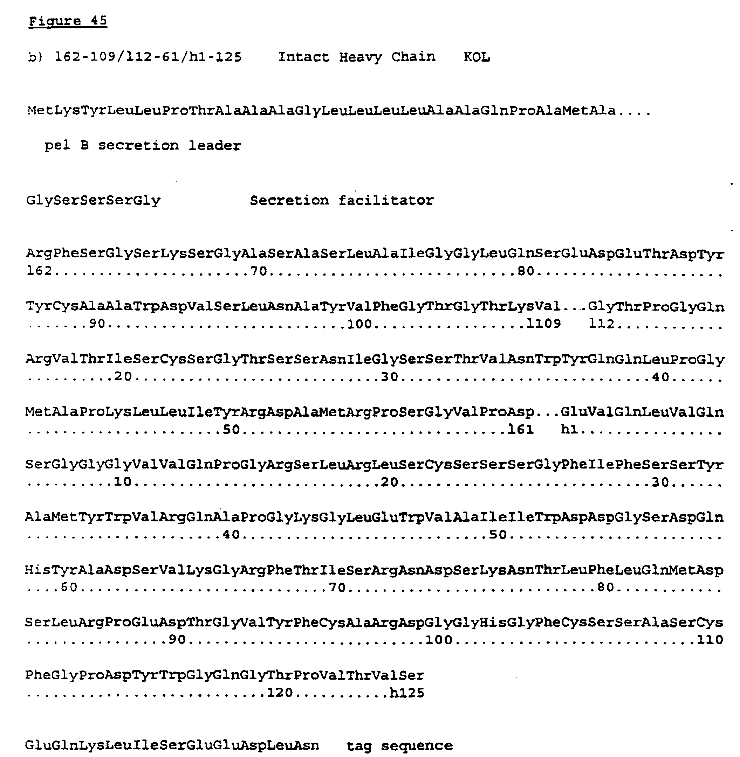

Utilising methods set out hereinbefore the V-min construct in Figure 45 was produced.

### EXAMPLE 8 Vmin Constructs for pac-1

Antibody directed against Integrin IIbIIIa

Tomiyama Y., Brojer E., Rugger Z.M., Shattil S.J., Smiltneck J., Gorski J.,
Kumar A., Kieber-Emmons T., Kunicki T.J.
Journal of Biological Chemistry 1992 267 18085-18092.

### 8.1 a) h66-118/h13-65/11-112 Intact Light Chain PAC-1

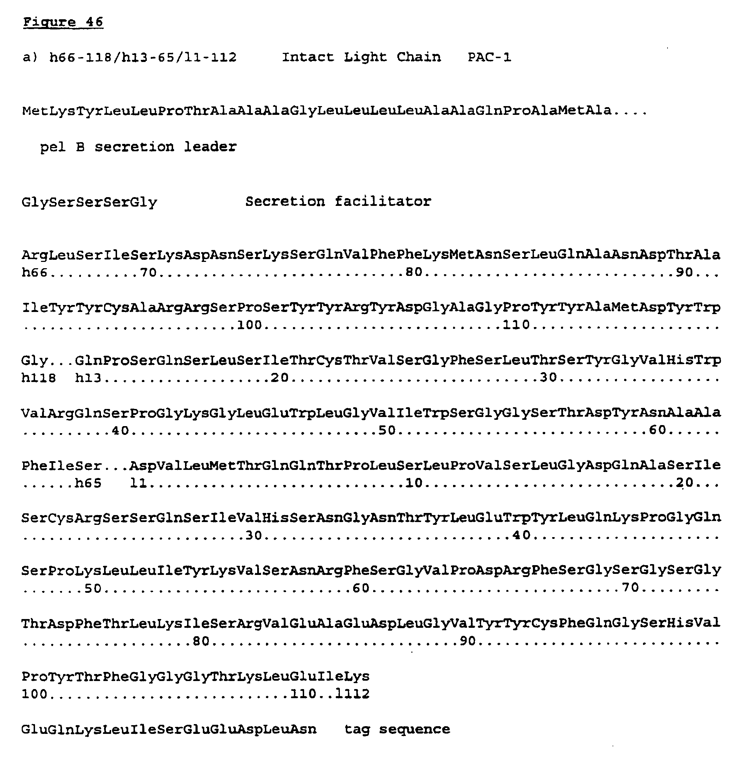

Utilising methods set out hereinbefore the V-min construct in Figure 46 was produced.

### 8.2 b) 166-111/112-65/h1-123 Intact Heavy Chain PAC-1

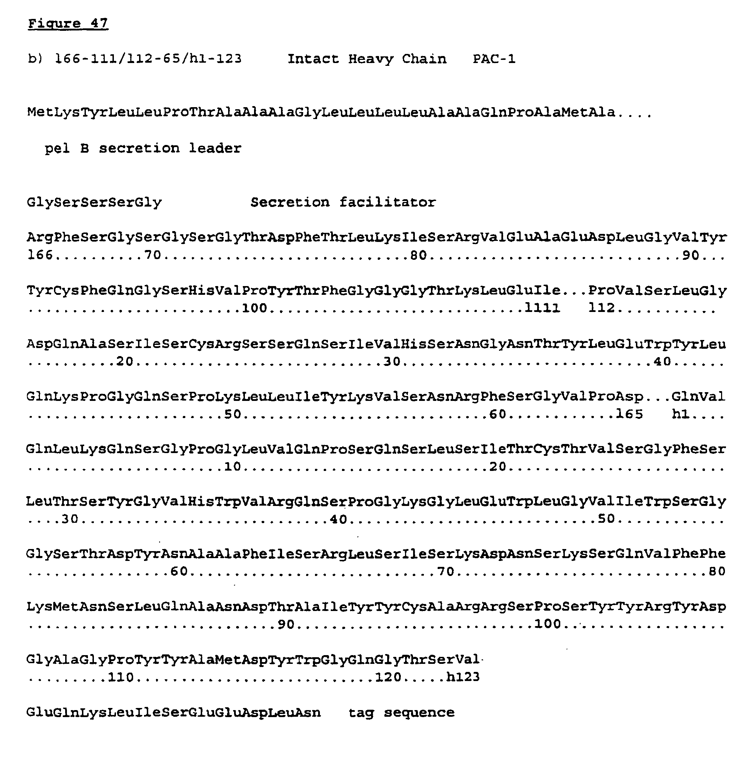

Utilising methods set out hereinbefore the V-min construct in Figure 47 was produced.

### References

1) Bagshawe, K. D et al, Br. J. Cancer (198B) 58, 700 - 703.
2) Y. Satow, G. H. Cohen, E. A. Padlan, D. R. Davies;
   J. Mol. Biol., 1987, 190, 593-604.
3) R. H. Lee, Nature, 1992, 356, 543-544
4) C. Chothia, A. M. Lesk, A. Tramontano, M. Levitt, S. J. Smith-Gill, G. Air, S. Sheriff, E. A. Padlan, D. Davies, W. R. Tulip, P. M. Colman, S. Spinelli, P. M. Alzari, R. J. Poljak;
   Nature, 1989, 342, 877-883.
5) QUANTA Polygen Corporation/York University 1986
6) B. R. Brooks, R. E. Bruccoleri, B. D. Olafson, D. J. States, S. Swaminathan, M. Karplus; J. Comp. Chem., 1983, 4, 187-217.
7) J.S. Richardson; Advances in Protein Chemistry, 1961, 34, 167-339.
8) W.Kabsch, C. Sander; Biopolymers, 1983, 22, 2577-2637.
9) A. Shrake, J.A. Rupley; J. Mol. Biol., 1973, 79, 351-372
10) T. Ooi, M. Oobatake,G. Nemethy, H.A. Scheraga;
   Proc. Natl. Acad. Sci., 1987, 84, 3086-3090.
11) C. Chothia; Annu. Rev. Biochem., 1984, 53, 537-572.
13) P.M. Kirkham, F. Mortari, J.A. Newton, H.W. Schroeder Jr., (1992) The EMBO Journal, 11, 603-609.
14) Sambrook J, Fritsch EF, Maniatis T: Molecular Cloning: A laboratory manual.
   New York, Cold spring Harbor Laboratory Press, 1989.
15) Harlow E, Lane DP: Antibodies: A laboratory manual.
   New York, Cold Spring Harbor Laboratory Press, 1988.
16) Evan GI, Lewis GK, Ramsay G, Bishop JM: Isolation of monoclonal antibodies specific for human c-myc proto-oncogene product.
   Mol Cell Biol 1985; 5: 129-136.
17) Clafin JL, Lieberman R, Davie JM: Clonal nature of the immune response to phosphorylcholine.
   J Exp Med 1974; 139; 58-73.

### SEQUENCE LISTING

(2) INFORMATION FOR SEQ ID NO: 1
   (i) SEQUENCE CHARACTERISTICS:
      (A) SEQUENCE LENGTH: 25 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:1:
(2) INFORMATION FOR SEQ ID NO: 2
   (i) SEQUENCE CHARACTERISTICS:
      (A) SEQUENCE LENGTH: 62 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:2:
(2) INFORMATION FOR SEQ ID NO: 3
   (i) SEQUENCE CHARACTERISTICS:
      (A) SEQUENCE LENGTH: 59 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:3:
(2) INFORMATION FOR SEQ ID NO: 4
   (i) SEQUENCE CHARACTERISTICS:
      (A) SEQUENCE LENGTH: 60 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:4:
(2) INFORMATION FOR SEQ ID NO: 5
   (i) SEQUENCE CHARACTERISTICS:
      (A) SEQUENCE LENGTH: 61 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:5:
(2) INFORMATION FOR SEQ ID NO: 6
   (i) SEQUENCE CHARACTERISTICS:
      (A) SEQUENCE LENGTH: 61 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:6:
(2) INFORMATION FOR SEQ ID NO: 7
   (i) SEQUENCE CHARACTERISTICS:
      (A) SEQUENCE LENGTH: 61 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:7:
(2) INFORMATION FOR SEQ ID NO: 8
   (i) SEQUENCE CHARACTERISTICS:
      (A) SEQUENCE LENGTH: 51 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:8:
(2) INFORMATION FOR SEQ ID NO: 9
   (i) SEQUENCE CHARACTERISTICS:
      (A) SEQUENCE LENGTH: 60 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:9:
(2) INFORMATION FOR SEQ ID NO: 10
   (i) SEQUENCE CHARACTERISTICS:
      (A) SEQUENCE LENGTH: 52 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:10:
(2) INFORMATION FOR SEQ ID NO: 11
   (i) SEQUENCE CHARACTERISTICS:
      (A) SEQUENCE LENGTH: 51 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:11:
(2) INFORMATION FOR SEQ ID NO: 12
   (i) SEQUENCE CHARACTERISTICS:
      (A) SEQUENCE LENGTH: 59 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:12:
(2) INFORMATION FOR SEQ ID NO: 13
   (i) SEQUENCE CHARACTERISTICS:
      (A) SEQUENCE LENGTH: 62 bases
      (B) SEQUENCE TYPE: Nucleotide
      (C) STRAHDEDNESS: Single
      (D) TOPOLOGY: Linear
   (xi) SEQUENCE DESCRIPTION: SEQ ID NO:13:
(2) INFORMATION FOR SEQ ID NO: 14
   (i) SEQUENCE CHARACTERISTICS:
      (A) SEQUENCE LENGTH: 60 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:14:
(2) INFORMATION FOR SEQ ID NO: 15
   (i) SEQUENCE CHARACTERISTICS:
      (A) SEQUENCE LENGTH: 53 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:15:
(2) INFORMATION FOR SEQ ID NO: 16
   (i) SEQUENCE CHARACTERISTICS:
      (A) SEQUENCE LENGTH: 60 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:16:
(2) INFORMATION FOR SEQ ID NO: 17
   (i) SEQUENCE CHARACTERISTICS:
      (A) SEQUENCE LENGTH: 61 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:17:
(2) INFORMATION FOR SEQ ID NO: 18
   (i) SEQUENCE CHARACTERISTICS:
      (A) SEQUENCE LENGTH: 58 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:18:
(2) INFORMATION FOR SEQ ID NO: 19
   (i) SEQUENCE CHARACTERISTICS:
      (A) SEQUENCE LENGTH: 63 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:19:
(2) INFORMATION FOR SEQ ID NO: 20
   (i) SEQUENCE CHARACTERISTICS:
      (A) SEQUENCE LENGTH: 34 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:20:
(2) INFORMATION FOR SEQ ID NO: 21
   (i) SEQUENCE CHARACTERISTICS:
      (A) SEQUENCE LENGTH: 60 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:21:
(2) INFORMATION FOR SEQ ID NO: 22
   (i) SEQUENCE CHARACTERISTICS:
      (A) SEQUENCE LENGTH: 59 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:22:
(2) INFORMATION FOR SEQ ID NO: 23
   (i) SEQUENCE CHARACTERISTICS:
      (A) SEQUENCE LENGTH: 58 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:23:
(2) INFORMATION FOR SEQ ID NO: 24
   (i) SEQUENCE CHARACTERISTICS:
      (A) SEQUENCE LENGTH: 46 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:24:
(2) INFORMATION FOR SEQ ID NO: 25
   (i) SEQUENCE CHARACTERISTICS:
      (A) SEQUENCE LENGTH: 47 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:25:
(2) INFORMATION FOR SEQ ID NO: 26
   (i) SEQUENCE CHARACTERISTICS:
      (A) SEQUENCE LENGTH: 55 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:26:
(2) INFORMATION FOR SEQ ID NO: 27
   (i) SEQUENCE CHARACTERISTICS:
      (A) SEQUENCE LENGTH: 59 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:27:
(2) INFORMATION FOR SEQ ID NO: 28
   (i) SEQUENCE CHARACTERISTICS:
      (A) SEQUENCE LENGTH: 56 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:28:
(2) INFORMATION FOR SEQ ID NO: 29
   (i) SEQUENCE CHARACTERISTICS:
      (A) SEQUENCE LENGTH: 55 bases
      (B) SEQUENCE TYPE: Nucleotide
      (C) STRAMDEDMESS: Single
      (D) TOPOLOGY: Linear
   (xi) SEQUENCE DESCRIPTION: SEQ ID NO:29:
(2) INFORMATION FOR SEQ ID NO: 30
   (i) SEQUENCE CHARACTERISTICS:
      (A) SEQUENCE LENGTH: 60 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:30:
(2) INFORMATION FOR SEQ ID NO: 31
   (i) SEQUENCE CHARACTERISTICS:
      (A) SEQUENCE LENGTH: 56 bases
      (B) SEQUENCE TYPE: Nucleotide
      (C) STRAMDEDNESS: Single
      (D) TOPOLOGY: Linear
   (xi) SEQUENCE DESCRIPTION: SEQ ID NO:31:
(2) INFORMATION FOR SEQ ID NO: 32
   (i) SEQUENCE CHARACTERISTICS:
      (A) SEQUENCE LENGTH: 45 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:32:
(2) INFORMATION FOR SEQ ID NO: 33
   (i) SEQUENCE CHARACTERISTICS:
      (A) SEQUENCE LENGTH: 48 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:33:
(2) INFORMATION FOR SEQ ID NO: 34
   (i) SEQUENCE CHARACTERISTICS:
      (A) SEQUENCE LENGTH: 56 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:34:
(2) INFORMATION FOR SEQ ID NO: 35
   (i) SEQUENCE CHARACTERISTICS:
      (A) SEQUENCE LENGTH: 60 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:35:
(2) INFORMATION FOR SEQ ID NO: 36
   (i) SEQUENCE CHARACTERISTICS:
      (A) SEQUENCE LENGTH: 60 bases
      (B) SEQUENCE TYPE: Nucleotide
      (C) STRANDEDNESS: Single
      (D) TOPOLOGY: Linear
   (xi) SEQUENCE DESCRIPTION: SEQ ID NO:36:
(2) INFORMATION FOR SEQ ID NO: 37
   (i) SEQUENCE CHARACTERISTICS:
      (A) SEQUENCE LENGTH: 152 bases
      (B) SEQUENCE TYPE: Nucleic acid
      (C) STRANDEDNESS: Single
      (D) TOPOLOGY: Linear
   (xi) SEQUENCE DESCRIPTION: SEQ ID NO:38:
(2) INFORMATION FOR SEQ ID NO: 38
   (i) SEQUENCE CHARACTERISTICS:
      (A) SEQUENCE LENGTH: 66 bases
      (B) SEQUENCE TYPE: Nucleotide
      (C) STRANDEDNESS: Double (top strand listed)
      (D) TOPOLOGY: Linear
   (xi) SEQUENCE DESCRIPTION: SEQ ID NO:37:
(2) INFORMATION FOR SEQ ID NO: 39
   (i) SEQUENCE CHARACTERISTICS:
      (A) SEQUENCE LENGTH: 210 amino acids
      (B) SEQUENCE TYPE: amino acid
      (D) TOPLOGY: linear
   (xi) SEQUENCE DESCRIPTION: SEQ ID NO:39:
(2) INFORMATION FOR SEQ ID NO: 40:
   (i) SEQUENCE CHARACTERISTICS:
      (A) LENGTH: 220 amino acids
      (B) TYPE: amino acid
      (D) TOPOLOGY: linear
   (xi) SEQUENCE DESCRIPTION: SEQ ID NO: 40
(2) INFORMATION FOR SEQ ID NO: 41:
   (i) SEQUENCE CHARACTERISTICS:
      (A) LENGTH: 222 amino acids
      (B) TYPE: amino acid
      (D) TOPOLOGY: linear
   (xi) SEQUENCE DESCRIPTION: SEQ ID NO: 41
(2) INFORMATION FOR SEQ ID NO: 42:
   (i) SEQUENCE CHARACTERISTICS:
      (A) LENGTH: 217 amino acids
      (B) TYPE: amino acid
      (D) TOPOLOGY: linear
   (xi) SEQUENCE DESCRIPTION SEQ ID NO: 42
(2) INFORMATION FOR SEQ ID NO: 43:
   (i) SEQUENCE CHARACTERISTICS:
      (A) LENGTH: 169 amino acids
      (B) TYPE: amino acid
      (D) TOPOLOGY: linear
   (xi) SEQUENCE DESCRIPTION: SEQ ID NO: 43
   Thr Ala Ile Tyr Tyr Cys Ala Arg Asn Tyr Tyr Gly Ser Thr Trp Tyr
   1 5 10 15
   Phe Asp Val Trp Gly Ala Gly Thr Thr Val Gly Gly Ser Leu Arg Leu
   20 25 30
   Ser Cys Ala Thr Ser Gly Phe Thr Phe Ser Asp Phe Tyr Met Glu Trp
   35 40 45
   Val Arg Gln Pro Pro Gly Lys Arg Leu Glu Trp Ile Ala Ala Ser Arg
   50 55 60
   Asn Lys Gly Asn Lys Tyr Thr Thr Glu Tyr Ser Ala Ser Val Asp Ile
   65 70 75 80
   Val Met Thr Gln Ser Met Ser Cys Lys Ser Ser Gln Ser Leu Leu Asn
   85 90 95
   Ser Gly Asn Gln Lys Asn Phe Leu Ala Trp Tyr Gln Gln Lys Pro Gly
   100 105 110
   Gln Pro Pro Lys Leu Leu Ile Tyr Gly Ala Ser Thr Arg Glu Ser Gly
   115 120 125
   Val Pro Asp Arg Phe Thr Glu Asp Leu Ala Val Tyr Tyr Cys Gln Asn
   130 135 140
   Asp His Ser Tyr Pro Leu Thr Phe Gly Ala Gly Thr Lys Leu Glu Gln
   145 150 155 160
   Lys Leu Ile Ser Glu Glu Asp Leu Asn
   165
(2) INFORMATION FOR SEQ ID NO: 44:
   (i) SEQUENCE CHARACTERISTICS:
      (A) LENGTH: 157 amino acids
      (B) TYPE: amino acid
      (D) TOPOLOGY: linear
   (xi) SEQUENCE DESCRIPTION: SEQ ID NO: 44
   Thr Gly Thr Tyr Tyr Cys Thr Thr Arg Phe Ala Tyr Trp Gly Gln Gly
   1 5 10 15
   Thr Leu Val Gly Gly Ser Met Lys Leu Ser Cys Ala Ala Ser Gly Phe
   20 25 30
   Thr Phe Ser Asp Ala Trp Met Asp Trp Val Arg Gln Ser Pro Glu Lys
   35 40 45
   Gly Leu Glu Trp Val Ala Glu Ile Gly Asn Lys Gly Asn Asn His Ala
   50 55 60
   Thr Tyr Tyr Ala Glu Ser Val Asp Ile Lys Met Thr Gln Ser Ile Thr
   65 70 75 80
   Cys Lys Ala Ser Gln Asp Ile Asn Ser Tyr Leu Ser Trp Phe Gln Gln
   85 90 95
   Lys Pro Gly Lys Ser Pro Lys Thr Leu Ile Tyr Arg Ala Asn Arg Leu
   100 105 110
   Val Asp Gly Val Pro Ser Arg Phe Ser Glu Asp Met Gly Ile Tyr Tyr
   115 120 125
   Cys Leu Gln Tyr Asp Glu Phe Pro Arg Thr Phe Gly Gly Gly Thr Lys
   130 135 140
   Leu Glu Gln Lys Leu Ile Ser Glu Glu Asp Leu Asn End
   145 150 155
(2) INFORMATION FOR SEQ ID NO:45:
   (i) SEQUENCE CHARACTERISTICS:
      (A) LENGTH: 211 amino acids
      (B) TYPE: protein
      (C) STRANDEDNESS: single
      (D) TOPOLOGY: linear
   (xi) SEQUENCE DESCRIPTION SEQ ID NO:45:
   1 Ser Arg Leu Ser Ile Ser Lys Asp Asn Ser Lys Ser Gln Val Phe Leu 16
   17 Lys Met Asn Ser Leu His Thr Asp Asp Thr Ala Arg Tyr Tyr Cys Ala 32
   33 Arg Glu Arg Asp Tyr Arg Leu Asp Tyr Trp Gly Gln Gly Thr Thr Leu 48
   49 Thr Val Ser Ala Pro Ser Gln Ser Leu Ser Ile Thr Cys Thr Val Ser 64
   65 Gly Phe Ser Leu Thr Gly Tyr Gly Val Asn Trp Val Arg Gln Pro Pro 80
   81 Gly Lys Gly Leu Glu Trp Leu Gly Met Ile Trp Gly Asp Gly Asn Thr 96
   97 Asp Tyr Asn Ser Ala Leu Lys Asp Ile Gln Met Thr Gln Ser Pro Ala 112
   113 Ser Leu Ser Ala Ser Val Gly Glu Thr Val Thr Ile Thr Cys Arg Ala 128
   129 Ser Gly Asn Ile His Asn Tyr Leu Ala Trp Tyr Gln Gln Lys Gln Gly 144
   145 Lys Ser Pro Gln Leu Leu Val Tyr Tyr Thr Thr Thr Leu Ala Asp Gly 160
   161 Val Pro Ser Arg Phe Ser Gly Ser Gly Ser Gly Thr Gln Tyr Ser Leu 176
   177 Lys Ile Asn Ser Leu Gln Pro Glu Asp Phe Gly Ser Tyr Tyr Cys Gln 192
   193 His Phe Trp Ser Thr Pro Arg Thr Phe Gly Gly Gly Thr Lys Leu Glu 208
   209 Ile Lys Arg 211
(2) INFORMATION FOR SEQ ID NO:46:
   (i) SEQUENCE CHARACTERISTICS:
      (A) LENGTH: 39 nucleotides
      (B) TYPE: nucleic acid
      (C) STRANDEDNESS: single
      (D) TOPOLOGY: linear
   (xi) SEQUENCE DESCRIPTION SEQ ID NO:46:
   1 GACTATCCCGGGTCTTCTGGCTCCCGTCTGAGCATCAGC 39
(2) INFORMATION FOR SEQ ID NO:47:
   (i) SEQUENCE CHARACTERISTICS:
      (A) LENGTH: 45 nucleotides
      (B) TYPE: nucleic acid
      (C) STRANDEDNESS: single
      (D) TOPOLOGY: linear
   (xi) SEQUENCE DESCRIPTION SEQ ID NO:47:
   1 GGCACCACGCTGACCGTCTCCGCGCCCTCACAGAGCCTGTCCATC 45
(2) INFORMATION FOR SEQ ID NO:48:
   (i) SEQUENCE CHARACTERISTICS:
      (A) LENGTH: 46 nucleotides
      (B) TYPE: nucleic acid
      (C) STRANDEDNESS: single
      (D) TOPOLOGY: linear
   (xi) SEQUENCE DESCRIPTION SEQ ID NO:48:
   1 CAGACTATAATTCAGCTCTCAAAGACATCCAGATGACCCAGTCTCC 46
(2) INFORMATION FOR SEQ ID NO:49:
   (i) SEQUENCE CHARACTERISTICS:
      (A) LENGTH: 45 nucleotides
      (B) TYPE: nucleic acid
      (C) STRANDEDNESS: single
      (D) TOPOLOGY: linear
   (xi) SEQUENCE DESCRIPTION SEQ ID NO:49:
   1 GATGGACAGGCTCTGTGAGGGCGCGGAGACGGTCAGCGTGGTGCC 45
(2) INFORMATION FOR SEQ ID NO:50:
   (i) SEQUENCE CHARACTERISTICS:
      (A) LENGTH: 46 nucleotides
      (B) TYPE: nucleic acid
      (C) STRANDEDNESS: single
      (D) TOPOLOGY: linear
   (xi) SEQUENCE DESCRIPTION SEQ ID NO:50:
   1 GGAGACTGGGTCATCTGGATGTCTTTGAGAGCTGAATTATAGTCTG 46
(2) INFORMATION FOR SEQ ID NO:51:
   (i) SEQUENCE CHARACTERISTICS:
      (A) LENGTH: 24 nucleotides
      (B) TYPE: nucleic acid
      (C) STRAKDEDNESS: single
      (D) TOPOLOGY: linear
   (xi) SEQUENCE DESCRIPTION SEQ ID NO:51:
   1 CGACGGCCAGTGAATTCGAGCTGG 24
(2) INFORMATION FOR SEQ ID NO:52:
   (i) SEQUENCE CHARACTERISTICS:
      (A) LENGTH: 72 nucleotides
      (B) TYPE: nucleic acid
      (C) STRANDEDNESS: single
      (D) TOPOLOGY: linear
   (xi) SEQUENCE DESCRIPTION SEQ ID NO:52:
   1     GCAACTAGTTAACTAGTACGCAAGTTCACGTAAAAAGGRWWWWWWWWATGTCCCGTCTGA   60
   61 GCATCAGCAAGG 72
(2) INFORMATION FOR SEQ ID NO:53:
   (i) SEQUENCE CHARACTERISTICS:
      (A) LENGTH: 738 base pairs
      (B) TYPE: nucleic acid
      (C) STRANDEDNESS: single
      (D) TOPOLOGY: linear
   (xi) SEQUENCE DESCRIPTION SEQ ID NO:53:
   1 GAC TAT CCC GGG TCT TCT GGC TCC CGT CTG AGC ATC AGC AAG GAC AAC  48
   1 Asp Tyr Pro Gly Ser Ser Gly Ser Arg Leu Ser Ile Ser Lys Asp Asn  16
   49 TCC AAG AGC CAA GTT TTC TTA AAA ATG AAC AGT CTG CAC ACT GAT GAC  96
   17 Ser Lys Ser Gln Val Phe Leu Lys Met Asn Ser Leu His Thr Asp Asp  32
   97 ACA GCC AGG TAC TAC TGT GCC AGA GAG AGA GAT TAT AGG CTT GAC TAC  144
   33 Thr Ala Arg Tyr Tyr Cys Ala Arg Glu Arg Asp Tyr Arg Leu Asp Tyr  48
   145 TGG GGC CAA GGC ACC ACG CTG ACC GTC TCC GCG CCC TCA CAG AGC CTG  192
    49 Trp Gly Gln Gly Thr Thr Leu Thr Val Ser Ala Pro Ser Gln Ser Leu  64
   193 TCC ATC ACA TGC ACC GTC TCA GGG TTC TCA TTA ACC GGC TAT GGT GTA  240
    65 Ser Ile Thr Cys Thr Val Ser Gly Phe Ser Leu Thr Gly Tyr Gly Val  80
   241  AAC TGG GTT CGC CAG CCT CCA GGA AAG GGT CTG GAG TGG CTG GGA ATG   288
    81  Asn Trp Val Arg Gln Pro Pro Gly Lys Gly Leu Glu Trp Leu Gly Met    96
   289  ATT TGG GGT GAT GGA AAC ACA GAC TAT AAT TCA GCT CTC AAA GAC ATC   336
    97  Ile Trp Gly Asp Gly Asn Thr Asp Tyr Asn Ser Ala Leu Lys Asp Ile   112
   337  CAG ATG ACC CAG TCT CCA GCC TCC CTT TCT GCG TCT GTG GGA GAA ACT   384
   113  Gln Met Thr Gln Ser Pro Ala Ser Leu Ser Ala Ser Val Gly Glu Thr   128
   385  GTC ACC ATC ACA TGT CGA GCA AGT GGG AAT ATT CAC AAT TAT TTA GCA   432
   129  Val Thr Ile Thr Cys Arg Ala Ser Gly Asn Ile His Asn Tyr Leu Ala   144
   433  TGG TAT CAG CAG AAA CAG GGA AAA TCT CCT CAG CTC CTG GTC TAT TAT   480
   145  Trp Tyr Gln Gln Lys Gln Gly Lys Ser Pro Gln Leu Leu Val Tyr Tyr   160
   481  ACA ACA ACC TTA GCA GAT GGT GTG CCA TCA AGG TTC AGT GGC AGT GGA   528
   161  Thr Thr Thr Leu Ala Asp Gly Val Pro Ser Arg Phe Ser Gly Ser Gly   176
   529  TCA GGA ACA CAA TAT TCT CTC AAG ATC AAC AGC CTG CAA CCT GAA GAT   576
   177  Ser Gly Thr Gln Tyr Ser Leu Lys Ile Asn Ser Leu Gln Pro Glu Asp   192
   577  TTT GGG AGT TAT TAC TGT CAA CAT TTT TGG AGT ACT CCT CGG ACG TTC   624
   193  Phe Gly Ser Tyr Tyr Cys Gln His Phe Trp Ser Thr Pro Arg Thr Phe   208
   625  GGT GGA GGG ACC AAG CTC GAG ATC AAA CGG GAA CAA AAA CTC ATC TCA   672
   209  Gly Gly Gly Thr Lys Leu Glu Ile Lys Arg Glu Gln Lys Leu Ile Ser   224
   673  GAA GAG GAT CTG AAT TAA TAA TGA TCA AAC GGT AAT AAG GAT CCA GCT   720
   225  Glu Glu Asp Leu Asn                                               240
   721  CGA ATT CAC TGG CCG TCG   738
(2) INFORMATION FOR SEQ ID NO:54:
   (i) SEQUENCE CHARACTERISTICS:
      (A) LENGTH: 244 amino acids
      (B) TYPE: protein
      (C) STRANDEDNESS: single
      (D) TOPOLOGY: linear
   (xi) SEQUENCE DESCRIPTION SEQ ID NO:54:
   1 Met Lys Tyr Leu Leu Pro Thr Ala Ala Ala Gly Leu Leu Leu Leu Ala 16
   17 Ala Gln Pro Ala Met Ala Ser Arg Leu Ser Ile Ser Lys Asp Asn Ser 32
   33 Lys Ser Gln Val Phe Leu Lys Met Asn Ser Leu His Thr Asp Asp Thr 48
   49 Ala Arg Tyr Tyr Cys Ala Arg Glu Arg Asp Tyr Arg Leu Asp Tyr Trp 64
   65 Gly Gln Gly Thr Thr Leu Thr Val Ser Ala Pro Ser Gln Ser Leu Ser 80
   81 Ile Thr Cys Thr Val Ser Gly Phe Ser Leu Thr Gly Tyr Gly Val Asn 96
   97 Trp Val Arg Gln Pro Pro Gly Lys Gly Leu Glu Trp Leu Gly Met Ile 112
   113 Trp Gly Asp Gly Asn Thr Asp Tyr Asn Ser Ala Leu Lys Asp Ile Gln 128
   129 Met Thr Gln Ser Pro Ala Ser Leu Ser Ala Ser Val Gly Glu Thr Val 144
   145 Thr Ile Thr Cys Arg Ala Ser Gly Asn Ile His Asn Tyr Leu Ala Trp 160
   161 Tyr Gln Gln Lys Gln Gly Lys Ser Pro Gln Leu Leu Val Tyr Tyr Thr 176
   177 Thr Thr Leu Ala Asp Gly Val Pro Ser Arg Phe Ser Gly Ser Gly Ser 192
   193 Gly Thr Gln Tyr Ser Leu Lys Ile Asn Ser Leu Gln Pro Glu Asp Phe 208
   209 Gly Ser Tyr Tyr Cys Gln His Phe Trp Ser Thr Pro Arg Thr Phe Gly 224
   225 Gly Gly Thr Lys Leu Glu Ile Lys Arg Glu Gln Lys Leu Ile Ser Glu 240
   241 Glu Asp Leu Asn 244
(2) INFORMATION FOR SEQ ID NO:55
   (i) SEQUENCE CHARACTERISTICS:
      (A) LENGTH: 211 amino acids
      (B) TYPE: amino acids
      (D) TOPOLOGY: linear
   (xi) SEQUENCE DESCRIPTION: SEQ ID NO 55:
(2) INFORMATION FOR SEQ ID NO:56
   (i) SEQUENCE CHARACTERISTICS:
      (A) LENGTH: 211 amino acids
      (B) TYPE: amino acids
      (D) TOPOLOGY: linear
   (xi) SEQUENCE DESCRIPTION: SEQ ID NO 56:
   Arg Phe Ser Gly Ser Gly Ser Gly Thr Gln Tyr Ser Leu Lys Ile Asn
   1               5                   10                  15
   Ser Leu Gln Pro Glu Asp Phe Gly Ser Tyr Thr Cys Gln His Phe Trp
               20                  25                  30
   Ser Thr Pro Arg Thr Phe Gly Gly Gly Thr Lys Leu Glu Ile Lys Ala
           35                  40                  45
   Ser Val Gly Glu Thr Val Thr Ile Thr Cys Arg Ala Ser Gly Asn Ile
       50                  55                  60
   His Asn Tyr Leu Ala Trp Tyr Gln Gln Lys Gln Gly Lys Ser Pro Gln
   65                  70                  75                  80
   Leu Leu Val Tyr Tyr Thr Thr Thr Leu Ala Asp Gly Val Pro Ser Gln
                   85                  90                  95
   Val Gln Leu Lys Glu Ser Gly Pro Gly Leu Val Ala Pro Ser Gln Ser
               100                 105                 110
   Leu Ser Ile Thr Cys Thr Val Ser Gly Phe Ser Leu Thr Gly Tyr Gly
           115                 120                 125
   Val Asn Trp Val Arg Gln Pro Pro Gly Lys Gly Leu Glu Trp Leu Gly
       130                 135                 140
   Met Ile Trp Gly Asp Gly Asn Thr Asp Tyr Asn Ser Ala Leu Lys Ser
   145                 150                 155                 160
   Arg Leu Ser Ile Ser Lys Asp Asn Ser Lys Ser Gln Val Phe Leu Lys
                   165                 170                 175
   Met Asn Ser Leu His Thr Asp Asp Thr Ala Arg Tyr Tyr Cys Ala Arg
               180                 185                 190
   Glu Arg Asp Tyr Arg Leu Asp Tyr Trp Gly Gln Gly Thr Thr Leu Thr
           195                 200                 205
   Val Ser Ser
       210
(2) INFORMATION FOR SEQ ID NO:57
   (i) SEQUENCE CHARACTERISTICS:
      (A) LENGTH:163 amino acids
      (B) TYPE: amino acids
      (D) TOPOLOGY: linear
   (xi) SEQUENCE DESCRIPTION: SEQ ID NO 57:
(2) INFORMATION FOR SEQ ID NO:58
   (i) SEQUENCE CHARACTERISTICS:
      (A) LENGTH: 249 amino acids
      (B) TYPE: amino acids
      (D) TOPOLOGY: linear
   (xi) SEQUENCE DESCRIPTION: SEQ ID NO 58:
(2) INFORMATION FOR SEQ ID NO:59
   (i) SEQUENCE CHARACTERISTICS:
      (A) LENGTH: 226 amino acids
      (B) TYPE: amino acids
      (D) TOPOLOGY: linear
   (xi) SEQUENCE DESCRIPTION: SEQ ID NO 59:
(2) INFORMATION FOR SEQ ID NO:60
   (i) SEQUENCE CHARACTERISTICS:
      (A) LENGTH: 224 amino acids
      (B) TYPE: amino acids
      (D) TOPOLOGY: linear
   (xi) SEQUENCE DESCRIPTION: SEQ ID NO 60:
(61) INFORMATION FOR SEQ ID NO:61
   (i) SEQUENCE CHARACTERISTICS:
      (A) LENGTH: 229 amino acids
      (B) TYPE: amino acids
      (D) TOPOLOGY: linear
   (xi) SEQUENCE DESCRIPTION: SEQ ID NO 61:

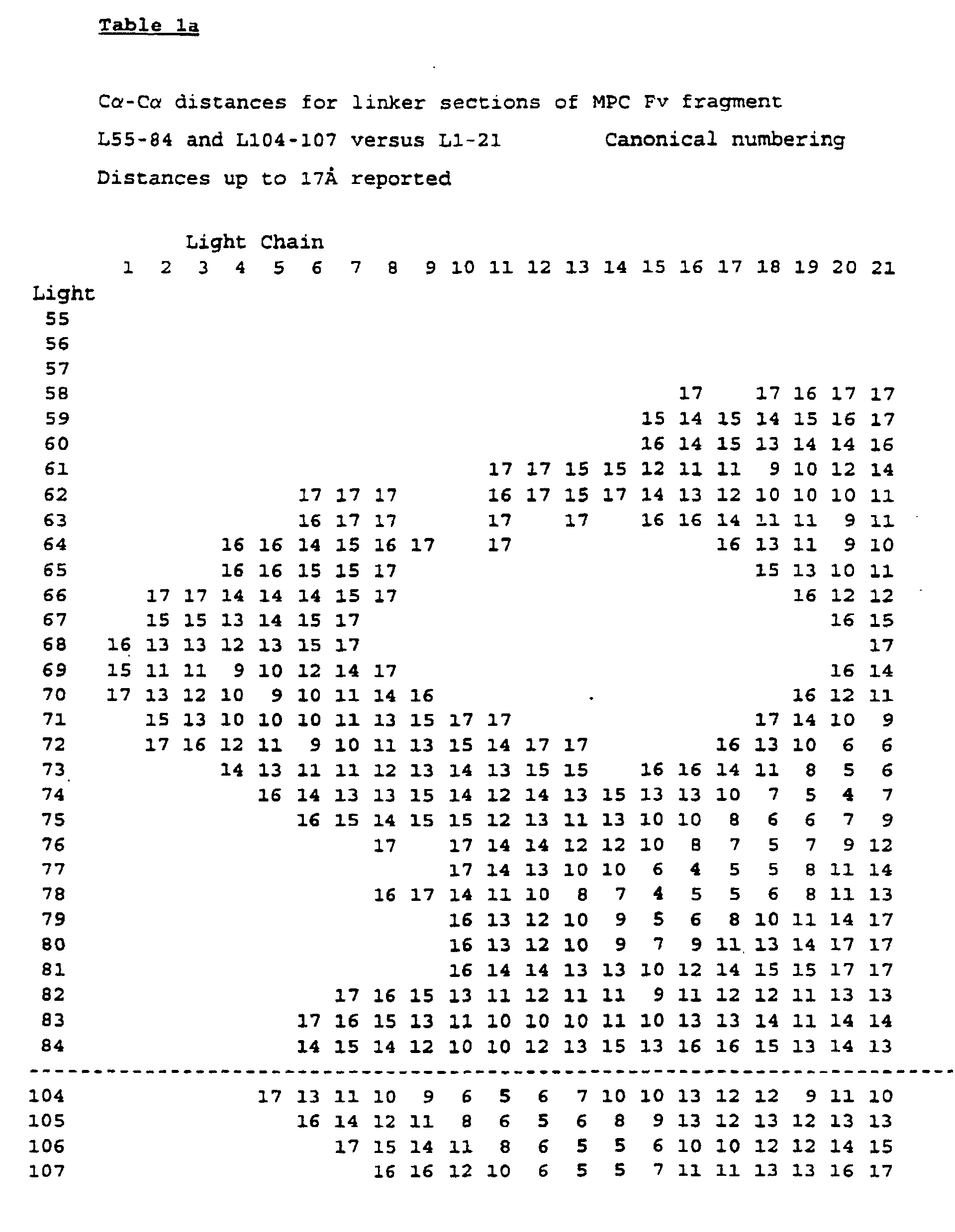

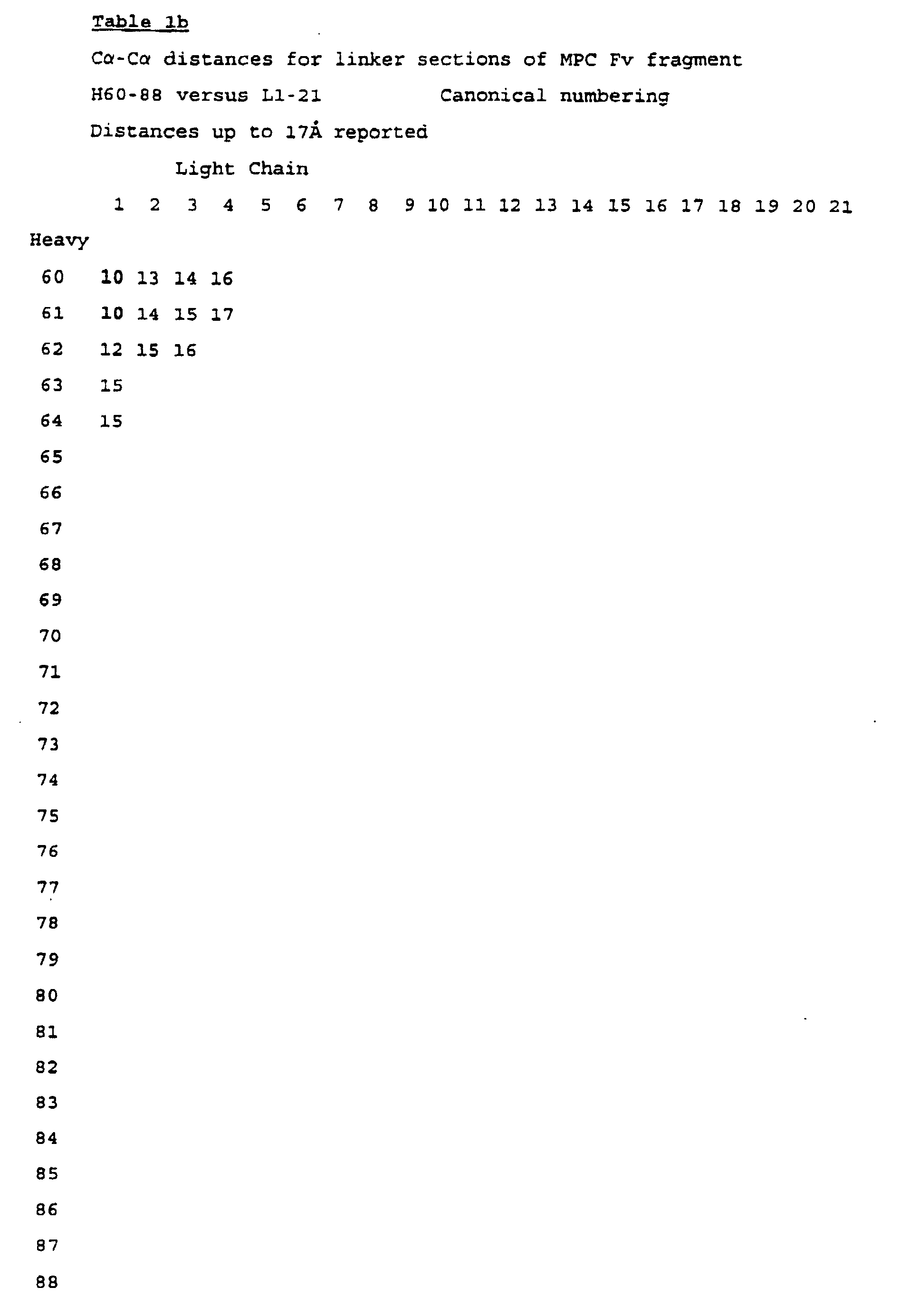

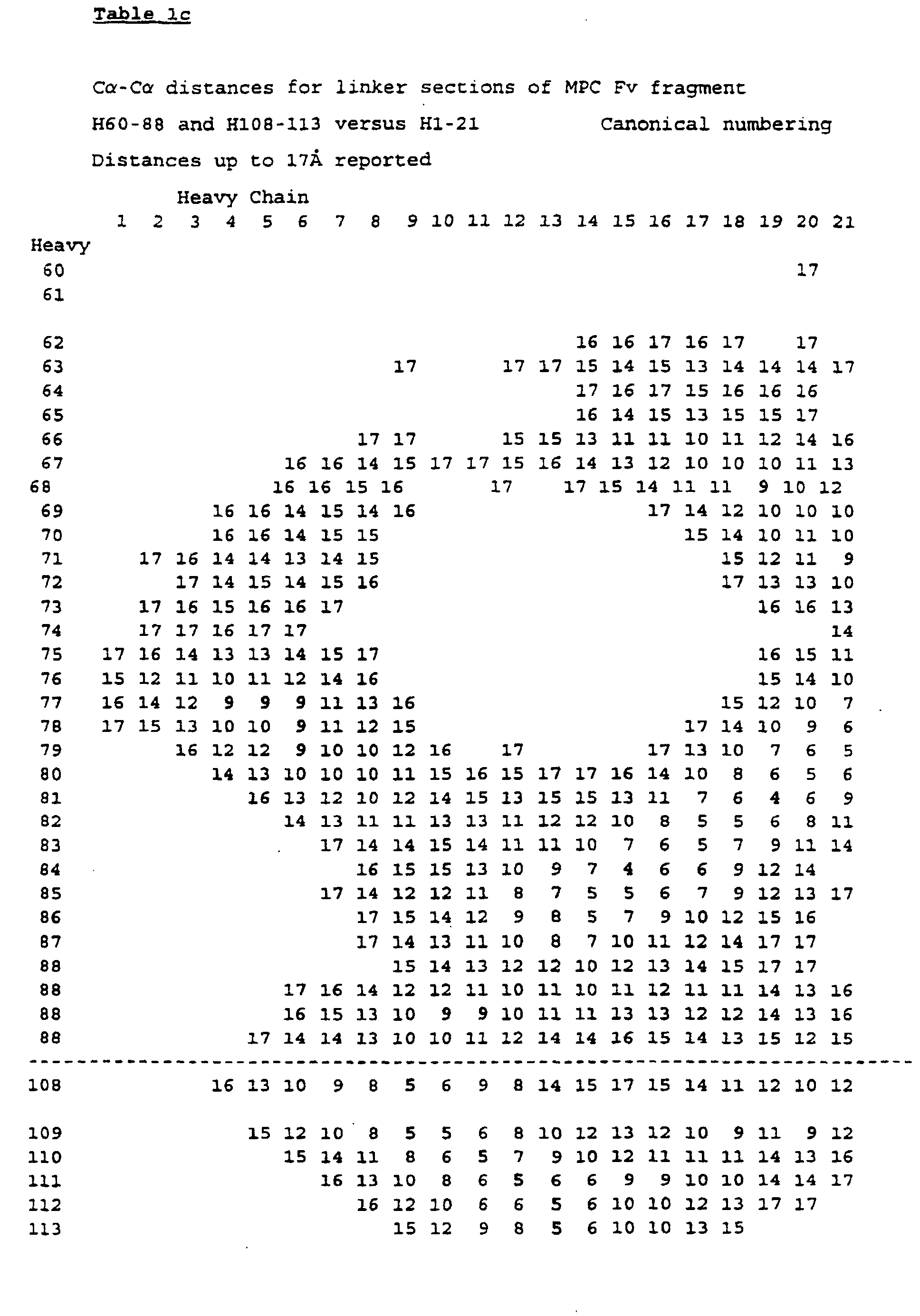

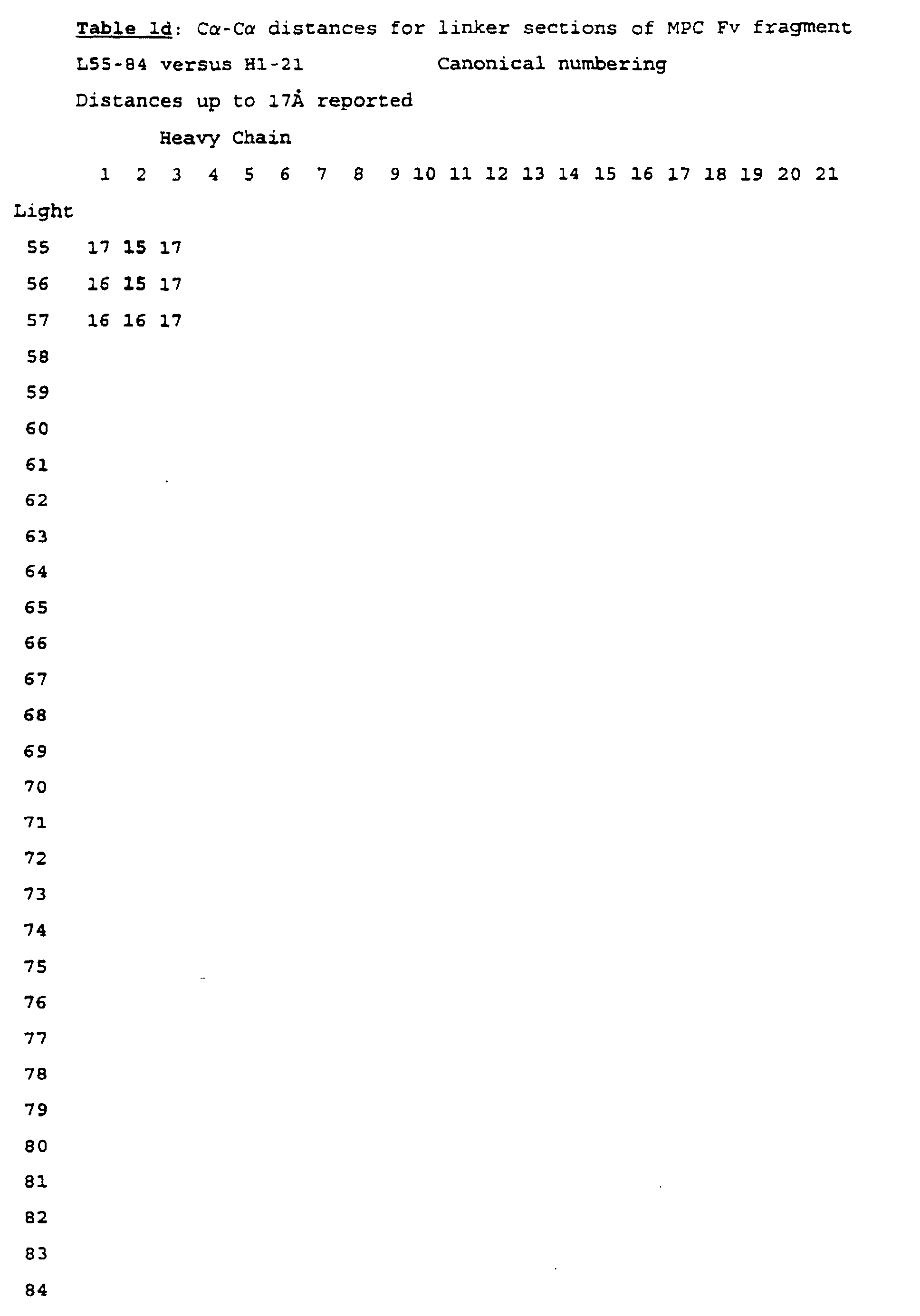

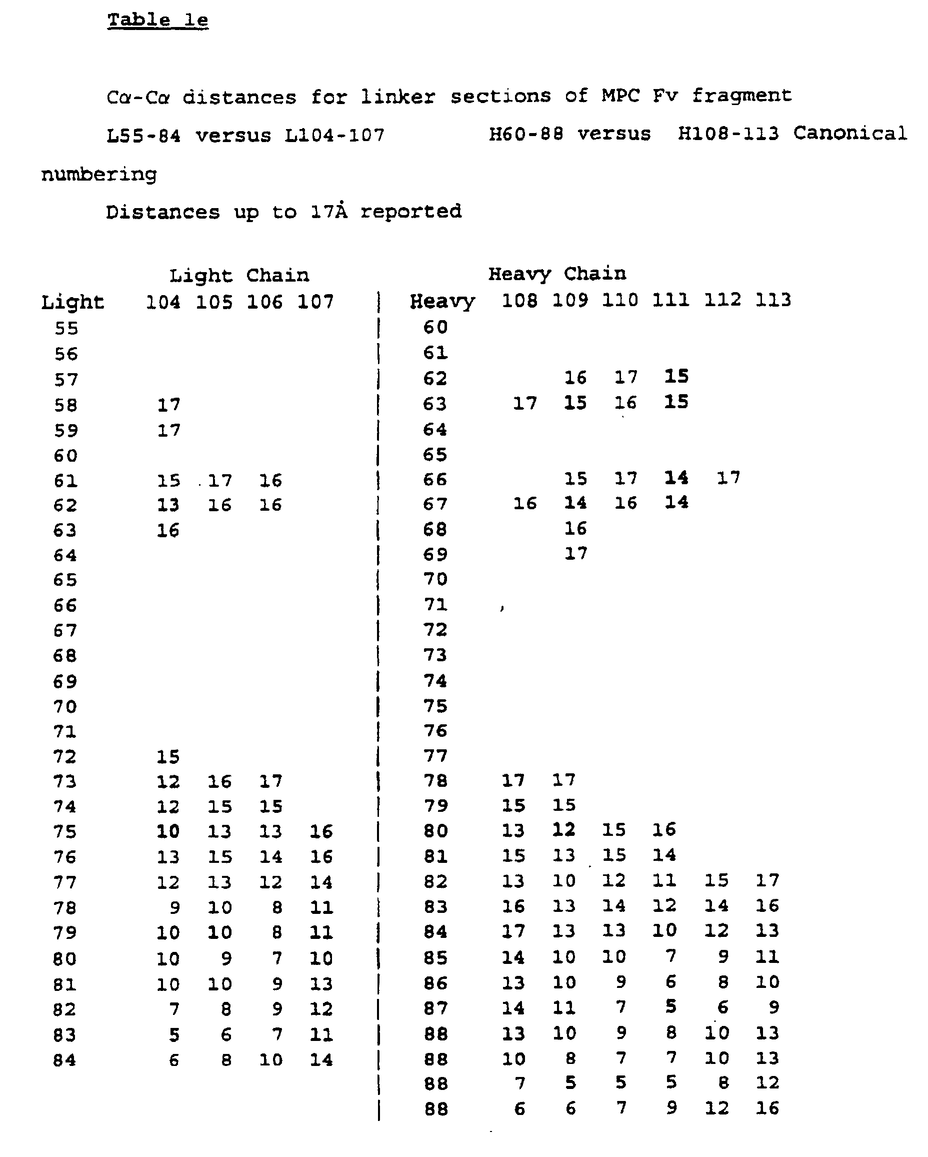

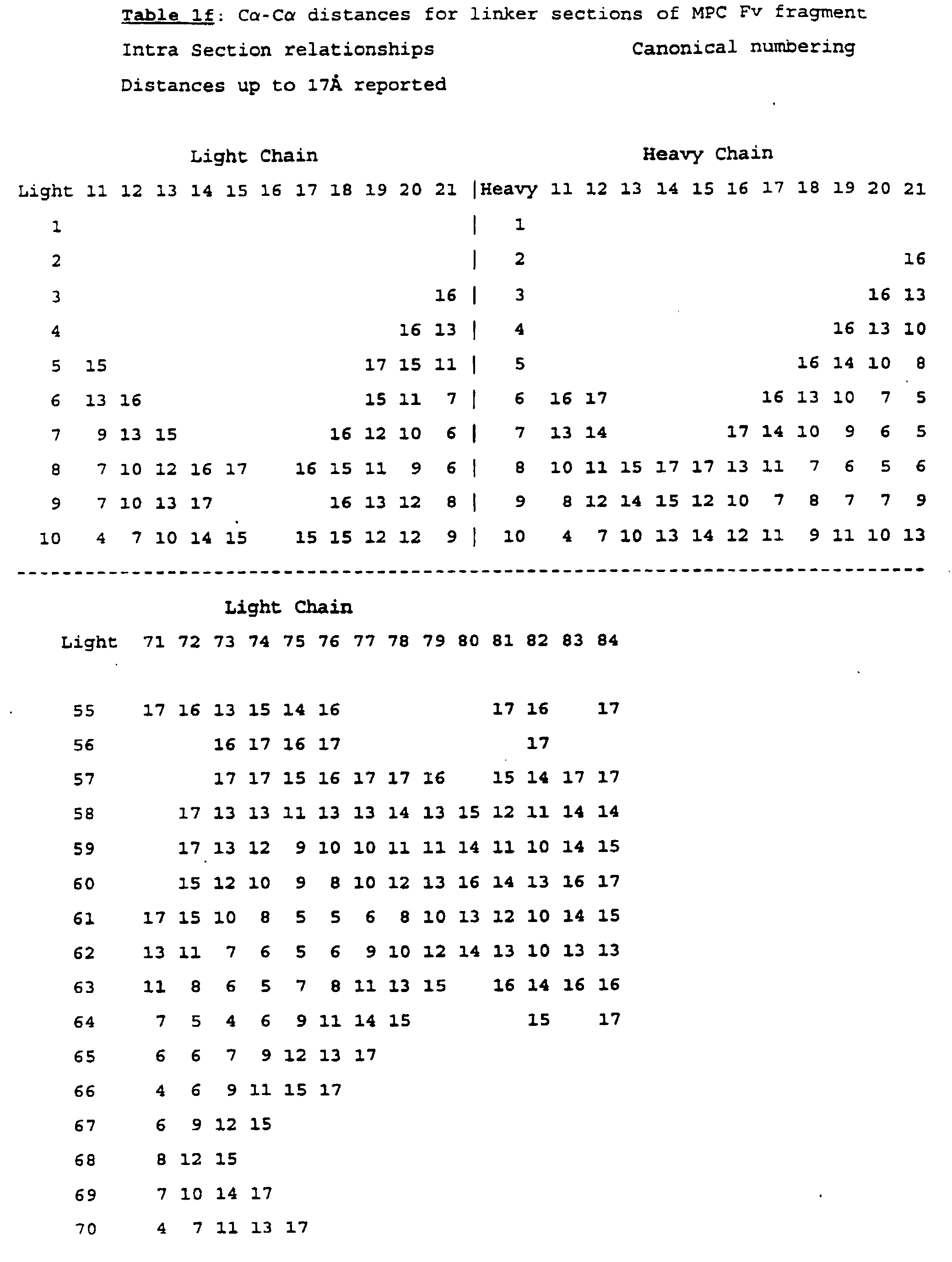

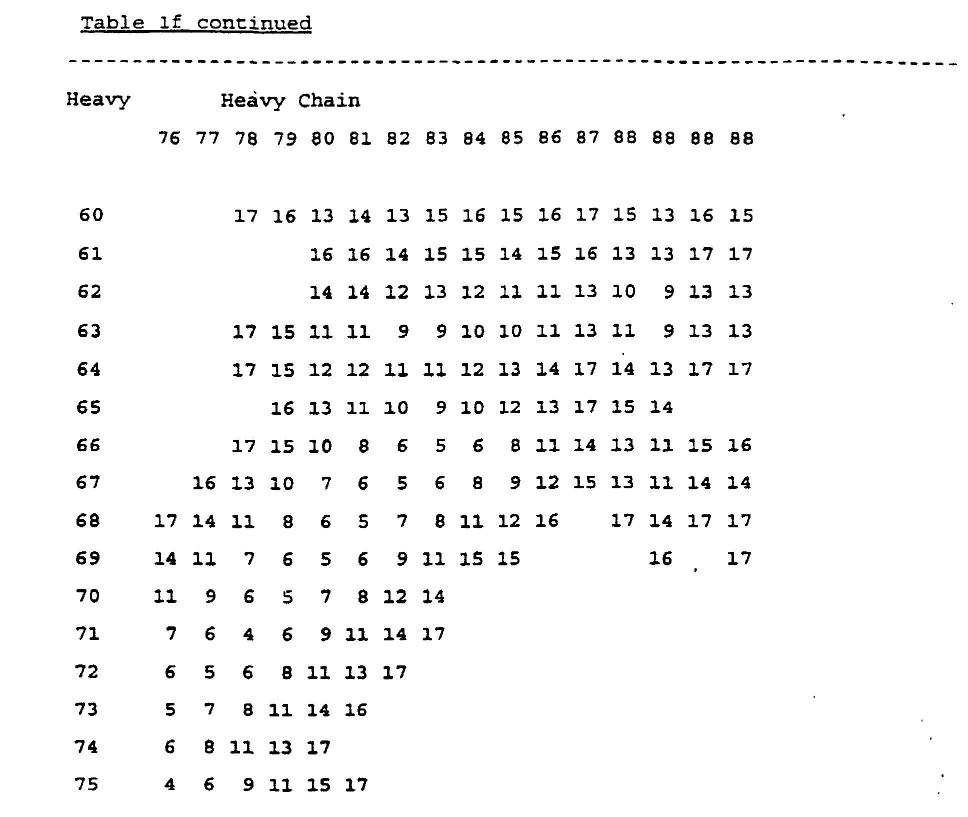

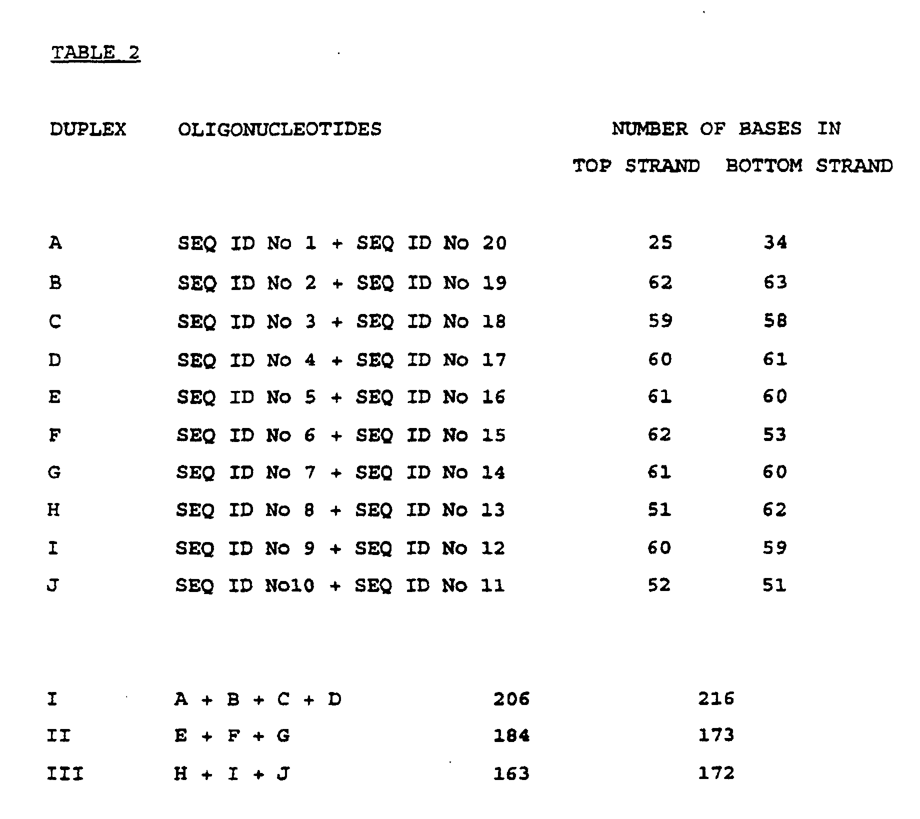

## Claims

1. A ligand binding variable domain (V-min) comprising:
(a) a cyclically permuted central β-barrel having intact strands;
(b) outer beta-sheet segments;
(c) complementarity determining regions (CDRs); and
(d) linker sections for linking integers (a), (b) & (c) into a V-min, whereby said central β-barrel, optionally in combination with said outer β-sheet segments, orients said CDRs in space for binding ligand.

2. A V-min according to claim 1 wherein framework integers (a), (b) and (d) per se have upto 180 amino acids.

3. A V-min according to any one of claims 1-2 wherein the CDRs substantially reproduce the spatial orientation of CDRs from a parent antibody and substantially retain the ligand binding specificity and affinity of the parent antibody.

4. A V-min according to any one of claims 1-3 wherein the linker sections essentially consist of sequences from the antibody used as the source of the central beta-barrel.

5. A V-min according to any one of claims 1-4 wherein the V-min is in the form of one continuous polypeptide.

6. A V-min according to any one of claims 1-5 comprising the following, canonically numbered, core segments: light chain residues 21-55; light chain residues 84-104; heavy chain residues 21-60 and; heavy chain residues 88-109.

7. A multivalent ligand binding moiety comprising at least one V-min as defined in any one of claims 1-6.

8. An conjugate comprising a V-min as defined in any one of claims 1-7 and an effector molecule.

9. A polynucleotide sequence encoding a V-min as defined in any one of claims 1-6.

10. An expression vector comprising a polynucleotide sequence as defined in claim 9.

11. A host cell comprising an expression vector as defined in claim 10.

12. A method of producing a V-min comprising culture of a host cell as defined in claim 11.

## Patentansprüche

1. Ligandenbindende variable Domäne (V-min), umfassend:
(a) einen zyklisch permutierten zentralen β-Tubus mit intakten Strängen;
(b) äußere β-Faltblattsegmente;
(c) komplementaritätsbestimmende Bereiche (CDRs); und
(d) Linkerabschnitte zur Verknüpfung von Ziffern (a), (b) & (c) zu V-min, wodurch der zentrale β-Tubus, gegebenenfalls in Kombination mit den äußeren β-Faltblattsegmenten, die CDRs räumlich für die Ligandenbindung ausrichtet.

2. V-min nach Anspruch 1, wobei die "framework"-Ziffern (a), (b) und (c) per se bis zu 180 Aminosäuren aufweisen.

3. V-min nach einem der Ansprüche 1 und 2, wobei die CDRs weitgehend die räumliche Orientierung von CDRs aus einem Ausgangsantikörper wiedergeben und weitgehend die Ligandenbindungsspezifität und -affinität des Ausgangsantikörpers behalten.

4. V-min nach einem der Ansprüche 1 bis 3, wobei die Linkerabschnitte im wesentlichen aus Sequenzen aus dem als Quelle für den zentralen β-Tubus verwendeten Antikörper bestehen.

5. V-min nach einem der Ansprüche 1 bis 4, wobei die V-min in Form eines einzigen, durchgehenden Polypeptids vorliegt.

6. V-min nach einem der Ansprüche 1 bis 5, umfassend die folgenden, kanonisch numerierten Kernabschnitte: leichte-Kette-Reste 21-55; leichte-Kette-Reste 84-104; schwere-Kette-Reste 21-60 und; schwere-Kette-Reste 88-109.

7. Mehrwertiger ligandenbindender Anteil, umfassend wenigstens eine wie in einem der Ansprüche 1 bis 6 definierte V-min.

8. Konjugat, umfassend eine wie in einem der Ansprüche 1 bis 7 definierte V-min sowie ein Effektormolekül.

9. Polynukleotidsequenz, kodierend eine wie in einem der Ansprüche 1 bis 6 definierte V-min.

10. Expressionsvektor, umfassend eine wie in Anspruch 9 definierte Polynukleotidsequenz.

11. Wirtszelle, umfassend einen wie in Anspruch 10 definierten Expressionsvektor.

12. Verfahren zur Herstellung einer eine V-min enthaltenden Kultur einer wie in Anspruch 11 definierten Wirtszelle.

## Revendications

1. Domaine variable de liaison de ligands (V-min) comprenant :
(a) une permutation cyclique de la structure centrale en β-baril ayant des brins intacts ;
(b) des segments extérieurs de feuille bêta ;
(c) des régions déterminant une complémentarité (CDRs) ; et
(d) des sections de lieur pour la liaison d'entiers (a), (b) et (c) dans un V-min, de cette façon ladite structure centrale en β-baril, facultativement en combinaison avec lesdits segments extérieurs de feuille β, orientant lesdits CDRs dans l'espace pour la liaison de ligands.

2. V-min selon la revendication 1 dans lequel les entiers d'encadrement (a), (b) et (d) en soi ont jusqu'à 180 acides aminés.

3. V-min selon l'une quelconque des revendications 1-2 dans lequel les CDRs reproduisent substantiellement l'orientation spatiale des CDRs d'un anticorps parent et conservent substantiellement la spécificité et l'affinité de la liaison de ligands de l'anticorps parent.

4. V-min selon l'une quelconque des revendications 1-3 dans lequel les sections de lieur consistent essentiellement en des séquences de l'anticorps utilisé comme source de la structure centrale en β-baril.

5. V-min selon l'une quelconque des revendications 1-4 dans lequel le V-min est sous la forme d'un polypeptide continu.

6. V-min selon l'une quelconque des revendications 1-5 comprenant les segments de noyau suivants, conformément numérotés : des résidus de chaîne légère 21-55 ; des résidus de chaîne légère 84-104 ; des résidus de chaîne lourde 21-60 et des résidus de chaîne lourde 88-109.

7. Partie de liaison de ligands multivalente comprenant au moins un V-min comme défini dans l'une quelconque des revendications 1-6.

8. Conjugaison comprenant un V-min comme défini dans l'une quelconque des revendications 1-7 et une molécule effectrice.

9. Séquence de polynucléotide codant un V-min comme défini dans l'une quelconque des revendications 1-6.

10. Vecteur d'expression comprenant une séquence de polynucléotide comme défini dans la revendication 9.

11. Cellule hôte comprenant un vecteur d'expression comme défini dans la revendication 10.

12. Procédé de production de V-min comprenant la culture d'une cellule hôte comme défini dans le revendication 11.
